(19) Europäisches Patentamt
European Patent Office
Office européen des brevets

(11) Publication number: 0 174 131
A2

# (12) EUROPEAN PATENT APPLICATION

(21) Application number: 85305930.1

(22) Date of filing: 21.08.85

(51) Int. Cl.[4]: C 07 D 211/90
C 07 D 405/12, C 07 D 409/12
C 07 D 401/12, C 07 D 401/04
C 07 D 409/14, C 07 D 417/12
C 07 D 401/06, A 61 K 31/44
//C07D331/04, C07D305/08,
C07D231/20, C07D209/48,
C07C47/54

(30) Priority: 01.09.84 GB 8422139
19.10.84 GB 8426559
19.10.84 GB 8426560
19.10.84 GB 8426562
19.10.84 GB 8426563
19.10.84 GB 8426569
19.10.84 GB 8426570
19.10.84 GB 8426571
30.11.84 GB 8430296
20.03.85 GB 8507163

(43) Date of publication of application:
12.03.86 Bulletin 86/11

(84) Designated Contracting States:
AT BE CH DE FR GB IT LI LU NL SE

(71) Applicant: FISONS plc
Fison House Princes Street
Ipswich Suffolk IP1 1QH(GB)

(72) Inventor: Baxter, Andrew John Gilby
78 Mount Pleasant
Keyworth Nottinghamshire(GB)

(72) Inventor: Dixon, John
Church Farmhouse Main Street Great Dalby
Melton Mowbray Leicestershire(GB)

(72) Inventor: Gould, Kenneth John
3A Turvey Lane
Long Whatton Leicestershire(GB)

(72) Inventor: Tinker, Alan Charles
97 Knightthorpe Road
Loughborough Leicestershire(GB)

(74) Representative: Craig, Christopher Bradberry et al,
Fisons plc 12 Derby Road
Loughborough Leicestershire LE11 0BB(GB)

## (54) Dihydropyridines, processes and intermediates for their production, and pharmaceutical formulations containing them.

(57) There are described compounds of formula I,

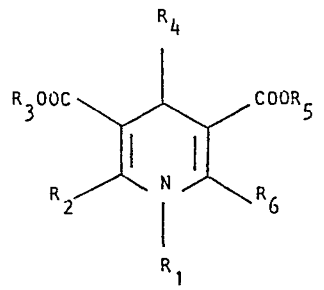

(I)

in which $R_1$, $R_2$, $R_3$, $R_5$ and $R_6$ are as defined in the specification and $R_4$ is a phenyl group carrying 3 or 4 substituents or is an unsaturated 5 membered heterocyclic group containing a single hetero-atom selected from oxygen, sulphur or nitrogen, $R_4$ being substituted by $CF_3$, $XR_{22}$, nitro, halogen, CN, alkyl C1 to 6, formyl dioxalanyl, $-N(R_{23})COR_{24}$ $-CONR_{25}R_{26}$, or amino which is optionally substituted by phenylsulphonyl or by trifluoroacetyl, and $R_4$ carries a substituent on the atom adjacent to the atom through which $R_4$ is connected to the remainder of the molecule,

$R_{22}$ is phenyl or alkyl C1 to 6 optionally substituted by halogen,

X is O or $S(O)_n$,

n is 0, 1 or 2, and

$R_{23}$, $R_{24}$, $R_{25}$ and $R_{26}$, which may be the same or different, are each hydrogen or alkyl C1 to 6.

There are also described methods for making the compounds, and their formulation and use as pharmaceuticals, eg for the treatment of cardiovascular conditions.

There are further described compounds $R_4CHO$ which are useful in making compounds of formula I.

EP 0 174 131 A2

0174131

NOVEL DIHYDROPYRIDINES, PROCESSES AND INTERMEDIATES FOR THEIR PRODUCTION AND PHARMACEUTICAL FORMULATIONS CONTAINING THEM

This invention relates to new compounds, methods and intermediates for their preparation and compositions containing them.

A wide variety of dihydropyridines have been described as being useful as pharmaceuticals and some, notably nifedipine, have been sold for this use.

We have now found a new group of dihydropyridines having an unsaturated cyclic group in the 4-position wherein the cyclic group has a specific substitution pattern.

According to the invention we provide compounds of formula I,

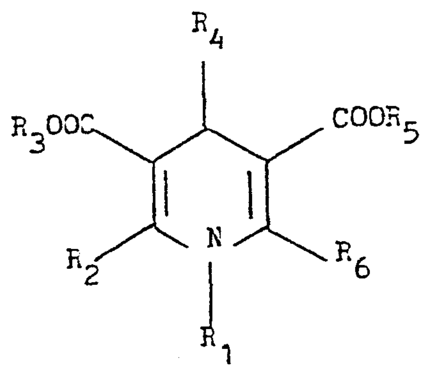

I

in which $R_1$ is hydrogen or alkyl C1 to 6,

$R_3$ and $R_5$, which may be the same or different, are each $-(CH_2)_x-(CHR_{34})_yR_{35}$,

x is a whole number from 0 to 7 inclusive,

y is 0 or 1,

$R_{34}$ is hydrogen, alkyl C1 to 6 or cycloalkyl containing up to and including 8 carbon atoms,

$R_{35}$ is hydrogen; alkyl C1 to 6 optionally substituted by halogen, by alkoxy C1 to 6, by alkanoyl C1 to 6, or by hydroxy; a 4, 5 or 6 membered oxygen, nitrogen or $S(O)_n$ containing heterocyclic ring which ring is optionally substituted by alkyl C1 to 6; cycloalkyl or cycloalkenyl containing up to and including 8 carbon atoms which cycloalkyl is in turn optionally bridged, substituted by alkyl C1 to 6 or is fused to a phenyl ring; a group $-CH=CHR_{13}$ in which $R_{13}$ is cycloalkyl containing up to and including 8 carbon atoms; or is an alkyl C1 to 6 amino group in which the alkyl group(s) are optionally substituted by phenyl; or, when x is a whole number from 1 to 7 inclusive $R_{35}$ is CN, $-OSO_2R_{33}$ or a group of formula II or III,

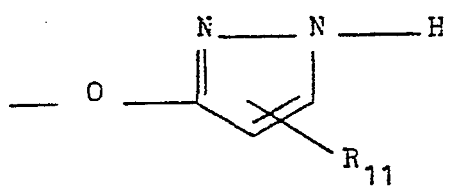

II

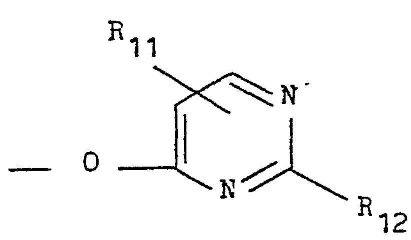

III

in which $R_{11}$ and $R_{12}$, which may be the same or different, are each alkyl C1 to 6 or phenyl, and

$R_{33}$ is alkyl C1 to 6 or alkyl C1 to 6-phenyl,

$R_2$ and $R_6$, which may be the same or different, are each CN, CHO or alkyl C1 to 6 optionally interrupted by an oxygen atom and optionally substituted by halogen, by hydroxy, by -COOH or an alkyl C1 to 6 ester thereof, by a C1 to 6 betaketoester group, by a 2-amino-6-hydroxy-pyridmidin-4-yl group or by a group $-NR_{14}R_{15}$,

$R_{14}$ and $R_{15}$, which may be the same or different, are each hydrogen, alkyl C1 to 6, -C(=NCN)Salkyl C1 to 6 or a group of formula IV,

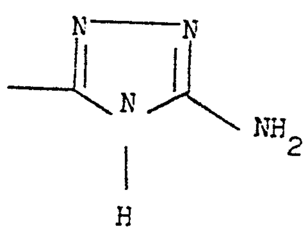

IV

or together form a chain $-CR_{16}=N-CR_{17}=CR_{18}-$, $-CH_2CH_2N(R_{19})CH_2CH_2-$ or -CO(o-phenylene)CO-,

$R_{16}$, $R_{17}$ and $R_{18}$, which may be the same or different, are each alkyl C1 to 6,

$R_{19}$ is hydrogen; alkyl C1 to 6 optionally substituted by phenyl which in turn is optionally substituted by halogen; $-CONR_{20}R_{21}$; or a 5 membered unsaturated ring containing nitrogen and optionally also sulphur,

$R_{20}$ and $R_{21}$, which may be the same or different,

are each hydrogen or alkyl C1 to 6,

$R_4$ is a phenyl group carrying 3 or 4 substituents or is an unsaturated 5 membered heterocyclic group containing a single hetero atom selected from oxygen, sulphur or nitrogen, $R_4$ being substituted by $CF_3$, $XR_{22}$, nitro, halogen, CN, alkyl C1 to 6, formyl, dioxalanyl, $-N(R_{23})COR_{24}$, $-CONR_{25}R_{26}$, or amino which is optionally substituted by phenylsulphonyl or by trifluoroacetyl, and $R_4$ carries a substituent on the atom adjacent to the atom through which $R_4$ is connected to the remainder of the molecule,

$R_{22}$ is phenyl or alkyl C1 to 6 optionally substituted by halogen,

X is O or $S(O)_n$,

n is 0, 1 or 2, and

$R_{23}$, $R_{24}$, $R_{25}$ and $R_{26}$, which may be the same or different, are each hydrogen or alkyl C1 to 6,

provided that i) when $R_1$ is hydrogen or methyl, $R_2$ is $CH_2F$, $R_3$ is methyl and $R_5$ is 1-methylethyl or cyclopentyl, then $R_4$ is not 2-(trifluoromethyl)-3-chloro-6-fluorophenyl,

ii) when $R_1$ is hydrogen, $R_2$ is $CH_2F$, $R_3$ is methyl and $R_5$ is 1-methylethyl then $R_4$ is not 2,3-dichloro-6-fluorophenyl, and

iii) when $R_4$ is a heterocyclic group then at least

one of $R_2$ and $R_6$ is other than alkyl or alkoxy-alkyl,

and pharmaceutically acceptable acid addition salts of those compounds containing a basic nitrogen atom.

According to the invention we also provide the compounds of formula I, and pharmaceutically acceptable acid addition salts thereof, for use as pharmaceuticals.

According to the invention we further provide a process for the production of a compound of formula I, or a pharmaceutically acceptable acid addition salt thereof, which comprises

a) reaction of the compounds of formula V, VII, X and XIV,

$R_4CHO$ V

$R_5OOCCH_2COR_6$ VII

$R_3OOCCH_2COR_2$ X

$R_1NH_2$ XIV

or their partial condensates,

in which $R_1$, $R_2$, $R_3$, $R_4$, $R_5$ and $R_6$ are as defined above,

b) production of a compound of formula I containing a group $-S(O)_m-$ in which m is 1 or 2 by selective oxidation of a corresponding compound of formula I in which m is 0 or 1,

c) production of a compound of formula I in which one or both of $R_2$ and $R_6$ is $-CHF_2$ or $-CH_2F$ by reaction of

a corresponding compound of formula I in which one or both of $R_2$ and $R_6$ is -CHO or $-CH_2L$ respectively, where L is -OH or a good leaving group, with a fluorinating agent,

d) production of a compound of formula I carrying a -CHO substituent by

i) selective hydrolysis of a corresponding compound of formula I carrying a $-CH(OR_{27})_2$ substituent,

in which $R_{27}$ is alkyl C1 to 6, or the two group $R_{27}$ together form a $-CH_2CH_2-$ chain, or

ii) selective oxidation of a corresponding compound of formula I carrying a $-CH_2OH$ substituent,

e) production of a compound of formula I in which one of $R_2$ and $R_6$ is $-CH_2OH$ by selective hydrolysis of a corresponding compound of formula I in which one of $R_2$ and $R_6$ is $-CH_2Z$ and Z is a good leaving group,

f) production of a compound of formula I carrying a group -CN by elimination of ROH from a corresponding compound of formula I carrying a group -CH=NOR, in which -OR is a good leaving group,

g) production of a compound of formula I in which at least one of $R_3$ and $R_5$ is hydrogen, or in which at least one of $R_2$ and $R_6$ carries a -COOH group, by reductive cleavage or hydrolysis of a corresponding compound of formula I in which at least one of $R_3$ and $R_5$ is other than hydrogen or in which at least one of

$R_2$ and $R_6$ carries a carboxylic acid ester group,

h) production of a compound of formula I in which at least one of $R_3$ and $R_5$ is other than hydrogen, or in which at least one of $R_2$ and $R_6$ carries a carboxylic acid ester group or a betaketoester group, by esterification or transesterification of a corresponding compound of formula I in which at least one of $R_3$ and $R_5$ is hydrogen or is a group $R_3$ or $R_5$ other than that desired in the end product, or in which at least one of $R_2$ and $R_6$ carries a -COOH group,

i) production of a compound of formula I in which at least one of $R_2$ and $R_6$ is methyl by selective reduction of a corresponding compound of formula I in which at least one of $R_2$ and $R_6$ is $-CH_2Y$ and Y is a group reducible to hydrogen,

j) production of a compound of formula I carrying an $-NH_2$ substituent by removal of a protecting group from a corresponding compound carrying a protected amino group,

k) production of a compound of formula I in which at least one of $R_3$ and $R_5$ is alkyl substituted by a group of formula II or III, by reaction of a corresponding compound of formula I in which at least one of $R_3$ and $R_5$ is alkyl substituted by a good leaving group, with a compound of formula XI or XII respectively,

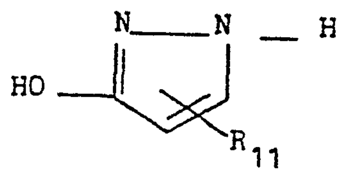

XI

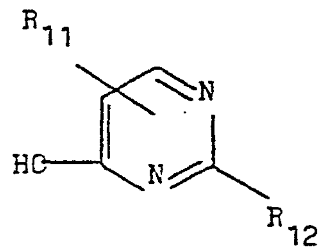

XII

or a tautomer or salt thereof,

in which $R_{11}$ and $R_{12}$ are as defined above,

l) production of a compound of formula I in which at least one of $R_3$ and $R_5$ represents alkyl substituted by a group $-CH(R_{36})(CH_2OH)-CH_2OH$, in which $R_{36}$ represents hydrogen or alkyl, by selective hydrolysis of a corresponding compound of formula I in which at least one of $R_3$ and $R_5$ represents alkyl substituted by 3-oxetanyl which in turn is optionally substituted in the 3-position by alkyl,

m) production of a compound of formula I in which $R_1$ is alkyl C1 to 6, by C1 to 6 alkylation of a corresponding compound of formula I in which $R_1$ is hydrogen,

n) production of a compound of formula I in which one of

$R_{14}$ and $R_{15}$ is a group of formula IV, by reaction of a corresponding compound of formula I in which one of $R_{14}$ and $R_{15}$ is -C(=NCN)Salkyl C1 to 6 with hydrazine,

o) production of a compound of formula I in which $R_{14}$ and $R_{15}$ together form a chain $-CR_{16}=N-CR_{17}=CR_{18}-$, by reaction of a corresponding compound of formula I in which $R_{14}$ and $R_{15}$ are both hydrogen with ammonia and an alkanaldehyde and an alkanendione in which the two oxygen atoms are carried on adjacent carbon atoms,

p) production of a compound of formula I in which $R_{19}$ is alkylamino-carbonyl or a 5 membered ring, by reacting a corresponding compound of formula I in which $R_{19}$ is hydrogen with an alkylisocyanate or with a corresponding 5 membered ring compound carrying a good leaving group,

q) production of a compound of formula I in which $R_{14}$ or $R_{15}$ is a group -C(=NCN)Salkyl C1 to 6, by reaction of a corresponding compound of formula I in which at least one of $R_{14}$ and $R_{15}$ is hydrogen, with a di-alkyl(C1 to 6)N-cyanoiminodithiocarbonate,

r) production of a compound of formula I in which at least one of $R_2$ and $R_6$ is substituted by a 2-amino-6-hydroxy-pyrimidin-4-yl group, by reacting a corresponding compound of formula I in which at least one of $R_2$ and $R_6$ is substituted by a betaketoester group with guanidine, or

s) production of an optical isomer of a compound of formula I by resolution of a mixture of optical isomers of the compound,

and where desired or necessary converting the resulting compound of formula I to a pharmaceutically acceptable acid addition salt thereof or vice versa.

The reactions of process a) (Hantzch Synthesis) may be carried out by subjecting the reagents to an elevated temperature, eg of from about 20° to 140°C in the presence or absence of a suitable solvent, eg a lower alkanol. The reaction of process a) may comprise

i) reaction of a compound of formula V,

$R_4CHO$ V

with compounds of formulae VI and VII,

$R_3OOCCH=C(R_2)NH_2$ VI

$R_5OOCCH_2COR_6$ VII

in which formulae $R_2$, $R_3$, $R_4$, $R_5$ and $R_6$ are as defined above, or

ii) reaction of a compound of formula VIII,

$R_4CH=C(COOR_5)COR_6$ VIII

in which $R_4$, $R_5$, and $R_6$ are as defined above, with a compound of formula VI, or

iii) reaction of compounds of formulae VII, IX and XIV,

$R_4CH=C(COOR_3)COR_2$ IX

or reaction of compounds of formulae VIII, X and XIV,

or

reaction of compounds of formulae V, VII, X and XIV.

In process a)iii) the compound (XIV) may be present as a salt, eg ammonium acetate. In process a) a proportion of an intermediate hydroxy-tetrahydropyridine may be formed and the yield of the desired dihydropyridine may be increased by dehydration of this intermediate, e.g. using a dehydrating agent such as trifluoroacetic anhydride in a solvent which is inert under the reaction conditions, eg methylene chloride.

Process b) may be carried out using a suitable oxidising agent, eg peracetic or metachloroperbenzoic acid. The reaction may be carried out in a suitable solvent, eg dichloromethane or a mixture of methanol and acetic acid.

Process c) is preferably carried out at a temperature of from about $-70^{o}$ to $100^{o}C$, and in a solvent which is inert under the reaction conditions, e.g. a halogenated hydrocarbon and preferably methylene chloride. The fluorinating agent is preferably a dialkylaminosulphur trifluoride, e.g. diethylaminosulphur trifluoride, or (2-chloro-1,1,2-trifluoroethyl)diethylamine. The group L may be, for example, $-OSO_2Rx$, where Rx is alkyl C1 to 6, e.g. methyl, or aryl, e.g. p-tolyl.

The hydrolysis of process d) may be carried out using

an aqueous acid, for example hydrochloric acid (eg 0.5 to 2.5 molar) in a water miscible organic solvent, eg acetone or tetrahydrofuran. The reaction may be carried out at a temperature of from about -10 to 50°C. The selective oxidation of process d) may be carried out using a suitable oxidising agent, e.g. pyridinium chlorochromate.

The hydrolysis of process e) may be carried out in a water miscible solvent, e.g. dioxan, at a temperature of from about 25° to 100°C. The group Z may be a halogen atom, e.g. fluorine. Surprisingly we find that fluorine is a good leaving group in this reaction.

The elimination of process f) may be carried out using a variety of dehydrating agents which will not adversely effect the other substituents in the molecule, e.g., an excess of acetic anhydride, thionyl chloride in ether or N,N'-dicylohexylcarbodiimide in pyridine. The group -OR may be, for example, a 2,4-dinitrophenoxy group. The reaction may be carried out at a temperature of from about 0° to 150°C depending on the reagent and solvent used. The oxime may, if desired, be formed <u>in situ</u> from the corresponding formyl compound using conventional methods known <u>per se</u>.

The reductive cleavage of process g) may be carried out chemically, eg using zinc and formic acid. The reaction may conveniently be carried out in a solvent

which is inert under the reaction conditions, eg acetonitrile. When process i) involves a hydrolysis the hydrolysis may be carried out using conventional techniques known per se.

Process h) may, when it involves an esterfication, be carried out using the appropriate alcohol, preferably in excess and in the presence of a dehydrating agent, eg dicyclohexylcarbodiimide, or by using the acid halide, imidazole or the mixed anhydride (which compounds may be prepared by conventional process known per se). Alternatively, the reaction may be affected by reacting a salt of the free acid with a compound $R_3L$ or $R_5L$ in which L is a good leaving group, eg halogen. The reaction may conveniently be carried out in a solvent which is inert under the reaction conditions, eg ethyl acetate. When a transesterification is involved the process may be carried out by treating the starting ester with the sodium alcoholate corresponding to the desired ester moiety.

The selective reduction of process i) may be carried out catalytically, e.g. using a precious metal catalyst.

In process j) the protecting group may be, for example, phenylalkyl C7 to 12, especially benzyl, haloalkanoyl C2 to 6, especially trifluoroacetyl or phthalimide. Other protecting groups are well known and include those described in Protective Groups in Organic

Chemistry, ed: J W F McOmie, Plenum Press (1973), and Protective Groups in Organic Synthesis, T W Greene, Wiley-Interscience (1981). Removal of the protecting groups depends on the nature of the protecting group; however conventional techniques may be generally employed, including acidic or basic cleavage or hydrogenolysis.

In process k) the good leaving group may be, for example, a p-toluenesulphonate group and the reaction may be carried out in the presence of a base or using a salt of the compound of formula XI or XII. The reaction may be carried out in a solvent which is inert under the reaction conditions, e.g. dimethyl formamide or dimethyl sulphoxide.

The selective hydrolysis of process l) may be carried out under acidic conditions, eg in the presence of aqueous HCl, and in a solvent which is inert under the reaction conditions, eg ethyl acetate. The reaction may be carried out at from about 0° to 40°C.

The reaction of process m) may be carried out using a suitable alkylating agent, eg an alkyl halide, preferably an alkyl iodide, at a temperature of from about 20° to 100°C in the presence of a suitable solvent, eg dimethylformamide or dimethyl sulphoxide. The reaction is preferably carried out in the presence of a base, eg potassium hydroxide or sodium hydride.

The reaction of process n) may be carried out at an elevated temperature, eg at reflux, in a solvent which is inert under the reaction conditions, eg an alkanol such as ethanol.

In process o) we prefer to use a symmetrical alkanedione. The reaction may be carried out in a solvent which is inert under the reaction conditions, eg methanol, at a temperature of from about -10 to +20°C.

The reaction of process p) may be carried out in the presence or absence of a solvent which is inert under the reaction conditions, eg chloroform, at a temperature of from about 0° to 120°C. The good leaving group may be a halogen atom, e.g. bromine.

Process q) may be carried out in a solvent which is inert under the reaction conditions, eg an alkanol such as isopropanol, at a temperature of from about 0° to 40°C.

The reaction of process r) may be carried out in a solvent which is inert under the reaction conditions, eg methanol and in the presence of a non-nucleophilic base.

The resolution of process s) may be carried out by means of conversion of the mixture to, when $R_3$ or $R_5$ is H, a salt with an optically active base or an ester with an optically active alcohol (eg $CCl_3(C_6H_5)CHOH$ or $C_6H_5(OCH_3)CHCH_2OH$), or, when $R_3$ or $R_5$ is aminoalkyl, a salt with an optically active acid and

separation of the product by selective crystallisation, or, preferably, by means of high performance liquid chromatography (HPLC). The separated product may then be converted to the desired optically active acid or ester by, for example, process g) or h).

The starting materials for the above processes are either known, or if they are not specifically known they may be made by processes described in the Examples, or they may be made from known compounds using one or more process steps which are known *per se* or are analogous to those described in the Examples.

Certain of the compounds of formula V are novel and the invention therefore also provides those compounds of formula V in which $R_4$ is a phenyl group carrying 3 or 4 substituents $R_4$ being substituted by $CF_3$, $XR_{22}$, nitro, halogen, CN, alkly C1 to 6, formyl, dioxalanyl, $-N(R_{23})COR_{24}$, $-CONR_{25}R_{26}$ or amino which is substituted by phenylsulphonyl or by trifluoroacetyl and $R_4$ carries a fluoro or $CF_3$ substituent on the atom adjacent to the atom through which $R_4$ is connected to the -CHO group, provided that $R_4$ is not 2,3-dichloro-6-fluorophenyl, 3-chloro-6-fluoro-2-(trifluoromethyl)phenyl or 2,3,5,6-tetrafluorophenyl.

Preferred aldehydes, and more specifically preferred

benzaldehydes, are those corresponding to the preferred values of $R_4$ described later in this specification.

The compounds of formula I and the intermediates therefor may be isolated from their reaction mixtures using conventional processes, eg crystallisation or chromatography.

Pharmaceutically acceptable salts of those compounds of formula I carrying basic groups include salts with organic or inorganic non toxic anions, e.g. the hydrochloride or maleate salts.

The compounds of formula I, and the pharmaceutically acceptable salts thereof, are useful because they exhibit pharmacological properties in animals. More particularly they block the entry of calcium into vascular and cardiac muscle leading to falls in blood pressure, inotropy and heart rate. They are active in the following systems:-

(a) Relaxation of contracted vascular smooth muscle. Van Breemen, Aaronson, Loutzenhiser and Meisheri, Chest, 78, Supplement, 157-165, 1980.

(b) Reduction of inotropy and chronotropy of isolated atria. Henry, Excerpta Med. Int. Congr. Ser., 474, 14-23, 1979.

(c) Reduction of blood pressure and increased cardiac output in anaesthetised dogs. Hirakawa, Ito, Kondo, Watanbe, Hiei, Banno & Hyase, Arzneim-Forsch, 22,

344-349, 1972.

(d) Reduction of blood pressure in conscious dogs when given by the intravenous and oral routes. Newman, Bishop, Peterson, Leroux & Horowitz, J Pharm. Exp. Ther. 201, 723-730, 1977.

The compounds are thus indicated for use in the treatment of renovascular, malignant or essential hypertension (including hypertensive emergencies), pulmonary hypertension, vasospastic angina, chronic stable angina and congestive heart failure. Other indications are the treatment of renal failure, cardiac arrhythmias, hypertrophic cardiomyopathy, cerebrovascular diseases (including cerebral haemorrhage, ischaemia and vasospasm, migraine, altitude sickness and hearing loss), peripheral vascular diseases (including Raynauds syndrome, intermittent claudication and digital ulceration); use as a cardioplegic agent during surgery eg in cardiopulmonary bypass, and for the treatment of, and protection against, myocardial infarction and ischaemia.

By virtue of their ability to inhibit calcium entry into other cells and tissues the compounds are also indicated in the treatment of thrombosis, atherosclerosis, respiratory diseases (including asthma and bronchitis) glaucoma, aldosteronism, uterine hypermotility and for the relief of oesophageal and skeletal muscle spasm.

For the above uses the dosage will depend upon the compound used, the route of administration and the effect desired, but in general will be in the range of 0.1-10mg per kilogram body weight per day. For man the indicated total daily dose will be from about 5-500mg, preferably from 5 to 200mg and more preferably from 5 to 100mg, which may be administered preferably once daily, or in divided doses of about 1-200mg, preferably 2 to 25mg, e.g. 2 to 4 times per day.

The compounds of formula I are also useful as intermediates for the production of other pharmacologically active compounds.

The compounds of formula I are advantageous in that they possess greater potency (e.g. with respect to hypotensive and direct negative chronotropic effects), produce a lower level of reflex tachycardia, are more selective (e.g. for vascular smooth muscle vs cardiac muscle), produce less depression of cardiac contractility, are longer acting, have a slower onset of action, are more readily absorbed or less readily metabolised, are more easily formulated, possess less, or less undesirable, side effects, are more stable or have other more beneficial properties than known compounds of similar structure.

The compounds of the invention may be administered by a wide variety of routes and may act systemically or

locally. Thus the compounds may be administered by oral or nasal inhalation to the lung, to the buccal cavity, oesophageally, rectally, topically to the skin, the eye or to other available surfaces of the body; by injection, eg intravenously, intramuscularly, intraperitoneally, or by surgical implant.

We prefer $R_1$ to be methyl or more preferably hydrogen.

$R_2$ may be CN, CHO or alkyl C1 to 6 optionally substituted by halogen, eg fluorine, or by hydroxy.

$R_2$ is preferably methyl or more preferably monofluoromethyl.

We prefer at least one of $R_3$ and $R_5$ (and preferably $R_3$) to be alkyl C1 to 6, e.g. methyl, ethyl, 1-methylethyl or 2-methylpropyl.

When $R_3$ or $R_5$ represents optionally substituted alkyl we prefer the alkyl to be C1 to 6 which may be straight or branched.

When $R_3$ or $R_5$ represents or contains a 4, 5 or 6 membered oxygen, nitrogen or $-S(O)_n-$ containing heterocyclic ring that ring is preferably a 3-thietanyl, a 3-thietanyl-S,S-dioxide, a 3-thietanyl-S-oxide, a 3-oxetanyl, a 4-tetrahydropyranyl or a 3-azetidinyl ring, which rings are optionally substituted, e.g. in the 3-position, by alkyl C1 to 6, e.g. methyl.

When $R_3$ or $R_5$ represents or contains a cycloalkyl or cycloalkenyl group that group preferably contains 3,4 or 5 carbon atoms and is optionally substituted by alkyl C1 to 6, e.g. methyl. When the cycloalkyl group is carried on an alkyl group we prefer that alkyl group to be methyl. We specifically provide two cycloalkyl groups carried on an alkyl (e.g. methyl) group. When the cycloalkyl is fused to a phenyl ring it may be 2,3-dihydro-2-idenyl, and when it is bridged it may be octahydro-endo-2-pentalenyl.

When $R_3$ or $R_5$ contains a group of formula II or III we prefer $R_{11}$ and $R_{12}$ to be methyl or phenyl. We also prefer the group of formula II or III to be carried on a hexamethylene group.

When $R_3$ or $R_5$ represents alkyl substituted by halogen the halogen may be chlorine or preferably fluorine.

When $R_3$ or $R_5$ is alkyl substituted by alkyl amino we prefer the alkylamino group to be an alkyl-(phenylalkyl)amino group, e.g. a methyl-(phenylmethyl)amino group.

$R_6$ may be alkyl C1 to 6 optionally interrupted by an oxygen atom and optionally substituted by halogen, a 2-amino-6-hydroxy-pyrimidin-4-yl group or by a group $-NR_{14}R_{15}$.

We prefer $R_{16}$, $R_{17}$ and $R_{18}$ each to be methyl.

$R_{19}$ is preferably hydrogen, p-halo-, eg p-chloro-

benzyl, $-CONHCH_3$ or 2-thiazolyl.

$R_6$ is preferably methyl, monofluoromethyl, (2-aminoethoxy)methyl or 4-aminobutyl.

We prefer $R_4$ to be of formula XIII,

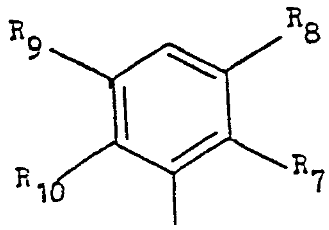

XIII

in which $R_7$ is trifluoromethyl, phenylthio, fluoro, phenylsulphonyl, nitro or chloro,

$R_8$ is nitrile, chloro, fluoro, phenylthio, methylthio, phenylsulphonylamino, trifluoroacetyl, trifluoromethyl, methoxy, propylthio, nitro or amino,

$R_9$ is hydrogen, fluoro or chloro, and

$R_{10}$ is hydrogen, fluoro, methoxy or chloro.

We particularly prefer that both of $R_7$ and $R_{10}$, and more preferably that all of $R_7$, $R_8$ and $R_{10}$, are other than hydrogen. Particularly favoured are compounds in which $R_7$ is trifluoromethyl, $R_8$ is chloro or nitrile, $R_9$ is hydrogen and $R_{10}$ is fluoro.

When $R_4$ is a heterocyclic group we prefer that group to be aromatic. We also prefer the heteroatom to be adjacent to the atom through which $R_4$ is connected to the remainder of the molecule.

An especially preferred group of compounds are those in which $R_1$ is hydrogen, $R_2$ is $-CH_2F$ or $CH_3$, $R_3$ is methyl, $R_5$ is 3-thietanyl, 1-methylethyl, cyclobutyl, 3-thietanyl-S,S-dioxide, 6-(5-phenyl-3-pyrazolyloxy)hexyl, cyclopropylmethyl, 6-((6-methyl-2-phenyl-4-pyrimidinyl)oxy) hexyl or ethyl, $R_6$ is methyl or (2-aminoethoxy)methyl and $R_4$ is 3-nitro-2-thienyl, or a group of formula XIII in which $R_7$ is $CF_3$, $R_8$ is Cl or CN, $R_9$ is hydrogen and $R_{10}$ is F.

A specific and characteristic feature of the invention is the substitution pattern around the group $R_4$.

According to the invention we also provide the following specific groups of compounds within the scope of formula I:-

a) $R_1$ is as defined with respect to formula I,

$R_2$ and $R_6$ are each alkyl C1 to 6,

$R_3$ and $R_5$ each represent straight, branched or cycloalkyl containing up to and including 5 carbon atoms,

$R_4$ is a group of formula XIII,

$R_7$ and $R_8$ each represent halogen, $-CF_3$, $-NO_2$ or -CN and one of $R_9$ and $R_{10}$ is fluorine and the other is hydrogen or fluorine.

b) $R_1$ is as defined with respect to formula I,

$R_2$ is alkyl C1 to 6 optionally substituted by

fluorine,

$R_3$ and $R_5$ are each alkyl C1 to 6 or cycloalkyl C4 to 8,

$R_4$ is a group of formula XIII,

$R_7$ and $R_8$ are each $-CF_3$, halogen, nitro or cyano,

$R_9$ is hydrogen,

$R_{10}$ is fluoro, and

$R_6$ is a group $-CH_2OCH_2CH_2NR_{14}R_{15}$,

c) $R_1$ is as defined with respect to formula I,

$R_2$ and $R_6$ are each alkyl C1 to 6 optionally substituted by fluorine,

$R_3$ and $R_4$ are as defined in b) above, and

$R_5$ is a C4 to 8 methylene chain substituted by a group of formula III,

d) $R_1$, $R_2$, $R_3$, $R_4$ and $R_6$ are as defined in c) above, and

$R_5$ is a C4 to 8 methylene chain substituted by a group of formula II in which $R_{11}$ is phenyl,

e) $R_1$ is as defined with respect to formula I,

$R_2$ and $R_6$ are each alkyl C1 to 6 optionally substituted by halogen,

$R_3$ and $R_5$ are as defined in b) above, and

$R_4$ is a group of formula XIV or XV,

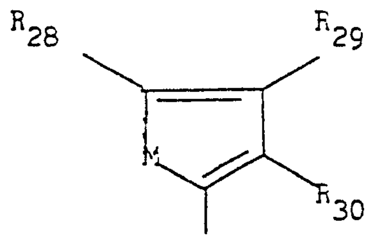

XIV

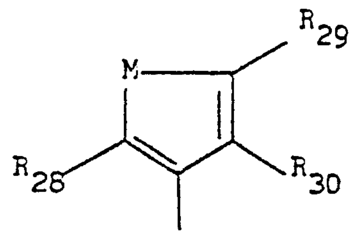

XV

in which M is O, S or $NR_{32}$,

$R_{32}$ is hydrogen or alkyl C1 to 6 or $S(O)_nR_{31}$,

$R_{28}$, $R_{29}$ and $R_{30}$ are each hydrogen, halogen, $NO_2$, $CF_3$, CN, alkyl C1 to 6 or $-S(O)_nR_{31}$,

n is 0, 1 or 2, and

$R_{31}$ is alkyl C1 to 6 or phenyl,

provided that

in formula XIV $R_{30}$ is other than hydrogen, or when M is $NR_{32}$ at least one of $R_{32}$ and $R_{30}$ is other than hydrogen, and

in formula XV at least one of $R_{28}$ and $R_{30}$ is other than hydrogen,

f) $R_1$ is as defined with respect to formula I,

$R_2$ is alkyl C1 to 6,

$R_6$ is alkyl C1 to 6 optionally substituted by one or more fluorine atoms,

$R_3$, $R_4$ and $R_5$ are as defined in b) above, save that one of $R_7$ and $R_8$ is $XR_{22}$, $-N(R_{23})COR_{24}$ or $-CONR_{25}R_{26}$,

g) $R_1$, $R_3$, $R_4$ and $R_5$ are as defined in b) above,

$R_2$ is alkyl C1 to 6, and

$R_6$ is $-CH_2OH$, -CN or -CHO, and

h) $R_1$ is hydrogen,

$R_2$ is methyl or monofluoromethyl,

$R_6$ is alkyl C1 to 6,

$R_4$ is a group of formula XIII in which $R_7$ is $CF_3$, $R_8$ is halo or -CN, $R_9$ is hydrogen and $R_{10}$ is fluoro,

one of $R_3$ and $R_5$ represents alkyl C1 to 6 and the other represents $-(CH_2)_p-(CHZ_1)_qZ_2$,

p is a whole number from 0 to 6 inclusive,

q is 0 or 1,

$Z_1$ is hydrogen or a 3 or 4 membered alicyclic or a 4 membered heterocyclic ring, which rings may contain a sulphoxide or sulphone group and are optionally substituted by alkyl C1 to 6,

$Z_2$ is a 3 or 4 membered alicyclic or a 4 membered heterocyclic ring, which rings may contain a sulphoxide or sulphone group and are optionally substituted by alkyl C1 to 6,

provided that when p and q are both 0 then $Z_2$ is a 4 membered sulphur containing heterocyclic ring.

According to our invention we also provide a pharmaceutical composition comprising preferably less than 80%, more preferably less than 50%, eg 1 to 20%, by weight of a compound of formula I, or a pharmaceutically

acceptable salt thereof, in admixture with a pharmaceutically acceptable adjuvant, diluent or carrier.

Thus the compound may be put up as a tablet, capsule, dragée, suppository, suspension, solution, injection, implant, a topical, eg transdermal, preparation such as a gel, cream, ointment, aerosol or a polymer system, or an inhalation form, e.g. an aerosol or a powder formulation.

We prefer compositions which are designed to be taken oesophageally and to release their contents in the gastrointestinal tract. Thus we prefer tablets which may, for example, be made by direct compression. In such a process the active ingredient is mixed with one or more of modified forms of starch, calcium phosphate, a sugar eg lactose, microcrystalline cellulose and/or other directly compressible excipients, together with lubricant(s), eg stearic acid or magnesium stearate, flow aid(s), eg talc or colloidal silicon dioxide, and disintegrant(s), eg starch, substituted sodium carboxymethyl cellulose, cross linked sodium carboxy methyl cellulose, carboxy methyl starch or cross linked polyvinylpyrrolidone. Tablets are then formed by direct compression, and may be sugar or film coated e.g. with hydroxypropylmethylcellulose.

Alternatively the active ingredient may be granulated before tabletting. In such cases the active ingredient is mixed with one or more of starch, calcium phosphate, a

sugar eg lactose, microcrystalline cellulose or other suitable excipients and granulated with a binder such as starch, pregelled starch, polyvinylpyrrolidone, gelatine, a modified gelatine, or a cellulose derivative, eg hydroxypropylmethylcellulose. The mass is then dried, sieved and mixed with lubricant(s), flow aid(s) and disintegrant(s), such as described in the previous paragraph. Tablets are then formed by compression of the granules, and may be sugar or film coated, eg with hydroxypropylmethylcellulose.

As a further alternative a powder, blend or granules, such as are described above as intermediates in tabletting, may be filled into a suitable, e.g. gelatine, capsule.

In order to improve the bioavailability, or decrease variability of availability, of the active ingredient the compound may be:-

a) dissolved in a suitable solvent, eg polyethylene glycol, Gelucaire, arachis oil, a (hydrogenated) vegetable oil or beeswax and the solution is then filled into a gelatine capsule,

b) produced as a spray-dried or freeze-dried form prior to mixing with other excipients,

c) milled and/or micronised to produce a powder with a large surface area prior to mixing with other excipients,

d) made into a solution and distributed over an inert excipient having a large surface area, eg colloidal silicon dioxide. The solvent is evaporated and further excipients added,

e) formed into a complex with cyclodextrin prior to mixing with other excipients. This complex also assists in increasing light stability, or

f) made into a solid solution or co-precipitated, eg with polyvinylpyrrolidone, polyethyleneglycol, modified cellulose, hydroxypropylmethylcellulose, urea or a sugar prior to mixing with further excipients.

The compounds, either in their normal form or in a modified form, eg as described immediately above, may be formulated in a controlled release form. Thus the compound may be dispersed, or contained in, a polymer matrix formed from, for example, ethylcellulose, hydroxypropylmethylcellulose or an acrylate/methacrylate polymer. Alternatively the compound may be formulated as a tablet or beads which are surrounded by a semi-permeable membrane, eg shellac, ethylcellulose or an acrylate/methacrylate polymer.

The compounds of this invention may be given in combination with other pharmaceutically active compounds, eg diuretics, beta-blockers, antihypertensives or inotropic agents. The dosage of the other

pharmaceutically active compound can be that conventionally used when the compound is administered on its own, but is preferably somewhat lower. To illustrate these combinations, a compound of this invention effective in the range, eg 5-100 milligrams per day, can be combined at levels ranging, eg from 1-200 milligrams per day with the following beta-blockers, antihypertensives and diuretics in dose ranges per day as indicated:

hydrochlorothiazide (15-200mg), chlorothiazide (125-2000mg), ethacrynic acid (15-100mg), amiloride (5-20mg), furosemide (5-80mg), propanolol (20-480mg), timolol (5-50mg), captopril (10-500mg), methyldopa (65-2000mg) or digoxin (0.1-0.5mg). In addition, the triple drug combinations of hydrochlorothiazide (15-200mg) plus amiloride (5-20mg) plus a compound of this invention (3-200mg) and hydrochlorothiazide (15-200mg) plus timolol (5-50mg) plus a compound of this invention (3-200mg), are provided. The above dose ranges may be adjusted on a unit basis as necessary to permit divided daily dosage. Also, the dose may vary depending on the severity of the disease, weight of patient and other factors which a person skilled in the art will recognise.

Certain of the compounds of formula I are assymetric and exhibit optical isomerism. Such compounds may be separated into their optical isomers using process s) or

may be made by stereospecific syntheses using conventional techniques know *per se*.

The invention therefore provides the compounds as their individual optical isomers and racemic or other mixtures of the individual isomers.

Certain of the compounds of the invention can form solvates, eg hydrates or alcoholates, and certain of the compounds are light sensitive and should therefore be produced, handled, stored and formulated in such a manner that they are not subjected to degrading amounts of light of the appropriate wavelengths.

The invention is illustrated, but in no way limited by the following Examples in which temperatures are in degrees centigrade.

Example 1

3-Methyl 5-(3-thietanyl) 4-(3-cyano-6-fluoro-2--(trifluoromethyl)phenyl)-2-(fluoromethyl)-1,4-dihydro-6-methyl-3,5-pyridinedicarboxylate

4-Fluoro-3-formyl-2-(trifluoromethyl)benzonitrile (1.6g, 7.4mmoles), methyl 4-fluoro-3-oxobutanoate (1.0g, 7.4mmoles) and 3-thietanyl 3-amino-2-butenoate (1.27g, 7.4mmoles) were heated in dry t-butanol (40ml) at 45$^{o}$ under nitrogen for 2 days. The solvent was evaporated and the residue, dissolved in dichloromethane (40ml), was treated with trifluoroacetic anhydride (0.3ml). After

10 minutes the solvent was evaporated and the residue was chromatographed on silica eluting with ethyl acetate/petroleum ether (60-80°). Crystallisation from 2-propanol/dichloromethane/petroleum ether (60-80°) gave the title compound (0.9g) as yellow crystals, m.p. 203-4°.

Example 2

Methyl 1-methylethyl 4-(3-chloro-6-fluoro-2-(trifluoromethyl)phenyl)-1,4-dihydro-2,6-dimethyl-3,5-pyridinedicarboxylate

A solution of 1-methylethyl 3-amino-2-butenoate (3g, 21mmole), methyl 3-oxobutanoate (2.26ml, 21mmole) and 3-chloro-6-fluoro-2-(trifluoromethyl)benzaldehyde (4.7g, 21mmole) in dry t-butanol (40ml) was heated at 60° for 36 hours. The cooled solution was concentrated *in vacuo*, and chromatographed on silica eluting with 2% ethyl acetate in dichloromethane.

Further purification by high pressure liquid chromatography on silica, eluting with a mixture of petroleum ether, dichloromethane and ethyl acetate (5:5:1), and recrystallisation from petroleum ether (60-80°)/acetone gave the title compound (0.8g), mp 154-6°.

Example 3

3-Methyl 5-(1-methylethyl) 2-(fluoromethyl)-1,4-dihydro-6-methyl-4-(3-nitro-2-thienyl)-3,5-pyridine-dicarboxylate

A mixture of 3-nitro-2-thiophenecarboxaldehyde (0.735g, 5mmoles), methyl 4-fluoro-3-oxobutanoate (0.67g, 5mmoles) and 1-methylethyl 3-amino-2-butenoate (0.715g, 5mmoles) was heated at 95° for 4 hours. The reaction mixture was chromatographed on silica eluting with ether/petroleum ether (60-80°), the product crystallised from petroleum ether (60-80°) and recrystallised from 2-propanol (0.15g) mp 123-4°.

Example 4

3-Methyl 5-(1-methylethyl) 4-(3-cyano-6-fluoro-2-(trifluoromethyl)phenyl)-2-(fluoromethyl)-1,4-dihydro-6-methyl-3,5-pyridinedicarboxylate

4-Fluoro-3-formyl-2-(trifluoromethyl)benzonitrile (1.57g), 7.23mmoles), 1-methylethyl 3-amino-2-butenoate (1.03g, 7.23mmoles) and methyl 4-fluoro-3-oxobutanoate (0.9g, 7.23mmoles) dissolved in t-butanol (50ml) were heated at 45° under nitrogen for 7 days. The solvent was evaporated and the residue chromatographed on silica eluting with ethyl acetate/petroleum ether (60-80°) mixtures. The title compound (0.74g) was obtained by recrystallisation from 2-propanol, mp 154-5°.

Example 5

5-Cyclobutyl 3-methyl 4-(3-cyano-6-fluoro-2-

(trifluoromethyl)phenyl)-2-(fluoromethyl)-1,4-dihydro-6-methyl-3,5-pyridinedicarboxylate

4-Fluoro-3-formyl-2-(trifluoromethyl)benzonitrile (1.1g, 5.1mmoles), cyclobutyl 3-amino-2-butenoate (0.79g, 5.1mmoles) and methyl 4-fluoro-3-oxobutanoate (0.67g, 5.1mmoles) dissolved in t-butanol (25ml) were heated at 45$^{o}$ under nitrogen for three days. The solvent was evaporated and the residue was dissolved in toluene (10ml). Trifluoroacetic anhydride (0.3ml) was added with stirring followed, five minutes later, by methanol (10ml). The solvent was evaporated and the residue crystallised from 2-propanol/dichloromethane. Further purification by chromatography on silica eluting with ethyl acetate/petroleum ether (66-80$^{o}$) mixtures followed by crystallisation from 2-propanol/petroleum ether (60-80$^{o}$) gave the title compound (0.56g) mp 188-9$^{o}$.

Example 6

3-Methyl 5-(3-thietanyl-S,S-dioxide) 4-(3-chloro--6-fluoro-2-(trifluoromethyl)phenyl)-2-(fluoromethyl)-1,4-dihydro-6-methyl-3,5-pyridinedicarboxylate

3-Thietanyl-S,S-dioxide 3-oxobutanoate (1.5g, 7.3mmoles) and ammonium acetate (0.56g, 7.3mmoles) were heated in t-butanol (25ml) at 55$^{o}$ for 1 hour. To this solution was added 3-chloro-6-fluoro-2-(trifluoromethyl)-benzaldehyde (1.65g, 7.3mmoles) and methyl 4-fluoro-3-oxobutanoate (0.98g, 7.3mmoles) and the reaction mixture was heated at 55$^{o}$ for 4 days. The solvent was evaporated and the residue chromatographed on silica eluting with ethyl acetate/petroleum ether (60-80$^{o}$). Crystallisation from ethyl acetate/petroleum ether (60-80$^{o}$) gave the title compound (0.65g) as an ethyl acetate complex (1:1), mp 110-112$^{o}$.

Example 7

3-Methyl 5-(6-(5-phenyl-3-pyrazolyloxy)hexyl) 4-(3-cyano-6-fluoro-2-(trifluoromethyl)phenyl)-2-(fluoromethyl)-1,4-dihydro-6-methyl-3,5-pyridinedicarboxylate

6-(5-Phenyl-3-pyrazolyloxy)hexyl) 3-oxobutanoate (1.31g, 3.8mmoles), ammonium acetate (0.29g, 3.8mmoles) and t-butanol (40ml) were heated at 50$^{o}$ for 6 hours. 4-Fluoro-3-formyl-2-(trifluoromethyl)benzonitrile (0.8g, 3.7mmoles) and methyl 4-fluoro-3-oxobutanoate (0.55g, 4.1mmoles) in t-butanol (35ml) were added and the heating continued at 50$^{o}$ for 2 days. The solvent was evaporated and the residue, dissolved in dichloromethane (50ml), was treated with trifluoroacetic anhydride (0.4ml) at room temperature. After 15 minutes, methanol (5ml) was added and then the solvent was evaporated. Purification by chromatography (x2) eluting with ethyl acetate/petroleum ether (60-80$^{o}$) and ethyl

acetate/dichloromethane followed by crystallisation from hexane gave the title compound (0.2g), mp 126-30°.

The method of Examples 1 and 5 was used to prepare the compounds of Examples 8 to 22.

Examples 8 - 22

8. 3-Methyl 5-(1-methylethyl) 4-(3,6-difluoro-2-(phenylthio)phenyl)-2-(fluoromethyl)-1,4-dihydro-6-methyl-3,5-pyridinedicarboxylate mp 93-5° (2-propanol).

9. 5-Cyclobutyl 3-methyl 4-(3,6-difluoro-2-(trifluoromethyl)phenyl)-2-(fluoromethyl)-1,4-dihydro-6-methyl-3,5-pyridinedicarboxylate mp 147-9° (cyclohexane).

10. 3-Methyl 5-(1-methylethyl) 4-(2,5-difluoro-3-(phenylthio)phenyl)-2-(fluoromethyl)-1,4-dihydro-6-methyl-3,5-pyridinedicarboxylate mp 149-51° (cyclohexane).

11. 5-Cyclopentyl 3-methyl 4-(3-cyano-6-fluoro-2-(trifluoromethyl)phenyl)-2-(fluoromethyl)-1,4-dihydro-6-methyl-3,5-pyridinedicarboxylate mp 154-6° (2-propanol/petroleum ether (60-80°)).

12. 5-(2-Cyanoethyl) 3-methyl 4-(3-cyano-6-fluoro-2-(trifluoromethyl)phenyl)-2-(fluoromethyl)-1,4-dihydro-6-methyl-3,5-pyridinedicarboxylate mp 165-6° (dichloromethane/2-propanol/cyclohexane).

13. 3-Methyl 5-(2-methylpropyl) 4-(3-cyano-6-fluoro-2-(trifluoromethyl)phenyl)-2-(fluoromethyl)-1,4-dihydro-6-methyl-3,5-pyridinedicarboxylate mp 151-2°

(cyclohexane).

14. 3-Methyl 5-(1-methylethyl) 2-(fluoromethyl)-4--(6-fluoro-3-(methylthio)-2-(trifluoromethyl)phenyl)-1,4--dihydro-6-methyl-3,5-pyridinedicarboxylate mp 111-12.5° (cyclohexane).

15. 3-Methyl 5-(1-methylethyl) 2-(fluoromethyl)-4-(6-fluoro-3-((phenylsulphonyl)amino)-2-(trifluoromethyl)-phenyl)-1,4-dihydro-6-methyl-3,5-pyridinedicarboxylate mp 132-4° (2-propanol/acetone/petroleum ether (60-80°)).

16. 3-Methyl 5-(1-methylethyl) 2-(fluoromethyl)-4--(6-fluoro-3-((2,2,2-trifluoroacetyl)amino)-2-(trifluoromethyl)phenyl)-1,4-dihydro-6-methyl-3,5-pyridinedicarboxylate mp 197-8° (2-propanol).

17. 3-Methyl 5-(1-methylethyl) 4-(6-fluoro-2,3-bis-(trifluoromethyl)phenyl)-2-(fluoromethyl)-1,4-dihydro-6-methyl-3,5-pyridinedicarboxylate mp 112-3° (petroleum ether (60-80°)).

18. 3-Methyl 5-(1-methylethyl) 4-(6-fluoro-3-methoxy-2-(trifluoromethyl)phenyl)-2-(fluoromethyl)-1,4-dihydro-6-methyl-3,5-pyridinedicarboxylate mp 159-60° (petroleum ether (60-80°)).

19. 3-Methyl 5-(1-methylethyl) 2-(fluoromethyl)-4-(6-fluoro-3-(propylthio)-2-(trifluoromethyl)phenyl)-1,4-dihydro-6-methyl-3,5-pyridinedicarboxylate mp 124-6°

(hexane).

20. 5-Cyclobutyl 3-methyl 4-(2,6-difluoro-3-nitrophenyl)-2-(fluoromethyl)-1,4-dihydro-6-methyl-3,5-pyridinedicarboxylate mp 142-3° (dichloromethane/2-propanol/petroleum ether (60-80°)).

21. 3-Methyl 5-(1-methylethyl) 4-(3-cyano-2,6-difluorophenyl)-2-(fluoromethyl)-1,4-dihydro-6-methyl-3,5-pyridinedicarboxylate mp 144-6° (2-propanol/petroleum ether (60-80°)).

22. 3-Methyl 5-(1-methylethyl) 4-(3-chloro-6-methoxy-2-(trifluoromethyl)phenyl)-2-(fluoromethyl)-1,4-dihydro-6-methyl-3,5-pyridinedicarboxylate mp 150-1° (2-propanol/petroleum ether (60-80°)).

The method of Examples 2 and 4 was used to prepare the compounds of Examples 23 to 35.

23. Dimethyl 1,4-dihydro-2,6-dimethyl-4-(2,3,5,6-tetrafluorophenyl)-3,5-pyridinedicarboxylate mp 178-9° (2-propanol).

24. Dimethyl 4-(3-chloro-6-fluoro-2-(trifluoromethyl)-phenyl)-1,4-dihydro-2,6-dimethyl-3,5-pyridinedicarboxylate mp 243° (acetone/petroleum ether (60-80°)).

25. 3-Methyl 5-(1-methylethyl) 2-(fluoromethyl)-1,4-dihydro-6-methyl-4-(2,4,5-trichloro-3-thienyl)-3,5-pyridine-dicarboxylate mp 102-4° (petroleum ether (60-80°)).

26. 3-Methyl 5-(1-methylethyl) 4-(3,6-difluoro-2-(phenylsulphonyl)phenyl)-2-(fluoromethyl)-1,4-dihydro-6-methyl-3,5-pyridinedicarboxylate mp 192-3° (t-butanol).

27. Di(1-methylethyl) 4-(3-chloro-6-fluoro-2-(trifluoromethyl)phenyl)-1,4-dihydro-2,6-dimethyl-3,5-pyridinedicarboxylate mp 70° (petroleum ether (60-80°)).

28. 3-Methyl 5-(1-methylethyl) 2-(fluoromethyl)-1,4-dihydro-6-methyl-4-(1-methyl-3-pyrryl)-3,5-pyridinedicarboxylate mp 63-5° (2-propanol/hexane).

29. 3-Methyl 5-(3-thietanyl) 4-(3,6-difluoro-2-(trifluoromethyl)phenyl)-2-(fluoromethyl)-1,4-dihydro-6-methyl-3,5-pyridinedicarboxylate mp 148-9° (cyclohexane).

30. 3-Methyl 5-((3-methyl-3-thietanyl)methyl) 4-(3-cyano-6-fluoro-2-(trifluoromethyl)phenyl)-2-(fluoromethyl)-1,4-dihydro-6-methyl-3,5-pyridinedicarboxylate mp 176-7° (ethyl acetate/hexane).

31. 3-Methyl 5-(1-methylethyl) 4-(3-(1,3-dioxolan-2-yl)-2-thienyl)-2-(fluoromethyl)-1,4-dihydro-6-methyl-3,5-pyridinedicarboxylate mp 114-6° (petroleum ether (60-80°)).

32. 3-Methyl 5-(1-methylethyl) 4-(2,6-difluoro-3-nitrophenyl)-2-(fluoromethyl)-1,4-dihydro-6-methyl-3,5-pyridinedicarboxylate mp 134-6° (petroleum ether (60-80°)).

33. 3-Methyl 5-(1-methylethyl) 4-(3-chloro-6-fluoro-2-nitrophenyl)-2-(fluoromethyl)-1,4-dihydro-6-methyl-3,5-pyridinedicarboxylate mp 123-5° (acetone/petroleum ether (60-80°)).

34. 3-Methyl 5-(1-methylethyl) 4-(3,5-dichloro-2-fluoro-6-(trifluoromethyl)phenyl)-2-(fluoromethyl)-1,4-dihydro-6-methyl-3,5-pyridinedicarboxylate mp 174-5° (t-butanol).

35. 3-Methyl 5-(1-methylethyl) 4-(3,6-dichloro-2-(trifluormethyl)phenyl)-2-(fluoromethyl)-1,4-dihydro-6-methyl-3,5-pyridinedicarboxylate $M^+$, 483/5/7; nmr ($CDCl_3$) delta 3.6 (S,3H) and 2.4 (S,3H).

The method of Example 3 was used to prepare the compounds of Examples 36 to 45.

36. Diethyl 4-(2,3-dichloro-6-fluorophenyl)-1,4-dihydro-2,6-dimethyl-3,5-pyridinedicarboxylate mp 144-6° softens at 139° (petroleum ether (60-80°)).

37. Diethyl 4-(3-chloro-6-fluoro-2-(trifluoromethyl)-phenyl)-1,4-dihydro-2,6-dimethyl-3,5-pyridinedicarboxylate mp 135-7° (petroleum ether (60-80°)).

38. 3-Methyl 5-(1-methylethyl) 4-(4,5-dichloro-3-thienyl)-2-(fluoromethyl)-1,4-dihydro-6-methyl-3,5-pyridinedicarboxylate mp 121-2° (2-propanol).

39. 3-Methyl 5-(1-methylethyl) 2-(fluoromethyl)-1,4-dihydro-6-methyl-4-(3-methyl-2-thienyl)-3,5-pyridinedicarboxylate mp 96-8° (2-propanol).

40. 3-Methyl 5-(1-methylethyl) 4-(3,6-difluoro-2--(trifluoromethyl)phenyl)-2-(fluoromethyl)-1,4-dihydro-6-methyl-3,5-pyridinedicarboxylate mp 125-6° (hexane).

41. 3-Methyl 5-(1-methylethyl) 2-(fluoromethyl)-4-(6-fluoro-3-nitro-2-(trifluoromethyl)phenyl)-1,4-dihydro-6--methyl-3,5-pyridinedicarboxylate mp 184-5° (2-propanol/hexane).

42. 3-Methyl 5-(1-methylethyl) 4-(2,5-difluoro-3-(trifluoromethyl)phenyl)-2-(fluoromethyl)-1,4-dihydro-6-methyl-3,5-pyridinedicarboxylate mp 143-5° (hexane).

43. 3-Methyl 5-(1-methylethyl) 4-(2-chloro-6-fluoro-3-nitrophenyl)-2-(fluoromethyl)-1,4-dihydro-6-methyl-3,5-pyridinedicarboxylate mp 159-61° (acetone/petroleum ether (60-80°)).

44. 3-Methyl 5-(1-methylethyl) 2-(fluoromethyl)-1,4-dihydro-6-methyl-4-(2,3,6-trifluorophenyl)-3,5-pyridinedicarboxylate mp 111-3° (petroleum ether (60-80°)).

45. 3-Methyl 5-(1-methylethyl) 4-(2,5-dichloro-3-(trifluoromethyl)phenyl)-2-(fluoromethyl)-1,4-dihydro-6-methyl-3,5-pyridinedicarboxylate mp 158-9° (petroleum ether (60-80°)).

The method of Example 6 was used to prepare the compounds of Examples 46 to 50.

46. 3-Methyl 5-((3-methyl-3-oxetanyl)methyl) 4-(3-chloro

-6-fluoro-2-(trifluoromethyl)phenyl)-2-(fluoromethyl) -1,4-dihydro-6-methyl-3,5-pyridinedicarboxylate mp 170-1° (2-propanol).

47. 5-(Dicyclopropylmethyl) 3-methyl 4-(3-chloro-6-fluoro -2-(trifluoromethyl)phenyl)-2-(fluoromethyl)-1,4-dihydro-6-methyl-3,5-pyridinedicarboxylate mp 121-2.5° (petroleum ether (40-60°)).

48. 3-Methyl 5-((3-methyl-3-oxetanyl)methyl) 4-(3-cyano-6-fluoro-2-(trifluoromethyl)phenyl)-2-(fluoromethyl)-1,4-dihydro-6-methyl-3,5-pyridinedicarboxylate mp 198-200° (2-propanol/petroleum ether (60-80°)).

49. 3-Methyl 5-(3-oxetanyl) 4-(3-cyano-6-fluoro-2-(trifluoromethyl)phenyl)-2-(fluoromethyl)-1,4-dihydro-6-methyl-3,5-pyridinedicarboxylate mp 209-10° (2-propanol/acetone/dichloromethane).

50. (E) 5-(4-Cyclopropyl-3-butenyl) 3-methyl 4-(3-chloro -6-fluoro-2-(trifluoromethyl)phenyl)-2-(fluoromethyl)-1,4-dihydro-6-methyl-3,5-pyridinedicarboxylate mp 110-2° (petroleum ether (40-60°)).

The method of Example 7 was used to prepare the compound of Example 51.

51. 3-Methyl 5-(3-oxetanyl) 4-(3,6-difluoro-2-(trifluoromethyl)phenyl)-2-(fluoromethyl)-1,4-dihydro-6-methyl-3,5-pyridinedicarboxylate mp 163-4° (petroleum ether (60-80°)).

Example 52

Ethyl methyl 4-(2,3-dichloro-6-fluorophenyl)-1,4-dihydro-2,6-dimethyl-3,5-pyridinedicarboxylate

A mixture of 2,3-dichloro-6-fluorobenzaldehyde (1.93g, 10mmoles), ethyl 3-oxobutanoate (1.27ml, 10mmoles), piperidine (0.01ml) and hexanoic acid (0.03ml) were heated at reflux in benzene (150ml) in a Dean and Stark apparatus. After 2 and 4 hours, piperidine (0.01ml) and hexanoic acid (0.03ml) were added and the reaction continued a further 30 minutes. The cooled solution was washed with brine, saturated sodium bicarbonate and brine, dried ($Na_2SO_4$) and the solvent removed in vacuo to leave ethyl 2-((2,3-dichloro-6-fluorophenyl)methylene)-3-oxobutanoate (3.1g) as a yellow oil. This oil was dissolved in t-butanol (12ml) and methyl 3-amino-2-butenoate (1.15g, 10mmoles) was added. After 6 days at room temperature, the solvent was removed and the residue chromatographed on silica eluting with ethyl acetate/ 60-80$^{o}$ petroleum ether. Recrystallisation from 2-propanol gave the title compound (1.0g) mp 167-8$^{o}$.

Example 53

3-Methyl 5-(3-thietanyl) 4-(3-chloro-6-fluoro-2-(trifluoromethyl)phenyl)-2-(fluoromethyl)-1,4-dihydro-6-methyl-3,5-pyridinedicarboxylate

Phosphorous pentachloride (2.1g, 0.011mmoles) was

added with stirring to 4-(3-chloro-6-fluoro-2-(trifluoromethyl)phenyl)-6-(fluoromethyl)-1,4-dihydro-5-(methoxycarbonyl)-2-methyl-3-pyridinecarboxylic acid (4.25g, 0.01mmoles) in methylene chloride (250ml). After four hours, 3-thietanol (1.8g, 0.02mmoles) was added and the reaction mixture stirred for 16 hours. Aqueous sodium carbonate was added with stirring; after two hours the organic layer was separated, washed with brine and dried ($MgSO_4$). The solvent was evaporated and the residue was titurated with petroleum ether (60-80°) and the resulting solid was recrystallised for petroleum ether (60-80°) to give the title compound (0.21g) mp 137-9°.

Example 54

3-Methyl 5-(3-thietanyl) 4-(3-cyano-6-fluoro-2-(trifluoromethyl)phenyl)-2-(fluoromethyl)-1,4-dihydro-6-methyl-3,5-pyridinedicarboxylate

Phosphorous pentachloride (0.5g, 2.4mmoles) was added with stirring to 4-(3-cyano-6-fluoro-2-(trifluoromethyl)-phenyl)-6-(fluoromethyl)-1,4-dihydro-5-(methoxycarbonyl)-2-methyl-3-pyridinecarboxylic acid (1g, 2.4mmoles) in methylene chloride (80ml). After stirring for three hours, 3-thietanol (0.432g) was added and the mixture stirred for 2.5 hours. Aqueous sodium carbonate was added with stirring; after one hour the organic layer was separated, washed with brine and dried ($MgSO_4$). The

solvent was evaporated and the residue was purified by chromatography on silica eluting with ethyl acetate/ petroleum ether (60-80°) mixtures. Crystallisation from acetone/petroleum ether (60-80°) gave the title compound (0.27g) mp 207-8°.

Example 55

5-(Cyclopropylmethyl) 3-methyl 4-(3-cyano-6-fluoro-2-(trifluoromethyl)phenyl)-2-(fluoromethyl)-1,4-dihydro-6-methyl-3,5-pyridinedicarboxylate

Phosphorous pentachloride (0.5g, 2.4mmoles) was added with stirring to 4-(3-cyano-6-fluoro-2-(trifluoromethyl)-phenyl)-6-(fluoromethyl)-1,4-dihydro-5-(methoxycarbonyl) -2-methyl-3-pyridinecarboxylic acid (1.0g, 2.4mmoles) in dry dichloromethane (30ml). After stirring for one hour, cyclopropylmethanol (0.25g, 3.6mmoles) was added and the mixture stirred for four hours. The reaction mixture was poured onto aqueous sodium carbonate and, after stirring for 10 minutes, the organic layer was separated and dried ($MgSO_4$). The solvent was evaporated and the residue crystallised from 2-propanol/methylene chloride to give the title compound (0.58g) mp 184-5°.

The compounds of Examples 56 to 60 were prepared using appropriate starting materials and the method described in Examples 53-55.

56. 5-(Cyclobutylmethyl) 3-methyl 4-(3-chloro-6-fluoro-2-

(trifluoromethyl)phenyl)-2-(fluoromethyl)-1,4-dihydro-6-methyl-3,5-pyridinedicarboxylate mp 169-70° (2-propanol/cyclohexane).

57. 5-(Cyclopropylmethyl) 3-methyl 4-(3-chloro-6-fluoro-2-(trifluoromethyl)phenyl)-2-(fluoromethyl)-1,4-dihydro-6-methyl-3,5-pyridinedicarboxylate mp 160-1° (2-propanol/cyclohexane).

58. 5-(Cyclopropylmethyl) 3-methyl 4-(3,6-difluoro-2-(trifluoromethyl)phenyl)-2-(fluoromethyl)-1,4-dihydro-6-methyl-3,5-pyridinedicarboxylate mp 140-1° (cyclohexane).

59. 3-Methyl 5-((1-methylcyclopropyl)methyl) 4-(3-cyano-6-fluoro-2-(trifluoromethyl)phenyl)-2-(fluoromethyl)-1,4-dihydro-6-methyl-3,5-pyridinedicarboxylate mp 157-8° (2-propanol/dichloromethane/petroleum ether (60-80°)).

60. 3-Methyl 5-(2-(methyl(phenylmethyl)amino)ethyl) 4-(3-chloro-6-fluoro-2-(trifluoromethyl)phenyl)-2-(fluoromethyl)-1,4-dihydro-6-methyl-3,5-pyridinedicarboxylate mp 110-1° (2-propanol/hexane).

Example 61

3-Methyl 5-(6-(5-phenyl-3-pyrazolyloxy)hexyl) 4-(3-chloro-6-fluoro-2-(trifluoromethyl)phenyl)-1,4-dihydro-2,6-dimethyl-3,5-pyridinedicarboxylate

3-Methyl 5-(6-(5-phenyl-3-pyrazolyloxy)hexyl) 4-(3-chloro-6-fluoro-2-(trifluoromethyl)phenyl)-2-

(fluoromethyl)-1,4-dihydro-6-methyl-3,5-pyridinedicarboxylate (1.05g, 1.57mmoles) was hydrogenated at 1 atm. at room temperature over 5% Pd/C (0.5g) in ethanol (35ml). The catalyst was filtered off and the solvent was evaporated. Purification by chromatography on silica eluting with ethyl acetate/hexane mixtures followed by crystallisation from ethyl acetate/cyclohexane gave the title compound (0.47g) mp 194-5°.

The compounds of Examples 62 to 68 were prepared using appropriate starting materials and the method described in Example 61.

62. 3-Methyl 5-(6-(5-phenyl-3-pyrazolyloxy)hexyl) 4-(3-cyano-6-fluoro-2-(trifluoromethyl)phenyl)-1,4-dihydro-2,6-dimethyl-3,5-pyridinedicarboxylate mp 175-7° (ethyl acetate/cyclohexane/hexane).

63. 3-Methyl 5-(1-methylethyl) 4-(3-cyano-6-fluoro-2-(trifluoromethyl)phenyl)-1,4-dihydro-2,6-dimethyl-3,5-pyridinedicarboxylate mp 188-9° (petroleum ether (60-80°)).

64. 5-Cyclobutyl 3-methyl 4-(3,6-difluoro-2-(trifluoromethyl)phenyl)-1,4-dihydro-2,6-dimethyl-3,5-pyridinedicarboxylate mp 181-3° (2-propanol/petroleum ether (60-80°)).

65. 5-Cyclobutyl 3-methyl 4-(3-cyano-6-fluoro-2-(trifluoromethyl)phenyl)-1,4-dihydro-2,6-dimethyl-3,5-pyridinedicarboxylate mp 204-5° (2-propanol/petroleum ether (60-80°)).

66. 3-Methyl 5-(6-(5-phenyl-3-pyrazolyloxy)hexyl) 4-(3,6-difluoro-2-(trifluoromethyl)phenyl)-1,4-dihydro-2,6-dimethyl-3,5-pyridinedicarboxylate mp 154-6° (ethyl acetate/cyclohexane).

67. 3-Methyl 5-(2-(methyl(phenylmethyl)amino)ethyl) 4-(3-chloro-6-fluoro-2-(trifluoromethyl)phenyl)-1,4-dihydro-2,6-dimethyl-3,5-pyridinedicarboxylate mp 60-2° (2-propanol/hexane).

68. 3-Methyl 5-(6-(5-methyl-3-pyrazolyloxy)hexyl) 4-(3-chloro-6-fluoro-2-(trifluoromethyl)phenyl)-1,4-dihydro-2,6-dimethyl-3,5-pyridinedicarboxylate mp 158-60° (ethyl acetate/cyclohexane/hexane).

Example 69

3-Methyl 5-(1-methylethyl) 4-(3-chloro-6-fluoro-2-(trifluoromethyl)phenyl)-1,4-dihydro-2-(hydroxymethyl)-6-methyl-3,5-pyridinedicarboxylate

3-Methyl 5-(1-methylethyl) 4-(3-chloro-6-fluoro-2-(trifluoromethyl)phenyl)-2-(fluoromethyl)-1,4-dihydro-6-methyl-3,5-pyridinedicarboxylate (2.0g) in dioxan (140ml) and aqueous pH7 buffer (70ml of a solution of disodium hydrogen phosphate (23.3g) and citric acid (3.46g) in water (1000ml)) were heated at reflux for 36 hours. The cooled solution was poured onto ethyl acetate/water. The

organic layer was separated, dried ($MgSO_4$) and the solvent evaporated. Tituration with dichloromethane at 0° and filtration gave 1-methylethyl 4-(3-chloro-6-fluoro-2-(trifluoromethyl)phenyl)-1,4,5,7-tetrahydro-2-methyl-5-oxofuro[3,4-b]pyridine-3-carboxylate (0.55g) mp greater than 250°. The dichloromethane filtrant was evaporated to dryness and the residue recrystallised from acetone/petroleum ether (60-80°) to give the title alcohol (1.2g) mp 127-9°.

Example 70

3-Methyl 5-(1-methylethyl) 4-(3-chloro-6-fluoro-2-(trifluoromethyl)phenyl)-2-formyl-1,4-dihydro-6-methyl-3,5-pyridinedicarboxylate

3-Methyl 5-(1-methylethyl) 4-(3-chloro-6-fluoro-2-(trifluoromethyl)phenyl)-1,4-dihydro-2-(hydroxymethyl)-6-methyl-3,5-pyridinedicarboxylate (1.2g, 2.6mmoles) in dichloromethane (20ml) was added to pyridinium chlorochromate (0.84g, 3.9mmoles) in dichloromethane (80ml). The mixture was stirred for 1.75 hours and then ether (100ml) was added. The solid was filtered off and the filtrate was run through an activated magnesium silicate column washing well with ether. The dichloromethane/ether was evaporated and the residue chromatographed on silica eluting with ethyl acetate/petroleum ether (60-80°) mixtures. The title

compound (0.16g) was obtained after crystallisation. mp 118-9° (petroleum ether (60-80°)).

Example 71

3-Methyl 5-(3-thietanyl-S,S-dioxide) 4-(3-chloro-6-fluoro-2-(trifluoromethyl)phenyl)-2-(fluoromethyl)-1,4-dihydro-6-methyl-3,5-pyridinedicarboxylate

3-Chloroperbenzoic acid (0.39g, 2.3mmoles) was added to 3-methyl 5-thietanyl 4-(3-chloro-6-fluoro-2-(trifluoromethyl)phenyl)-2-(fluoromethyl)-1,4-dihydro-6-methyl-3,5-pyridinedicarboxylate (0.25g, 0.5mmoles) in dichloromethane (25ml). After stirring for 16 hours, saturated aqueous sodium bicarbonate solution was added. After 15 minutes, the organic layer was separated, washed with sodium metabisulphite solution and brine, dried ($MgSO_4$) and the solvent removed. Chromatography on silica eluting with ethyl acetate/petroleum ether (bp 60-80°) mixtures and crystallisation from acetone/petroleum ether (bp 60-80°) gave the title sulphone (0.03g). mp 174.5-176°.

Example 72

3-Methyl 5-(cis-3-thietanyl-S-oxide) 4-(3-chloro-6-fluoro-2-(trifluoromethyl)phenyl)-2-(fluoromethyl)-1,4-dihydro-6-methyl-3,5-pyridinedicarboxylate and 3-methyl 5-(trans-3-thietanyl-S-oxide) 4-(3-chloro-6-fluoro-2-(trifluoromethyl)phenyl)-2-(fluoromethyl)-1,4-dihydro-6-

methyl-3,5-pyridinedicarboxylate

3-Chloroperbenzoic acid (0.34g, 2mmoles) was added with stirring at 0° to a solution of 3-methyl 5-thietanyl 4-(3-chloro-6-fluoro-2-(trifluoromethyl) phenyl)-2-(fluoromethyl)-1,4-dihydro-6-methyl-3,5-pyridinedicarboxylate (0.6g, 1.2mmoles) in dichloromethane (50ml). The reaction was monitored by thin layer chromatography and, when no starting thietanyl ester remained, saturated aqueous sodium bicarbonate was added. After 30 minutes the organic layer was separated, washed with sodium bisulphite solution and brine, dried ($MgSO_4$) and the solvent was evaporated. Separation of isomers was effected using HPLC eluting with tetrahydrofuran/hexane mixtures. The first eluted isomer was recrystallised from acetone/petroleum ether (60-80°). It crystallised with 0.5mmole of acetone (0.104g) mp 179-81° and the stereochemistry was assigned as "cis". The second isomer was recrystallised from acetone/petroleum ether (60-80°). It crystallised with 0.5mmole of acetone (0.24g) mp 168-70° and the stereochemistry was assigned as "trans".

Example 73

3-Methyl 5-(1-methylethyl) 4-(3-chloro-6-fluoro-2-(trifluoromethyl)phenyl)-2-cyano-1,4-dihydro-6-methyl -3,5-pyridinedicarboxylate

3-Methyl 5-(1-methylethyl) 4-(3-chloro-6-fluoro-2-(trifluoromethyl)phenyl)-2-formyl-1,4-dihydro-6-methyl-3,5-pyridinedicarboxylate (0.46g, 1mmole), hydroxylamine hydrochloride (0.08g, 1.15mmoles) and sodium acetate (0.16g) in acetic acid (10ml) was stirred at room temperature for 1.5 hours. The solution was poured into water/dichloromethane. The organic layer was separated, washed with sodium bicarbonate solution and water, dried ($Na_2SO_4$) and the solvent evaporated. Tituration with petroleum ether (60-80°) gave 3-methyl 5-(1-methylethyl) 4-(3-chloro-6-fluoro-2-(trifluoromethyl) phenyl)-1,4-dihydro-2-(hydroxyiminomethyl)-6-methyl-3,5-pyridinedicarboxylate (0.31g) mp 155-9°.

This oxime (0.3g, 0.65mmoles) and N-(trifluoroacetyl)-imidazole (0.11g, 0.67mmoles) in dry tetrahydrofuran (5ml) were heated at reflux for 1.5 hours. The solvent was evaporated and the residue titurated with ether. The solid was filtered off and the filtrate evaporated to dryness. Recrystallisation from ethyl acetate/petroleum ether (60-80°) gave the title compound (0.095g) mp 212-4°.

Example 74

3-Methyl 5-(1-methylethyl) 4-(3-cyano-2-thienyl)-2-(fluoromethyl)-1,4-dihydro-6-methyl-3,5-pyridinedicarboxylate

Prepared by the method of Example 73 using the product of Example 81. mp 145-6° (acetone/petroleum ether (60-80°)).

Example 75

3-Methyl 5-(6-(5-phenyl-3-pyrazolyloxy)hexyl) 4-(3-chloro-6-fluoro-2-(trifluoromethyl)phenyl)-2-(fluoromethyl)-1,4-dihydro-6-methyl-3,5-pyridinedicarboxylate

2,4-Dihydro-5-phenyl-3H-pyrazol-3-one (0.4g, 2.5mmoles) was added to a stirred solution of sodium hydride (0.12g of 50%, 2.5mmoles) in dimethylsulphoxide (8ml). After 45 minutes, 3-methyl 5-(6-(((4-methylphenyl) sulphonyl)oxy)hexyl) 4-(3-chloro-6-fluoro-2-(trifluoromethyl)phenyl)-2-(fluoromethyl)-1,4-dihydro-6-methyl-3,5-pyridinedicarboxylate (1.7g, 2.5mmoles) in dimethylsulphoxide (5ml) was added and the resulting solution was stirred for 7 hours. The reaction mixture was poured onto dilute hydrochloric acid and extracted with methylene chloride. The organic extract was washed with water and brine, dried ($MgSO_4$) and the solvent was evaporated. Purification by chromatography on silica eluting with tetrahydrofuran/hexane mixtures followed by crystallisation from 2-propanol/hexane gave the title compound (0.98g) mp 141-2°.

Example 76

3-Methyl 5-(6-((6-methyl-2-phenyl-4-pyrimidinyl)oxy) hexyl) 4-(3-chloro-6-fluoro-2-(trifluoromethyl)phenyl)-2-(fluoromethyl)-1,4-dihydro-6-methyl-3,5-pyridine-dicarboxylate hydrochloride hemietherate

Sodium hydride (0.13g of 50%, 2.5mmoles) was added to a stirred solution of 6-methyl-2-phenyl-4-pyrimidinol (0.47g, 2.5mmoles) in dimethylsulphoxide (9ml). After 1 hour, 3-methyl 5-(6-(((4-methylphenyl)sulphonyl)oxy) hexyl) 4-(3-chloro-6-fluoro-2-(trifluoromethyl)phenyl)-2-(fluoromethyl)-1,4-dihydro-6-methyl-3,5-pyridine-dicarboxylate (1.7g, 2.5mmoles) in dimethylsulphoxide (2ml) was added and the resulting solution was stirred for 16 hours. Water was added and the product extracted with ethyl acetate. The organic extract was washed with water (x2) and brine, dried ($Na_2SO_4$) and the solvent evaporated. Chromatograhy on silica eluting with ethyl acetate/petroleum ether (60-80$^o$) gave the free base of the title compound (0.6g). The hydrochloride salt was formed in 2-propanol/ethyl acetate and the solvent evaporated. Trituration of the residue with ether gave the title compound (0.52g) mp 113-5$^o$.

Example 77

3-Methyl 5-(6-(5-phenyl-3-pyrazolyloxy)hexyl) 4-(3,6-difluoro-2-(trifluoromethyl)phenyl)-2-(fluoromethyl) -1,4-dihydro-6-methyl-3,5-pyridinedicarboxylate

2,4-Dihydro-5-phenyl-3H-pyrazol-3-one (0.723g, 4.5mmoles) was added to a stirred solution of sodium hydride (0.217 of 50%, 4.5mmoles) in dimethylsulphoxide (60ml). After 1 hour, 3-methyl 5-(6-(((4-methylphenyl) sulphonyl)oxy)hexyl) 4-(3,6-difluoro-2-(trifluoromethyl) phenyl)-2-(fluoromethyl)-1,4-dihydro-6-methyl-3,5-pyridinedicarboxylate (3.0g, 4.5mmoles) in dimethylsulphoxide (5ml) was added and the resulting solution was stirred for 15 hours. The reaction mixture was poured onto dilute hydrochloric acid and extracted with ethyl acetate (x3). The combined organic extracts were washed with water and brine, dried ($MgSO_4$) and the solvent evaporated. Purification by chromotography on silica twice eluting with ethyl acetate/hexane and then dichloromethane/ethyl acetate mixtures followed by crystallisation from ethyl acetate/hexane gave the title compound. mp 126-8°.

Example 78

3-Methyl 5-(6-(5-methyl-3-pyrazolyloxy)hexyl) 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 75-77. mp 128-30° (ethyl acetate/cyclohexane).

Example 79

3-Methyl 5-(1-methylethyl) 4-(3,6-difluoro-2-(trifluoromethyl)phenyl)-2-formyl-1,4-dihydro-6-methyl-3,5-pyridinedicarboxylate

Methyl 4,4-dimethoxy-3-oxobutanoate (6.5g, 3.7mmoles) and ammonium acetate (2.84g, 3.7mmoles) in t-butanol (60ml) was heated at 55° for 6 hours to give methyl 3-amino-4,4-dimethoxy-2-butenoate. 3,6-Difluoro-2-(trifluoromethyl)benzaldehyde (7.75g, 3.7mmoles) and 1-methylethyl 3-oxobutanoate (5.32g, 3.7mmoles) in t-butanol (60ml) were added and the mixture heated at 50° for 4 days. The solvent was evaporated and the residue purified by HPLC eluting with ethyl acetate/hexane mixtures to give 3-methyl 5-(1-methylethyl) 4-(3,6-difluoro-2-(trifluoromethyl)phenyl)-2-(dimethoxymethyl)-1,4-dihydro-6-methyl-3,5-pyridinedicarboxylate (3.8g) as an oil.

This oil (3.8g) was stirred at room temperature in formic acid (20ml) for 3 hours. Ethyl acetate (100ml) was added and this solution was washed with water, sodium bicarbonate solution and water, dried ($MgSO_4$) and the solvent evaporated. Chromatography on silica eluting with ethyl acetate/hexane mixtures followed by tituration with hexane gave the title aldehyde (1.2g) mp 127-8°.

Example 80

3-Methyl 5-(1-methylethyl) 4-(3-chloro-6-fluoro-2-(trifluoromethyl)phenyl)-2-formyl-1,4-dihydro-6-methyl-3,5-pyridinedicarboxylate

Prepared by the method of Example 79 and identical with that obtained in Example 70.

Example 81

3-Methyl 5-(1-methylethyl) 2-(fluoromethyl)-4-(3-formyl-2-thienyl)-1,4-dihydro-6-methyl-3,5-pyridinedicarboxylate

3-Methyl 5-(1-methylethyl) 4-(3-(1,3-dioxolan-2-yl)-2-thienyl)-2-(fluoromethyl)-1,4-dihydro-6-methyl-3,5-pyridinedicarboxylate (1.4g) in tetrahydrofuran (25ml) and dilute hydrochloric acid (5ml) was stirred at room temperature for 15 minutes. The mixture was poured onto water/ether. The ethereal extract was washed with sodium bicarbonate solution and water, dried ($MgSO_4$) and the solvent was evaporated. Crystallisation from ethyl acetate/petroleum ether (60-80$^{o}$) gave the title aldehyde (1.1g) mp 135-7$^{o}$.

Example 82

3-Ethyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate maleate hydrate

3-Ethyl 5-methyl 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate (2.56g, 4mmoles) and hydrazine hydrate (0.58ml, 12mmoles) in ethanol (50ml) was heated at reflux for 1 hour. The solvent was removed and the residue titurated with ether (5 x 50ml). The combined ethereal extracts were washed with water, dried ($MgSO_4$) and the solvent evaporated. Chromatography on silica eluting with dichloromethane/ethanol mixtures gave the title compound (1.5g) as the free base. Addition of this oil to a solution of maleic acid (0.34g, 3mmoles) in ethanol (10ml) and dilution with ether (40ml) gave the title compound (1.6g) mp 161-3°.

Example 83

3-Ethyl 5-methyl 2-(4-aminobutyl)-4-(3-chloro-6-fluoro-2-(trifluoromethyl)phenyl)-6-(fluoromethyl)-1,4-dihydro-3,5-pyridinedicarboxylate maleate

3-Ethyl 5-methyl 4-(3-chloro-6-fluoro-2-(trifluoromethyl)phenyl)-2-(4-(1,3-dihydro-1,3-dioxo-2H-isoindol-2-yl)butyl)-6-(fluoromethyl)-1,4-dihydro-3,5-pyridinedicarboxylate (2.9g, 4.53mmoles) and hydrazine hydrate (0.67ml, 13.6mmoles) in ethanol (50ml) was heated at reflux for 3 hours. The cooled reaction mixture was filtered and the ethanol evaporated. The residue was extracted with ether (100ml) and the ether solution washed with water, dried ($MgSO_4$) and evaporated to dryness.

The residue was dissolved in ethanol and maleic acid (0.525g, 4.5mmoles) was added. Addition of ether gave the solid salt which was recrystallised from ethanol/ether to give the title compound (1.8g) mp 161-2$^{o}$.

Example 84

5-Methyl 3-(1-methylethyl) 2-(4-aminobutyl)-4-(3-chloro-6-fluoro-2-(trifluoromethyl)phenyl)-6-(fluoromethyl)-1,4-dihydro-3,5-pyridinedicarboxylate maleate

Prepared by the method of Examples 82 and 83 using the appropriate starting material mp 167-8$^{o}$ (ether/ethanol).

Example 85

3-Methyl 5-(1-methylethyl) 4-(3-amino-6-fluoro-2-(trifluoromethyl)phenyl)-2-(fluoromethyl)-1,4-dihydro-6-methyl-3,5-pyridinedicarboxylate

3-Methyl 5-(1-methylethyl) 2-(fluoromethyl)-4-(6-fluoro-3-((2,2,2-trifluoroacetyl)amino)-2-trifluoromethyl)phenyl)-1,4-dihydro-6-methyl-3,5-pyridinedicarboxylate (1.0g, 1.8mmoles) and sodium borohydride (0.5g) in tetrahydrofuran (30ml) was stirred at room temperature for 4 days. The solvent was evaporated and the residue taken up in ethyl acetate/water. The organic layer was separated, dried ($MgSO_4$) and the solvent evaporated. The residue in

tetrahydrofuran (30ml) was treated with sodium borohydride (0.2g) and left stirring overnight. Work up as described immediately above and chromatography on silica eluting with toluene/ethyl acetate mixtures gave the title compound (0.23g) mp 197-8$^{o}$ (2-propanol/petroleum ether (60-80$^{o}$)).

Example 86

5-(2-Cyanoethyl) 3-methyl 4-(3-chloro-6-fluoro-2-(trifluoromethyl)phenyl)-2-(fluoromethyl)-1,4-dihydro-6-methyl-3,5-pyridinedicarboxylate

3-Chloro-6-fluoro-2-(trifluoromethyl)benzaldehyde (68g), 0.3moles), methyl 4-fluoro-3-oxobutanoate (40.2g, 0.3moles) and 2-cyanoethyl 3-amino-2-butenoate (46g, 0.3moles) were heated in dry t.butanol (600ml) under $N_2$ at 55$^{o}$ for 8 days. The reaction mixture was cooled and the product filtered off. Crystallisation from 2-propanol gave the title compound (34g) mp 167-8$^{o}$.

Example 87

4-(3-Chloro-6-fluoro-2-(trifluoromethyl)phenyl)-6-(fluoromethyl)-1,4-dihydro-5-(methoxycarbonyl)-2-methyl-3-pyridinecarboxylic acid

Sodium hydroxide (3.75g, 0.094moles) in water (120ml) was added to a stirred solution of 5-(2-cyanoethyl) 3-methyl 4-(3-chloro-6-fluoro-2-(trifluoromethyl)phenyl)-2-(fluoromethyl)-1,4-dihydro-6-methyl-3,5-pyridinedicarboxylate (17.6g, 0.037moles) in glyme (60ml). After stirring for 16 hours, the solution was poured into dilute hydrochloric acid (350ml) and the solid was filtered off, washed with water and methanol and dried in vacuo to give the title acid (14.0g) mp 209-210°.

Example 88

4-(3-Cyano-6-fluoro-2-(trifluoromethyl)phenyl)-6-(fluoromethyl)-1,4-dihydro-5-(methoxycarbonyl)-2-methyl-3-pyridinedicarboxylic acid

Sodium hydroxide (1.42g, 35mmoles) in water (30ml) was added to a stirred solution of 5-(2-cyanoethyl) 3-methyl 4-(3-cyano-6-fluoro-2-(trifluoromethyl)phenyl)-2-(fluoromethyl)-1,4-dihydro-6-methyl-3,5-pyridinedicarboxylate (8.34g, 17mmoles) in glyme (30ml). After stirring for 3 hours, the reaction mixture was poured onto dilute hydrochloric acid, the solid filtered off, washed with water and dried in vacuo to give the title acid (5.3g). $M^+$ 416.

Example 89

5-(2-Cyanoethyl) 3-methyl 4-(3,6-difluoro-2-trifluoromethyl)phenyl)-2-(fluoromethyl)-1,4-dihydro-6-methyl-3,5-pyridinedicarboxylate

3,6-Difluoro-2-(trifluoromethyl)benzaldehyde (21g, 0.1moles), methyl 4-fluoro-3-oxobutanoate (13.4g, 0.1moles) and 2-cyanoethyl 3-amino-2-butenoate (15.4g,

0.1moles) were heated in dry t.butanol (100ml) under $N_2$ at 60° for 4 days. The solvent was evaporated and the residue, in dichloromethane (250ml), was treated with trifluoroacetic anhydride (5mls). After 15 minutes methanol (20ml) was added and then the solvent was evaporated. Purification by HPLC eluting with ethyl acetate/petroleum ether (60-80°) mixtures gave the title ester (12.4g). mp 143-5° (2-propanol/petroleum ether (60-80°)).

Example 90

4-(3,6-Difluoro-2-(trifluoromethyl)phenyl)-6-(fluoromethyl)-1,4-dihydro-5-(methoxycarbonyl)-2-methyl-3-pyridinecarboxylic acid

Sodium hydroxide (3.2g, 80mmoles) in water (70ml) was added to a solution of 5-(2-cyanoethyl) 3-methyl 4-(3,6-difluoro-2-(trifluoromethyl)phenyl)-2-(fluoromethyl)-1,4-dihydro-6-methyl-3,5-pyridinedicarboxylate (12.4g, 27mmoles) in glyme (70ml). After stirring for 16 hours, the solution was added to water (600ml) and the mixture extracted once with ether. The aqueous layer was acidified with dilute hydrochloric acid and the precipitate extracted with ethyl acetate. The organic layer was separated, dried ($MgSO_4$) and the solvent evaporated to give the title acid (10.5g) mp 187°.

Example 91

3-Methyl 5-(6-(((4-methylphenyl)sulphonyl)oxy)hexyl) 4-(3-chloro-6-fluoro-2-(trifluoromethyl)phenyl)-2-(fluoromethyl)-1,4-dihydro-6-methyl-3,5-pyridinedicarboxylate

Phosphorous pentachloride (4.88g, 23.5mmoles) was added to a stirred suspension of 4-(3-chloro-6-fluoro-2-(trifluoromethyl)phenyl)-6-(fluoromethyl)-1,4-dihydro-5-(methoxycarbonyl)-2-methyl-3-pyridinecarboxylic acid (10g, 23.5mmoles) in dichloromethane (350ml). After 1 hour, 6-hydroxyhexyl 4-methylbenzenesulphonate (5.64g, 23.5mmoles) in dichloromethane (100ml) was added slowly. After 16 hours, 5% aqueous sodium carbonate was added with vigorous stirring until the aqueous layer was pH 7-8. The organic layer was separated, washed with brine, dried ($MgSO_4$) and the solvent was evaporated. Purification by HPLC on silica eluting with ethyl acetate/petroleum ether (60-80$^{o}$) mixtures gave the title compound as an oil (11.8g).

Nmr delta ($CDCl_3$) 2.32 (s,3H), 2.45 (s,3H), 3.56 (s,3H) and 5.93 (q,1H).

Example 92

3-Methyl 5-(6-(((4-methylphenyl)sulphonyl)oxy)hexyl) 4-(3,6-difluoro-2-(trifluoromethyl)phenyl)-2-(fluoromethyl)-1,4-dihydro-6-methyl-3,5-pyridinedicarboxylate

3,6-Difluoro-2-(trifluoromethyl)benzaldehyde (3.32g,

16mmoles), methyl 4-fluoro-3-oxobutanoate (2.3g, 17mmoles) and 6-(((4-methylphenyl)sulphonyl)oxy)hexyl 3-amino-2-butenoate (5.2g, 16mmoles) in t.butanol (80ml) were heated at 55$^{o}$ for 1 week. The solvent was evaporated and the residue, in dichloromethane (80ml), was treated with trifluoroacetic anhydride (0.75ml) with stirring at room temperature. After 10 minutes methanol (10ml) was added and then the solvent was evaporated. Purification by HPLC on silica eluting with ethyl acetate/ petroleum ether (60-80$^{o}$) mixtures gave the title compound as an oil (5.8g).

Nmr delta ($CDCl_3$) 2.32 (s,3H), 2.44 (s,3H), 3.56 (s,3H) and 5.83 (q,1H).

Example 93

3-Ethyl 5-methyl 4-(3-chloro-6-fluoro-2-(trifluoromethyl)phenyl-2-((2-(1,3-dihydro-1,3-dioxo-2H-isoindol-2-yl)ethoxy)methyl)-6-(fluoromethyl)-1,4-dihydro-3,5-pyridinedicarboxylate

Ethyl 4-(2-(1,3-dihydro-1,3-dioxo-2H-isoindol-2-yl)-ethoxy-3-oxobutanoate (14.7g, 46mmoles) and ammonium acetate (3.9g, 51mmoles) in t.butanol (100ml) were heated at 60$^{o}$ for 2 hours. 3-Chloro-6-fluoro-2-(trifluoromethyl)benzaldehyde (10.4g, 46mmoles) and methyl 4-fluoro-3-oxobutanoate (6.2g, 46mmoles) were added and the heating at 60$^{o}$ continued for 4 days. The solvent

was evaporated and the residue extracted with ether (200ml). The ether was evaporated and the residue chromatographed on silica eluting with ethyl acetate/ petroleum ether (60-80$^o$) mixtures to give the title compound (2.6g) mp 159-60$^o$.

$M^+$, 642/4; nmr delta ($CDCl_3$) 3.63 (s,3H), 6.08 (q,1H) and 8.2 (d,1H).

Example 94

3-Ethyl 5-methyl 4-(3-chloro-6-fluoro-2-(trifloromethyl)phenyl)-2-(4-(1,3-dihydro-1,3-dioxo-2H-isoindol-2-yl)butyl)-6-(fluoromethyl)-1,4-dihydro-3,5-pyridinedicarboxylate

Prepared by the method of Example 93 using the appropriate starting materials. mp 170-1$^o$.

$M^+$, 640/2; nmr delta ($CDCl_3$) 3.55 (s,3H), 5.95 (q,1H), 7.15 (d,1H).

Example 95

5-Methyl 3-(1-methylethyl) 4-(3-chloro-6-fluoro-2-(trifluoromethyl)phenyl)-2-(4-(1,3-dihydro-1,3-dioxo-2H-isoindol-2-yl)butyl)-6-(fluoromethyl)-1,4-dihydro-3,5-pyridinedicarboxylate

Prepared by the method of Example 93 using appropriate starting materials. mp 142-3$^o$.

Nmr delta ($CDCl_3$) 3.57 (s,3H), 6.1 (q,1H), 7.05 (d,1H).

Example 96

3-Ethyl 5-methyl 2-((2-aminoethoxy)methyl)-4-(3-chloro-6-fluoro-2-(trifluoromethyl)phenyl)-1,4-dihydro-6-methyl-3,5-pyridinedicarboxylate maleate

3-Ethyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate (0.53g, 1.01mmoles) was hydrogenated at 1 atmosphere in ethanol (20ml) over 10% Pd/C (0.4g). After 3 hours, ethanolic hydrogen chloride (2ml) was added and the hydrogenation continued for a further 3 hours. The catalyst was filtered off and the solvent evaporated. The residue was dissolved in ethyl acetate/saturated sodium bicarbonate and the organic layer was separated, dried ($MgSO_4$) and the solvent evaporated. Chromatography on silica eluting with dichloromethane ethanol/triethylamine mixtures followed by salt formation with maleic acid gave the title compound as the maleate salt mp 182-3$^{o}$.

Example 97

5-Methyl 3-(6-(5-phenyl-3-pyrazolyloxy)hexyl) 4-(3-chloro-6-fluoro-2-(trifluoromethyl)phenyl)-2-(fluoromethyl)-1,4-dihydro-6-methyl-3,5-pyridinedicarboxylate mp 132-4$^{o}$ (cyclohexane/dichloromethane/ethyl acetate).

Example 98

3-Ethyl 5-methyl 2-(4-((5-amino-1H-1,2,4-triazol-3-yl)amino)butyl)-4-(3-chloro-6-fluoro-2-(trifluoromethyl)phenyl)-6-(fluoromethyl)-1,4-dihydro-3,5-pyridinedicarboxylate

Prepared by the method of Example 100 mp 108-10° (petroleum ether (60-80°)/ether).

Example 99

5-Ethyl 3-methyl 4-(3-chloro-6-fluoro-2-(trifluoromethyl)phenyl)-2-(fluoromethyl)-1,4-dihydro-2-(4-(2,4,5-trimethyl-1H-imidazol-1-yl)butyl)-3,5-pyridinedicarboxylate

Prepared by the method of Example 101 mp 169-71° (petroleum ether (60-80°)/acetone).

Example 100

3-Ethyl 5-methyl 2-((2-((5-amino-1H-1,2,4-triazol-3-yl)amino)ethoxy)methyl)-4-(3-chloro-6-fluoro-2-(trifluoromethyl)phenyl)-6-(fluoromethyl)-1,4-dihydro-3,5-pyridinedicarboxylate

Dimethyl N-cyanoiminodithiocarbonate (0.175g, 1.2mmoles) and 3-ethyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate (0.51g, 1mmole) in 2-propanol (5ml) were stirred at room temperature for 4 hours. Ether (10ml) was added and the stirring continued for a further 1.5 hours. Filtration

gave 3-ethyl 5-methyl 4-(3-chloro-6-fluoro-2-(trifluoromethyl)phenyl)-2-((2-(((cyanoimino)methylthio)methyl)amino)ethoxy)methyl)-6-(fluoromethyl)-1,4-dihydro-6-methyl-3,5-pyridinedicarboxylate (0.5g) mp 177-80° (decomp).

The above solid (0.5g) and hydrazine hydrate (0.2ml) in ethanol (5ml) were heated at reflux for 3 hours. Evaporation of the solvent and chromatography of the residue using dichloromethane/methanol gave the title compound (0.27g) mp 96-8°.

Example 101

5-Ethyl 3-methyl 4-(3-chloro-6-fluoro-2-(trifluoromethyl)phenyl)-2-(fluoromethyl)-1,4-dihydro-6-((2-(2,4,5-trimethyl-1H-imidazol-1-yl)ethoxy)methyl)-3,5-pyridinedicarboxylate

3-Ethyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate (0.51g, 1mmole) and hexahydro-2,4,6-trimethyl-1,3,5-triazine trihydrate (0.365g, 2mmoles) in methanol (5ml) were stirred at 0° for 1 hour. 2,3-Butanedione (0.35g, 4mmoles) in methanol (2ml) was added and the reaction mixture kept at 0° for 16 hours. Ammonia (5ml, d 0.88) was added and the mixture kept at 0° for 24 hours. The crystalline product was filtered off and recrystallised from ethanol/water to give

the title compound (0.49g) mp 76-8$^{o}$.

Example 102

3-Ethyl 5-methyl 4-(3-chloro-6-fluoro-2-(trifluoromethyl)phenyl)-2-((2-(dimethylamino)ethoxy)methyl)-6-(fluoromethyl)-1,4-dihydro-3,5-pyridinedicarboxylate maleate

Ethyl 4-((2-(dimethylamino)ethoxy)-3-oxobutanoate (6.47g, 30mmoles), ammonium acetate (2.4g, 31mmoles) and t-butanol (150ml) were heated at 60$^{o}$ for 2 hours. Methyl 4-fluoro-3-oxobutanoate (4.03g, 30mmoles) and 4-chloro-6-fluoro-2-(trifluoromethyl)benzaldehyde (6.8g, 30mmoles) were added and the heating continued for 3 days. The solvent was evaporated and the residue, dissolved in dichloromethane (100ml), was treated with trifluoroacetic anhydride (2.0ml). After 10 minutes the solvent was evaporated and the residue chromatographed on silica eluting with tetrahydrofuran/petroleum ether (60-80$^{o}$)/triethylamine mixtures. The maleate salt was formed and recrystallised from ethanol to give the title compound (1.95g) mp 123-5$^{o}$.

Example 103

5-Cyclobutyl 3-methyl 4-(3-cyano-6-fluoro-2-(trifluoromethyl)phenyl)-2-(fluoromethyl)-1,4-dihydro-1,6-dimethyl-3,5-pyridinedicarboxylate

Powdered potassium hydroxide (0.336g, 6mmoles) was

added to a stirred solution of 5-cyclobutyl 3-methyl 4-(3-cyano-6-fluoro-2-(trifluoromethyl)phenyl)-2-(fluoromethyl)-1,4-dihydro-6-methyl-3,5-pyridine-dicarboxylate (0.7g, 1.5mmoles) and iodomethane (0.43g, 3mmoles) in dimethylsulphoxide (14ml). After 15 minutes, the reaction mixture was poured into water and the product extracted with ethyl acetate. The organic extract was washed with brine, dried ($MgSO_4$) and the solvent evaporated. Chromatography on silica eluting with ethyl acetate/petroleum ether (60-80$^\circ$) gave the title compound (0.35g). mp 110-2$^\circ$.

Example 104

5-Methyl 3-(1-methylethyl) 2-((2-(1,3-dihydro-1,3-dioxo-2H-isoindol-2-yl)ethoxy)methyl)-6-(fluoromethyl)-1,4-dihydro-4-(2,3,6-trifluorophenyl)-3,5-pyridinedicarboxylate

Prepared by the method of Example 93 mp 141-3$^\circ$ (ethanol/ether).

Example 105

5-Methyl 3-(1-methylethyl) 2-((2-aminoethoxy)methyl)-6-(fluoromethyl)-1,4-dihydro-4-(2,3,6-trifluorophenyl)-3,5-pyridinedicarboxylate

Prepared by the method of Examples 82 and 83 Nmr delta ($CDCl_3$) 5.45 (s,1H) and 3.60 (s,3H).

Example 106

3-Ethyl 5-methyl 4-(3-chloro-6-fluoro-2-

(trifluoromethyl)phenyl)-2-((2-(4-((4-chlorophenyl)methyl)-1-piperazinyl)ethoxy)methyl)-6-(fluoromethyl)-1,4-dihydro-3,5-pyridinedicarboxylate bisoxalate

Prepared by the method of Example 7 and converted to the oxalate salt (methanol).

Nmr delta ($CDCl_3$) 5.89 (q,1H) and 5.55 (m,2H).

Example 107

5-Ethyl 3-methyl 4-(3-chloro-6-fluoro-2-(trifluoromethyl)phenyl)-1,4-dihydro-2-methyl-6-((2-(1-piperazinyl)ethoxy)methyl)-3,5-pyridinedicarboxylate

3-Ethyl 5-methyl 4-(3-chloro-6-fluoro-2-(trifluoromethyl)phenyl)-2-((2-(4-((4-chlorophenyl)methyl)-1-piperazinyl)ethoxy)methyl)-6-(fluoromethyl)-1,4-dihydro-3,5-pyridinedicarboxylate (3.5g, 4mmoles) was dissolved in methanol (500ml) and acetic acid (1ml). 5% Pd/C was added and the mixture hydrogenated at 45 p.s.i. for 16 hours. The catalyst was filtered off and the solvent was evaporated. The residue was dissolved in dichloromethane/sodium bicarbonate solution, the organic layer separated, dried ($MgSO_4$) and the solvent evaporated to give the title compound (1.4g).

Nmr delta ($CDCl_3$) 5.99 (q,1H), 4.70 (q,2H).

Example 108

5-Ethyl 3-methyl 4-(3-chloro-6-fluoro-2-(trifluoromethyl)phenyl)-1,4-dihydro-2-methyl-6-((2-(4-((methylamino)carbonyl)-1-piperazinyl)ethoxy)methyl)-3,5-pyridinedicarboxylate

5-Ethyl 3-methyl 4-(3-chloro-6-fluoro-2-(trifluoromethyl)phenyl)-1,4-dihydro-2-methyl-6-((2-(1-piperazinyl)ethoxy)methyl)-3,5-pyridinedicarboxylate (0.05g, 0.9mmoles) and methyl isocyanate (0.008ml, 0.9mmoles) in chloroform (1ml) were stirred for 1 hour. The solvent was evaporated and the residue chromatographed on silica eluting with dichloromethane/methanol to give the title compound (0.025g).

Nmr delta ($CDCl_3$) 5.97 (q,1H), 2.85 (d,3H), 2.4 (s,3H).

Example 109

5-Ethyl 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 2 and 4 mp 159-61$^{o}$ (acetone/petroleum ether (60-80$^{o}$).

Example 110

5-Cyclobutyl 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 2 and 4 mp 135-7$^{o}$ (petroleum ether (60-80$^{o}$)).

Example 111

3-Methyl 5-(2-methylpropyl) 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 2 and 4 mp 158-9° (2-propanol).

Example 112

5-Methyl 3-(1-methylethyl) 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 2 and 4 mp 135-7° (petroleum ether (60-80°)).

Example 113

3-Ethyl 5-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 2 and 4 mp 148-50° (2-propanol).

Example 114

3-Methyl 5-(3-oxetanyl) 4-(3-chloro-6-fluoro-2-(trifluoromethyl)phenyl)-2-(fluoromethyl)-1,4-dihydro-6-methyl-3,5-pyridinedicarboxylate

Prepared by the method of Example 6 mp 158.5-160° (acetone/petroleum ether (60-80°)).

Example 115

3-Methyl 5-(tetrahydro-4H-pyran-4-yl) 4-(3-chloro-6-

fluoro-2-(trifluoromethyl)phenyl)-2-(fluoromethyl)-1,4-dihydro-6-methyl-3,5-pyridinedicarboxylate

Prepared by the method of Examples 2 and 4 mp 88-90° (acetone/petroleum ether (60-80°)).

Example 116

3-Methyl 5-(2,2,2-trifluoroethyl) 4-(3-chloro-6-fluoro-2-(trifluoromethyl)phenyl)-2-(fluoromethyl)-1,4-dihydro-6-methyl-3,5-pyridinedicarboxylate

Prepared by the method of Example 6 mp 164-6° (acetone/petroleum ether (60-80°)).

Example 117

5-(2,3-Dihydro-2-indenyl) 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Example 6 mp 169-71° (acetone/petroleum ether (60-80°)).

Example 118

3-Methyl 5-(2-methyl-1-(methylethyl)propyl) 4-(3-chloro-6-fluoro-2-(trifluoromethyl)phenyl)-2-(fluoromethyl)-1,4-dihydro-6-methyl-3,5-pyridinedicarboxylate

Dicyclohexylcarbodiimide (0.48g, 2.3mmoles) was added to a stirred solution of 4-(3-chloro-6-fluoro-2-(trifluoromethyl)phenyl)-6-(fluoromethyl)-1,4-dihydro-5-(methoxycarbonyl)-2-methyl-3-pyridinecarboxylic acid

(1.0g, 2.3mmoles) and 2,4-dimethyl-3-pentanol (2.73g, 23mmoles) in dichloromethane (10ml). After 20 hours, the reaction mixture was filtered, the solvent evaporated and ether (20ml) added. After 1 hour the solution was filtered and the ether evaporated. Chromatography of the residue on silica eluting with ethyl acetate/hexane gave the title compound (0.135g) mp 179-81° (2-propanol/cyclohexane).

Example 119

5-(2,2-Dimethylpropyl) 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Example 7 mp 169-71° (petroleum ether (60-80°)).

Example 120

5-(1,1-Dimethylethyl) 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 53-55 mp 121-2° (hexane).

Example 121

5-(2-Fluoro-1-(fluoromethyl)ethyl) 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Example 118 mp 124-5° (cyclohexane).

Example 122

3-Methyl 5-(2,2,2-trifluoro-1-(trifluoromethyl)ethyl) 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 53-55 mp 102-4° (petroleum ether (60-80°)). $M^+$ 577/5, nmr delta ($CDCl_3$) 6.00 (q,1H), 3.59 (s,3H).

Example 123

5-(Ethoxymethyl) 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Example 124 mp 154-5° (2-propanol).

Example 124

3-Methyl 5-(2-oxopropyl) 4-(3-chloro-6-fluoro-2-(trifluoromethyl)phenyl)-2-(fluoromethyl)-1,4-dihydro-6-methyl-3,5-pyridinedicarboxylate

Sodium hydride (0.057g of 50%, 1.2mmoles) was added to a stirred solution of 4-(3-chloro-6-fluoro-2-(trifluoromethyl)phenyl)-6-(fluoromethyl)-1,4-dihydro-5-(methoxycarbonyl)-2-methyl-3-pyridinecarboxylate (0.5g, 1.2mmoles) in tetrahydrofuran (20ml). After 1 hour,

1-chloro-2-propanone (0.088g, 1mmole) was added. After 2 weeks at room temperature, the reaction mixture was poured onto dilute hydrochloric acid and ethyl acetate. The aqueous layer was re-extracted with ethyl acetate and the combined organic extracts were washed with water and brine and dried ($MgSO_4$). Evaporation of solvent and chromatography on silica eluting with ethyl acetate/hexane gave the title compound (0.3g) mp 142-3$^o$ (2-propanol/hexane).

Example 125

5-(1-Ethylpropyl) 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Example 6 mp 134-7$^o$ (petroleum ether (60-80$^o$)).

Example 126

5-(3-Cyclopentenyl) 3-methyl 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 53-55 mp 169-71$^o$ (acetone/petroleum ether (60-80$^o$)).

Example 127

5-(3-Hydroxy-2-(hydroxymethyl)-2-methylpropyl) 3-methyl 4-(3-chloro-6-fluoro-2-(trifluoromethyl)phenyl)-2-(fluoromethyl)-1,4-dihydro-6-methyl-3,5-pyridinedicarboxylate

3-Methyl 5-((3-methyl-3-oxetanyl)methyl) 4-(3-chloro-6-fluoro-2-(trifluoromethyl)phenyl)-2-(fluoromethyl)-1,4-dihydro-6-methyl-3,5-pyridinedicarboxylate (0.6g) was dissolved in ethyl acetate (10ml) and hydrochloric acid (10ml of 2N) was added. After standing for 16 hours, the organic layer was separated, dried ($Na_2SO_4$) and the solvent evaporated. Chromatography on silica eluting with ethyl acetate/petroleum ether (60-80°) mixtures followed by crystallisation from methanol/2-propanol/petroleum ether (60-80°) gave the title compound (0.28g) mp 122-4°.

Example 128

3-Methyl 5-(cis-octahydro-endo-2-pentalenyl) 4-(3-chloro-6-fluoro-2-(trifluoromethyl)phenyl)-2-(fluoromethyl)-1,4-dihydro-6-methyl-3,5-pyridinedicarboxylate

Prepared by the method of Examples 53-55 mp 205° (decomp).

Example 129

5-(2,2-Dimethyl-3-((methyl(phenylmethyl)amino)propyl) 3-methyl 4-(3-chloro-6-fluoro-2-(trifluoromethyl)phenyl)-2-(fluoromethyl)-1,4-dihydro-6-methyl-3,5-pyridinedicarboxylate hydrochloride

Prepared by the method of Example 7 mp 115°.

(decomp). $M^+$, 616/4, nmr delta ($D_6$DMSO) 5.76 (q,1H).

Example 130

3-Methyl 5-(1-methylethyl) 2-(difluoromethyl)-4-(3,6-difluoro-2-(trifluoromethyl)phenyl)-1,4-dihydro-6-methyl-3,5-pyridinedicarboxylate

Diethylaminosulphurtrifluoride (0.36g, 2.2mmoles) was added to a stirred solution at -20° of 3-methyl 5-(1-methylethyl) 4-(3,6-difluoro-2-(trifluoromethyl) phenyl)-2-formyl-1,4-dihydro-6-methyl-3,5-pyridine-dicarboxylate (0.9g, 2mmoles) in dichloromethane (50ml). After 1 hour at 20° and 1 hour at reflux, the solvent was evaporated and the residue chromatographed on silica eluting with ethyl acetate/hexane. Tituration with hexane of pure evaporated fractions gave the title compound (0.34g) mp 136-7°.

Example 131

3-Methyl 5-(1-methylethyl) 4-(3-chloro-6-fluoro-2-(trifluoromethyl)phenyl)-2-(difluoromethyl)-1,4-dihydro-6-methyl-3,5-pyridinedicarboxylate

Prepared by the method of Example 130 mp 131-2° (hexane).

Example 132

5-(3-Azetidinyl) 3-methyl 4-(3-chloro-6-fluoro-2-(trifluoromethyl)phenyl)-2-(fluoromethyl)-1,4-dihydro-6-methyl-3,5-pyridinedicarboxylate hydrochloride

Prepared by the method of Examples 53-55 mp 176° (decomp) (ethanol/ether). $M^+$, 480/2; nmr delta ($D_6DMSO$) 9.3 (s.1H), 5.85 (q,1H), 3.6 (s,3H).

Example 133

5-Methyl 3-(2,2,2-trichloroethyl) 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Example 6 Nmr delta ($CDCl_3$) 6.05 (q,1H), 3.56 (s,3H).

Example 134

4-(3-Chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-5-(methoxycarbonyl)-3-pyridinecarboxylic acid

Zinc dust (0.31g, 4.75mmoles) was added to a stirred solution of 5-methyl 3-(2,2,2-trichloroethyl) 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate (0.7g, 0.95mmoles) in acetonitrile (10ml) containing formic acid (0.18ml, 4.75mmoles). After 3 days, the reaction mixture was poured onto brine, acidified with 10% aqueous sulphuric acid and extracted with chloroform. The organic layer was filtered, washed with 10% aqueous sulphuric acid and

brine, dried ($MgSO_4$) and evaporated to dryness to give the title compound. $M^+ + 1$, 615/7.

Example 135

5-Methyl 3-(1-methylethyl) 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Examples 53 - 55. $M^+$, 656/8.

Example 136

5-Methyl 3-(1-methylethyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate

Prepared by the method of Examples 82 and 83. $(M+1)^+$ 527/9 Nmr delta ($CDCl_3$) 825 (d,1H), 60 (q,1H), 3.57 (s,3H).

Example 137

5-Methyl 3-(1-methylethyl) 4-(2,6-difluoro-3-nitrophenyl)-2-((2-(1,3-dihydro-1,3-dioxo-2H-isoindol-2-yl) ethoxy)methyl-6-(fluoromethyl)-1,4-dihydro-3,5-pyridinedicarboxylate

Prepared by the method of Example 93 $(M+1)^+$, 618; nmr delta ($CDCl_3$) 5.53 (s,1H), 3.60 (s,3H).

Example 138

5-Methyl 3-(1-methylethyl) 2-((2-aminoethoxy)methyl)

-4-(2,6-difluoro-3-nitrophenyl)-6-(fluoromethyl)-1,4-dihydro-3,5-pyridinedicarboxylate

Prepared by the method of Examples 82 and 83. (M+1) 488.

Example 139

5-Ethyl 3-methyl 4-(3-chloro-6-fluoro-2-(trifluoromethyl)phenyl)-1,4-dihydro-2-methyl-6-((2-(4-(2-thiazolyl)-1-piperazinyl)ethoxy)methyl)-3,5-pyridinedicarboxylate

5-Ethyl 3-methyl 4-(3-chloro-6-fluoro-2-(trifluoromethyl)phenyl)-1,4-dihydro-2-methyl-6-((2-(1-piperazinyl)ethoxy)methyl)-3,5-pyridinedicarboxylate (0.05g, 0.1mmoles) and 2-bromothiazole (0.02ml) were heated at 120$^{\circ}$ for 2 days. Chromatography on silica eluting with dichloromethane/methanol/triethylamine gave the title compound (0.02g).

$(M+1)^+$ 647/9; nmr delta ($CDCl_3$) 5.98 (q,1H), 3.58 (s,3H).

Example 140

5-Ethyl 3-methyl 4-(3-chloro-6-fluoro-2-(trifluoromethyl)phenyl)-2-(fluoromethyl)-1,4-dihydro-6-(4-methoxy-4-oxobutyl)-3,5-pyridinedicarboxylate

Prepared by the method of Example 6. $M^+$, 539/41.

Example 141

4-(4-(3-Chloro-6-fluoro-2-(trifluoromethyl)phenyl)-3-

(ethoxycarbonyl)-6-(fluoromethyl)-1,4-dihydro-5-(methoxycarbonyl)-2-pyridyl)butanoic acid

Prepared by the method of Examples 87, 88 and 90 from 5-ethyl 3-methyl 4-(3-chloro-6-fluoro-2-(trifluoromethyl)phenyl)-2-(fluoromethyl)-1,4-dihydro-6-(4-methoxy-4-oxobutyl)-3,5-pyridinedicarboxylate. Nmr delta ($CDCl_3$) 6.0 (q,1H), 3.57 (s,3H), 2.5 (t,2H).

Example 142

3-Ethyl 5-methyl 4-(3-chloro-6-fluoro-2-(trifluoromethyl)phenyl)-2-(6-ethoxy-4,6-dioxohexyl)-6-(fluoromethyl)-1,4-dihydro-3,5-pyridinedicarboxylate

Thionyl chloride (0.75ml) and dimethylformamide (1 drop) were added to 4-(4-(3-chloro-6-fluoro-2-(trifluoromethyl)phenyl)-3-(ethoxycarbonyl)-6-(fluoromethyl)-1,4-dihydro-5-(methoxycarbonyl)-2-pyridyl)butanoic acid (1.1g, 2mmoles) in dichloromethane (20ml). After stirring for 16 hours, the solvent was evaporated to give the intermediate acid chloride. This compound in dichloromethane (10ml) was added over 30 minutes to a solution at $-20^{o}$ of 2,2-dimethyl-1,3-dioxane-4,6-dione (0.36g, 2.5mmoles) and pyridine (0.23g, 3mmoles) in dichloromethane (50ml). The reaction mixture was allowed to reach room temperature and was stirred for 3 hours. The reaction mixture was then washed with hydrochloric acid and brine, dried ($MgSO_4$) and the solvent evaporated.

The residue was heated at reflux in ethanol for 2 hours and the solvent evaporated. The residue was purified by HPLC eluting with ethyl acetate/petroleum ether (60-80°) mixtures to give the title compound (0.46g).

Nmr delta ($CDCl_3$) 6.0 (q,1H), 3.58 (s,3H), 3.47 (s,2H) and 2.67 (t,2H).

Example 143

3-Ethyl 5-methyl 2-(3-(2-amino-6-hydroxy-4-pyrimidyl) propyl)-4-(3-chloro-6-fluoro-2-(trifluoromethyl)phenyl)-6-(fluoromethyl)-1,4-dihydro-3,5-pyridinedicarboxylate

3-Ethyl 5-methyl 4-(3-chloro-6-fluoro-2-(trifluoromethyl)phenyl)-2-(6-ethoxy-4,6-dioxohexyl)-6-(fluoromethyl)-1,4-dihydro-3,5-pyridinedicarboxylate (0.46g, 0.77mmoles), guanidine hydrochloride (0.075g, 0.79mmoles) and 1,5-diazabicyclo[4.3.0]non-5-ene (0.14g, 1.1mmoles) were heated at reflux in ethanol (15ml) for 3 hours. The solvent was evaporated and the residue chromatographed on silica eluting with dichloromethane/methanol to give the title compound (0.165g) $(M+1)^+$ 591/3.

Example 144

5-Ethyl 3-methyl 4-(3-chloro-6-fluoro-2-(trifluoromethyl)phenyl)-2-(fluoromethyl)-1,4-dihydro-6-((2-methoxy-2-oxoethoxy)methyl)-3,5-pyridinedicarboxylate

Prepared by the method of Example 6. Nmr delta

($CDCl_3$) 6.0 (q,1H), 4.81 (q,2H), 3.58 (s,3H).

Intermediates

Example A

4-Fluoro-3-formyl-2-(trifluoromethyl)benzonitrile

3-Chloro-6-fluoro-2-(trifluoromethyl)benzaldehyde (10g, 44mmoles), copper (I) cyanide (7.68g, 88mmoles) and copper (II) bromide (9.86g, 44mmoles) in 1-methyl-2-pyrolidinone (50ml) were heated at 180° for 7 hours and then 200° for 4 hours. The cooled reaction mixture was poured onto a solution of iron (III) chloride (57g) and conc. hydrochloric acid (9ml) in water (50ml). This solution was heated at 60-70° for 1.5 hours. The cooled solution was extracted with ethyl acetate and the organic extract washed with dilute hydrochloric acid and water, dried ($MgSO_4$) and the solvent evaporated. The residue was chromatographed on silica eluting with ethyl acetate/petroleum ether (60-80°) mixtures to give the title nitrile (1.82g).

$M^+$ 217; nmr ($CDCl_3$) delta 10.32 (q,1H).

Example B

3,6-Difluoro-2-(phenylthio)benzaldehyde and 2,5-difluoro-3-(phenylthio)benzaldehyde

Butyl lithium (12.5ml of 1.6M in hexane, 20mmoles) was added under nitrogen with stirring to a solution of 1,4-difluoro-2-(phenylthio)benzene (4.4g, 20mmoles) in

tetrahydrofuran (30ml) below -60°. After stirring at -70° for 2.5 hours, methyl formate (6.5ml) was added quickly. After a further 30 minutes at -70°, the reaction mixture was poured onto dilute hydrochloric acid/ether. The ethereal extract was washed with brine, dried ($Na_2SO_4$) and the solvent evaporated. Chromatography on silica eluting with ethyl acetate/petroleum ether (60-80°) gave the two pure aldehydes. First eluted was 2,5-difluoro-3-(phenylthio) benzaldehyde (1.95g) mp 72-3° (cyclohexane/hexane).

Second eluted was 3,6-difluoro-2-(phenylthio) benzaldehyde (0.5g) as an oil. $M^+$, 250; nmr delta ($CDCl_3$) 10.55 (s,1H).

Example C

3,6-Difluoro-2-(trifluoromethyl)benzaldehyde and 2,5-difluoro-3-(trifluoromethyl)benzaldehyde

Butyl lithium (28ml of 1.6M in hexane, 39.5mmoles) was added with stirring under nitrogen over 30 minutes to a solution of 1,4-difluoro-2-(trifluoromethyl)benzene (7g, 38.5mmoles) in tetrahydrofuran (90ml) at -78°. After 30 minutes, N-methyl-N-phenylformamide (5.2g, 38mmoles) in tetrahydrofuran (25ml) was added over 30 minutes and after a further 30 minutes, the mixture was poured onto ice (100ml), c. $H_2SO_4$ (10ml) and ether. The aqueous layer was extracted with ether and the combined extracts were

washed with water (x3), sodium bicarbonate solution, brine and dried $MgSO_4$). The solvent was removed and the residue purified by HPLC using ethyl acetate/toluene/ petroleum ether (60-80°) mixtures. 2,5-Difluoro-3-(trifluoromethyl)benzaldehyde (1.0g) was eluted first.

$M^+$ 210; nmr ($CDCl_3$) delta 10.35 (d,1H).

Further elution afforded 3,6-difluoro-2-(trifluoromethyl)benzaldehyde (2.55g).

$M^+$ 210; nmr ($CDCl_3$) delta 10.3 (q,1H).

The compounds of Examples D to J were prepared using appropriate starting materials and the method of Examples B and C.

Example D

6-Fluoro-2,3-bis(trifluoromethyl)benzaldehyde

M+, 260; nmr delta ($CDCl_3$) 10.35 (q,1H).

Example E

6-Fluoro-3-methoxy-2-(trifluoromethyl)benzaldehyde

$M^+$, 222; nmr delta ($CDCl_3$) 10.3 (q,1H).

Example F

3-Chloro-6-methoxy-2-(trifluoromethyl)benzaldehyde

$M^+$, 238/240; nmr delta ($CDCl_3$) 10.3 (q,1H).

Example G

3,6-Difluoro-2-(phenylsulphonyl)benzaldehyde

mp 90-1° (ether/cyclohexane).

Example H

3,5-Dichloro-6-fluoro-2-(trifluoromethyl)benzaldehyde

$M^+$, 260/262/264; nmr delta ($CDCl_3$) 10.25 (q,1H).

Example I

3,6-Dichloro-2-(trifluoromethyl)benzaldehyde and 2,5-dichloro-3-(trifluoromethyl)benzaldehyde

HPLC using ethyl acetate/petroleum ether (60-80°) mixtures gave 3 aldehydes.

First eluted: 2,5-dichloro-3-(trifluoromethyl)benzaldehyde $M^+$ 242/4/6, nmr delta ($CDCl_3$) 10.51 (s,1H).

Second eluted: 2,5-dichloro-4-(trifluoromethyl) benzaldehyde $M^+$ 242/4/6, nmr delta ($CDCl_3$) 10.42 (s,1H).

Third eluted: 3,6-dichloro-2-(trifluoromethyl)benzaldehyde $M^+$ 242/4/6, nmr delta ($CDCl_3$) 10.32 (q,1H).

Example J

2,3,6-Trifluorobenzaldehyde

Nmr delta ($CDCl_3$) 10.3 (1H,d).

Example K

1,4-Difluoro-2-(phenylthio)benzene

Butyl lithium (62.5ml of 1.6M in hexane, 0.1moles) was added with stirring under nitrogen to a solution of 1,4-difluorobenzene (11.4g, 0.1moles) in dry tetrahydrofuran (70ml) below -65°. After 1 hour at -70°, diphenyldisulphide (21.8g, 0.1moles) in tetrahydrofuran (30ml) was added below -60°. After

warming to 0°, the mixture was poured onto ether/water. The separated aqueous layer was extracted with ether, and the combined ethereal extracts were washed with 10% sodium hydroxide and brine, dried ($Na_2SO_4$) and the solvent removed. Distillation (oven temperature 200° at 20mmHg) gave the title compound as an oil (16.9g). $M^+$222.

Example L

4-Fluoro-1-iodo-2-(trifluoromethyl)benzene

A mixture of 4-fluoro-2-(trifluoromethyl)benzenamine (20g, 0.11moles), water (45ml) and conc. hydrochloric acid (45ml) were stirred vigorously at room temperature to give a white suspension. After cooling to 0-5°, sodium nitrite (7.8g, 0.11moles) in water (20ml) was added dropwise keeping the temperature below 5°. After stirring for a further 1 hour at 0°, the solution was poured into potassium iodide (60g) in water (200ml). After standing for 16 hours, the solution was extracted with ether. The organic extract was washed with sodium hydroxide solution (10%) and sodium bisulphite solution (5%), dried ($MgSO_4$) and the solvent evaporated. Bulb to bulb distillation (110° at 30mmHg) gave the title compound (25.5g).

$M^+$, 290; nmr delta ($CDCl_3$) 8.0 (q,1H), 7.4 (m,1H) and 7.0 (m,1H).

Example M

4-Fluoro-1,2-bis(trifluoromethyl)benzene

4-Fluoro-1-iodo-2-(trifluoromethyl)benzene (19g, 65mmoles), trifluoroiodomethane (85g), copper (precipitated, 23g) and dry dimethylformamide (70ml) were heated in a sealed tube at 130° for 20 hours. The reaction mixture was cooled, filtered and then distilled (bulb to bulb 100-145° at 1 atmosphere) to give a colourless oil. This oil was steam distilled and extracted from the distillated with ether. The ether extract was dried ($MgSO_4$) and the ether carefully distilled off to give the title compound (6.9g) contaminated with ether.

$M^+$, 232; nmr delta ($CDCl_3$) 7.82 (q,1H), 7.54 (dd,1H) and 7.34 (b,1H).

Example N

4-Fluoro-1-methoxy-2-(trifluoromethyl)benzene

Sodium nitrite (5.5g, 80mmoles) in water (10ml) was added dropwise below 0° to a stirred suspension of 4-methoxy-3-(trifluoromethyl)benzenamine hydrochloride (17.8g, 78mmoles) in water (31ml) and conc. hydrochloric acid (31ml). After stirring for 1 hour at 0°, tetrafluoroboric acid (24g of 40%) was added. After a further 1.5 hours at 10°, the solid was filtered off, washed with water (20ml) and ether (10ml) and dried *in vacuo* to give 4-methoxy-3-(trifluoromethyl)benzenediazonium tetrafluoroborate (16.5g).

The diazonium salt (16.5g) was heated at 150-160° until decomposition was complete. The residue was dissolved in ether and this solution washed with 10% sodium hydroxide solution and water, dried ($MgSO_4$) and the solvent evaporated. Bulb to bulb distillation (70-80° at 15mmHg) gave the title compound (5.4g).

$M^+$, 194; nmr delta ($CDCl_3$) 3.88 (s,3H).

Example O

1,4-Difluoro-2-(phenylsulphonyl)benzene

A solution of potassium peroxymonosulphate (Oxone) (11.1g, 0.1moles) in water (150ml) was added below 5° to a stirred solution of 1,4-difluoro-2-(phenylthio)benzene (8g, 0.036moles) in methanol (150ml). After stirring overnight at room temperature, the methanol was removed under reduced pressure and the resulting suspension dissolved in water and dichloromethane. The organic layer was separated, dried ($Na_2SO_4$) and the solvent removed to give the title sulphone (8.7g). Recrystallisation from cyclohexane gave pure title compound mp 108-109°.

Example P

2,4-Dichloro-5-(trifluoromethyl)benzenamine hydrochloride

Hydrochloric acid (3.5ml, conc.) was slowly added to

a stirred mixture of 2,4-dichloro-1-nitro-5-(trifluoromethyl)benzene (23.5g, 90mmoles), iron powder (29g), ethanol (40ml) and water (40ml) at $80^{o}$. After addition was complete, the reaction mixture was cooled, diluted with ethanol (100ml) and filtered. The solid was washed with ethanol and water; the combined filtrate was acidified with c.hydrochloric acid and then the solvent was evaporated. The residue was dissolved in ether/sodium bicarbonate solution, the ethereal layer was separated, dried ($Na_2SO_4$) and the solvent removed. Bulb to bulb distillation ( $100^{o}$ at 0.5mmHg) gave the benezenamine free base which was converted to the hydrochloride salt (20g) using methanol/c.hydrochloric acid.

$M^+$, 229/231/233; nmr delta ($CDCL_3$) 7.3 (s,1H) and 7.6 (s,1H).

Example Q

2,4-Dichloro-1-fluoro-5-(trifluoromethyl)benzene

Prepared by the method of Example N $M^+$, 232/4/6; nmr delta ($CDCl_3$) 7.52(d,1H) and 7.61 (d,1H).

Example R

2,4-Difluoro-3-formylbenzonitrile

Lithium 2,2,6,6-tetramethylpiperidine (35mmoles prepared from butyl lithium (22ml of 1.6M in hexane) and 2,2,6,6-tetramethylpiperidine (5g, 35mmoles) in

tetrahydrofuran (30ml), -70° to 0°) was added with stirring below -60° to a solution of 2,4-difluorobenzonitrile (5g, 36mmoles) in tetrahydrofuran (40ml). After stirring at -70° for 10 minutes, methyl formate (10ml) was added. After 5 minutes, the reaction mixture was poured onto ether/dilute hydrochloric acid. The ethereal layer was separated, washed with water, dried ($Na_2SO_4$) and the solvent evaporated. Chromatography on silica eluting with ethyl acetate/petroleum ether (60-80°) mixtures gave the desired aldehyde (3.8g).

$M^+$, 167; nmr delta ($CDCl_3$) 10.35 (s,1H).

Example S

2-(Dimethoxymethyl)-1,4-difluoro-3-(trifluoromethyl)benzene

3,6-Difluoro-2-(trifluoromethyl)benzaldehyde (21.0g, 100mmoles), trimethyl orthoformate (12.5ml), 4-(methylphenyl)sulphonic acid (cat. amount) and methanol (100ml) were heated at reflux for 3 hours. To the cooled solution was added potassium hydroxide in methanol to pH9 and then the solvent was evaporated. The residue was taken up in ether/sodium bicarbonate solution. The ethereal layer was separated, dried ($Na_2SO_4$) and the solvent evaporated. Distillation (bulb to bulb 150° oven temperature at 12mmHg) gave the desired acetal (25.3g).

$M^+$, 256; nmr delta ($CDCl_3$) 3.51 (s,6H) and 5.54 (brs,1H).

Example T

2-(Dimethoxymethyl)-1-fluoro-4-(methylthio)-3-(trifluoromethyl)benzene

Sodium hydride (1.5g of 50%, 30mmoles) was added to a solution of methanethiol (2ml, x.s.) in dimethylformamide (18ml) at $0^o$ with stirring. After 1 hour, 2-(dimethoxymethyl)-1,4-difluoro-3-(trifluoromethyl)benzene (7.6g, 30mmoles) was added and then the mixture was heated at $50^o$ for 6 hours. The cooled reaction mixture was poured onto water/ether. The ether layer was separated, washed with dilute hydrochloric acid (x2), water and sodium bicarbonate solution, dried ($Na_2SO_4$) and the solvent evaporated. Chromatography on silica eluting with petroleum ether (60-$80^o$)/ethyl acetate mixtures gave the desired acetal (5g) as an oil.

$M^+$, 284; nmr delta ($CDCl_3$) 3.50 (s,6H) and 5.53 (s,1H).

Example U

6-Fluoro-3-(methylthio)-2-(trifluoromethyl)benzaldehyde

To 2-(dimethoxymethyl)-1-fluoro-4-(methylthio)-3-(trifluoromethyl)benzene (2.0g) in acetone (150ml) was added hydrochloric acid (75ml of 6N). After 30 minutes at

room temperature, ether and water were added. The ether layer was separated, washed with water and sodium bicarbonate solution, dried ($Na_2SO_4$) and the solvent removed to give the desired aldehyde (1.7g) as an oil.

$M^+$, 238; nmr delta ($CDCl_3$) 10.37 (q,1H).

Example V

6-Fluoro-3-(propylthio)-2-(trifluoromethyl) benzaldehyde

Prepared by the method of Examples T and U.

$M^+$, 266; nmr delta ($CDCl_3$) 10.36 (q,1H).

Example W

6-Fluoro-3-nitro-2-(trifluoromethyl)benzaldehyde

2-Fluoro-6-(trifluoromethyl)benzaldehyde (4g, 21mmoles) was added over 15 minutes to a mixture of nitric acid (23.3ml, d1.5) and sulphuric acid (19ml, d1.84) at 5$^\circ$. After 2 hours at room temperature, the reaction mixture was poured onto ice/water and extracted with ether. Purification by HPLC using ethyl acetate/petroleum ether (60-80$^\circ$) followed by crystallisation from hexane gave the title aldehyde (2.65g) mp 48.5-9$^\circ$.

The compounds of Examples X to Z were prepared using appropriate starting materials and the method of Example W.

Example X

2,6-Difluoro-3-nitrobenzaldehyde mp 57-59$^\circ$.

Example Y

3-Chloro-6-fluoro-2-nitrobenzaldehyde mp 79-81°.

Example Z

2-Chloro-6-fluoro-3-nitrobenzaldehyde

mp 80-85° (cyclohexane).

Example AA

2-(Dimethoxymethyl)-1-fluoro-4-nitro-3-(trifluoromethyl)benzene

Prepared by the method of Example S $M^+$, 283; nmr delta ($CDCl_3$) 6.50 (s,1H) and 3.53 (s,6H).

Example BB

3-(Dimethoxymethyl)-4-fluoro-2-(trifluoromethyl) benzenamine

2-(Dimethoxymethyl)-1-fluoro-4-nitro-3-(trifluoromethyl)benzene (9.6g) was hydrogenated at 1 atmosphere over $PtO_2$ (0.1g) in ethanol (200ml) for 1.5 hours. The catalyst was filtered off and the solvent evaporated. Chromatography on silica eluting with ethyl acetate/petroleum ether (60-80°) mixtures gave the desired acetal (7.5g).

$M^+$, 253; nmr delta ($CDCl_3$) 5.5 (q,1H) and 3.5 (s,6H).

Example CC

N-(4-Fluoro-3-formyl-2-(trifluoromethyl)phenyl) benzenesulphonamide

Benzenesulphonyl chloride (0.83g, 4.7mmoles) was

added with stirring to a solution of 3-(dimethoxymethyl)-4-fluoro-2-(trifluoromethyl)benzenamine (1.2g, 4.7mmoles), pyridine (0.38ml, 4.7mmoles) and dichloromethane (30ml). After stirring for 16 hours, pyridine (0.1ml) and benzenesulphonyl chloride (0.3g) were added and the stirring continued for 24 hours. The reaction mixture was poured onto water and the dichloromethane layer separated, dried ($MgSO_4$) and the solvent evaporated to give N-(3-(dimethoxymethyl)-4-fluoro-2-(trifluoromethyl)phenyl) benzenesulphonamide (2g).

This sulphonamide (2g) was dissolved in acetone (20ml) and hydrochloric acid (10ml of 6M) was added. After stirring for 1 hour, the acetone was evaporated and the residue extracted with ethyl acetate. The organic extract was washed with sodium bicarbonate solution, dried ($MgSO_4$) and the solvent evaporated. Crystallisation from petroleum ether (60-80$^o$) gave the title sulphonamide (1.4g).

$M^+$, 347; nmr delta ($CDCl_3$) 10.4 (q,1H).

Example DD

N-(4-Fluoro-3-formyl-2-(trifluoromethyl)phenyl)-2,2,2-trifluoroacetamide

Trifluoroacetic anhydride (3.6ml) in dichloromethane (5ml) was added dropwise to a stirred solution at 0$^o$ of 3-(dimethoxymethyl)-4-fluoro-2-(trifluoromethyl)

benzenamine (4g, 16mmoles) in dichloromethane (20ml). After stirring for 16 hours, the solvent was evaporated and the residue recrystallised from ether/petroleum ether (60-80°) to give the title aldehyde (3.5g).

$M^+$, 303; nmr delta ($CDCl_3$) 10.4 (q,1H).

Example EE

3-Thietanyl 3-oxobutanoate

3-Thietanol (4.5g, 50mmoles) and 5-acetyl-2,2-dimethyl-1,3-dioxane-4,6-dione (9.3g, 50mmoles) in benzene (150ml) were heated at reflux for 4 hours. The solvent was evaporated and the residue chromatographed on silica eluting with ethyl acetate/petroleum ether (60-80°) mixtures to give the title compound (7.9g).

$M^+$ 174; nmr delta ($CDCl_3$) 1.97 (s,3H).

Example FF

Dicyclopropylmethyl 3-oxobutanoate and (E) 4-cyclopropyl-3-butenyl 3-oxobutanoate

Dicyclopropylmethanol (5.08g, 43mmoles) and 5-acetyl-2,2-dimethyl-1,3-dioxane-4,6-dione (9g, 48mmoles) in benzene (150ml) were heated at reflux for 3.5 hours. The solvent was evaporated and the residue chromatographed on silica eluting with ethyl acetate/petroleum ether (60-80°) mixtures to give first the dicyclopropylmethyl ester (1.11g) (nmr delta ($CDCl_3$) 4.0 (b,1H), 3.46 (s,2H), 2.29 (s,3H)) and then the cyclopropylbutenyl ester

(1.44g) (nmr delta ($CDCl_3$) 5.5-5.0 (m,2H), 3.5 (s,2H), 2.4 (s,3H)).

Example GG

2,3-Dihydro-2-indenyl 3-oxobutanoate

Prepared by the method of Example EE mp 51.5-2.5° Nmr delta ($CDCl_3$) 3.42 (s,2H), 2.22 (s,3H).

Example HH

(3-Methyl-3-thietanyl)methyl 3-oxobutanoate

Diketene (1.9g, 22.6mmoles) was added dropwise to a stirred mixture of triethylamine (1 drop) and 3-methyl-3-thietanemethanol (2.65g, 22.4moles) at 80°-85°. After heating for 3 hours at 95°, the reaction mixture was cooled to give the title compound (4.5g).

$M^+$, 202; nmr delta ($CDCl_3$) 2.30 (S,3H) and 1.32 (s,3H).

Example II

(3-Methyl-3-oxetanyl)methyl 3-oxobutanoate

Prepared by the method of Example FF Nmr delta ($CDCl_3$) 1.35 (3H,s), 2.29 (3H,s).

Example JJ

6-(5-Phenyl-3-pyrazolyoxy)hexyl 4-fluoro-3-oxobutanoate

6-(5-Phenyl-3-pyrazolyloxy)-1-hexanol (1.94g, 7.4mmoles), methyl 4-fluoro-3-oxobutanoate (1.0g,

7.4mmoles) and toluene (20ml) were heated at reflux for 3 hours. Methyl 4-fluoro-3-oxobutanoate (0.2g, 1.5mmoles) was added and the heating continued for a further hour. The solvent was evaporated and the residue purified by chromatography on silica eluting with ethyl acetate/petroleum ether (60-80$^{o}$) mixtures to give the title compound (1.8g).

$M^+$, 362; nmr delta ($CDCl_3$) 6.1 (s,1H) and 5.0 (d,2H).

Example KK

3-(Thietanyl-S,S-dioxide) 3-oxobutanoate

3-Thietanyl 3-oxobutanoate (2.17g, 12.5mmoles) and 3-chlorobenzeneperoxoic acid (4.3g, 25mmoles) in dichloromethane (60ml) were stirred at room temperature. Further peracid (2x2.15g) was added after 16 hours and 20 hours. After a further 2 hours, the organic solution was washed with sodium bicarbonate solution and sodium bisulphite solution. The aqueous extracts were saturated with sodium bicarbonate and exhaustively extracted with ethyl acetate/dichloromethane. The organic extracts were dried ($MgSO_4$) and the solvent evaporated to give the title compound (1.5g) mp 55-6$^{o}$.

$M^+$, 206; nmr delta ($CDCl_3$) 2.29 (s,3H).

Example LL

Ethyl 7-(1,3-dihydro-1,3-dioxo-2H-isoindol-2-yl)-3-

oxoheptanoate

Thionyl chloride (20ml) was added to 5-(1,3-dihydro-1,3-dioxo-2H-isoindol-2-yl)pentanoic acid (18.35g, 74mmoles) in dichloromethane (100ml) and the mixture heated at reflux for 1 hour. The solvent was evaporated and the residue titurated with petroleum ether (60-80$^{o}$) and the solid acid chloride filtered off. This solid was dissolved in dichloromethane (75ml) and added slowly between 0$^{o}$ and 5$^{o}$ to a stirred solution of 2,2-dimethyl-1,3-dioxane-4,6-dione (11.7g, 81mmoles) and pyridine (17.2ml, 22mmoles) in dichloromethane (100ml). After 20 hours, the reaction mixture was poured onto dilute hydrochloric acid, the organic layer was separated, washed with brine, dried ($MgSO_4$) and the solvent evaporated. Extraction with ethyl acetate/ether (1:4) and removal of solvent gave 2-(5-(2,2-dimethyl-4,6-dioxo-1,3-dioxan-5-yl)pentyl)-1,3-dihydro-2H-isoindole-1,3-dione (15.8g). This compound (8g) and ethanol (50ml) were heated at reflux for 2 hours. The solvent was evaporated and the residue dissolved in ether. The ethereal solution was washed with water, sodium bicarbonate solution and water, dried ($MgSO_4$) and the solvent removed to give the title compound (6.6g).

$M^+$, 317; nmr delta ($CDCl_3$) 1.28 (t,3H), 3.44 (s,2H), 4.2 (q,2H).

Example MM

1-Methylethyl 7-(1,3-dihydro-1,3-dioxo-2H-isoindol-2-yl)-3-oxoheptanoate

Prepared by the method of Example LL.

$M^+$, 331; nmr delta ($CDCl_3$) 1.25 (d,6H), 3.4 (s,2H).

Example NN

1-Methylethyl 4-(2-(1,3-dihydro-1,3-dioxo-2H-isoindol-2-yl)ethoxy)-3-oxobutanoate

1,3-Dihydro-1,3-dioxo-2H-isoindole-2-ethanol (8.6g, 45mmoles) was added to a stirred suspension of sodium hydride (4.3g of 50%, 90mmoles) in tetrahydrofuran (100ml) at -10°. 1-Methylethyl 4-chloro-3-oxobutanoate (8.0g, 45mmoles) in tetrahydrofuran (80ml) was added and the mixture stirred at room temperature for 26 hours. Ether and hydrochloric acid (1M) were added and the ether layer was separated, washed with sodium bicarbonate (5% solution), dried ($MgSO_4$) and the solvent evaporated. Chromatography on silica eluting with ethyl acetate/petroleum ether (60-80°) gave the title compound (1.3g).

$M^+$, 333; nmr delta ($CDCl_3$) 4.16 (s,2H), 3.44 (s,2H).

Example OO

2,2,2-Trichloroethyl 4-chloro-3-oxobutanoate

4-Chloro-3-oxobutanoyl chloride (36.9g, 0.238moles) in tetrachloromethane (100ml) was added with stirring to a solution at 7$^{o}$ of 2,2,2-trichloroethanol (45.9ml, 0.476moles). After 20 minutes the solvent was evaporated and the residue distilled to give the title compound (38g) (bp 160-4$^{o}$ at 15mmHg).

Example PP

2,2,2-Trichloroethyl 4-(2-(1,3-dihydro-1,3-dioxo-2H-isoindol-2-yl)ethoxy)-3-oxobutanoate

Prepared by the method of Example NN. $M^+$ 422/4/6/8.

Example QQ

3-Thietanyl 3-amino-2-butenoate

3-Thietanyl 3-oxobutanoate (4.35g, 25mmoles) and ammonium acetate (2.0g, 26mmoles) in t.butanol (75ml) were heated at 55-60$^{o}$ for 3.5 hours. The solvent was evaporated to give the title compound (3.8g) mp 75-6$^{o}$.

$M^+$, 173; nmr delta ($CDCl_3$) 2.32 (s,3H).

Example RR

(3-Methyl-3-thietanyl)methyl 3-amino-2-butenoate

Prepared by the method of Example QQ. $M^+$, 201; nmr delta ($CDCl_3$) 1.92(s,3H) and 1.32 (s,3H).

What we claim is:-

1. A compound of formula I,

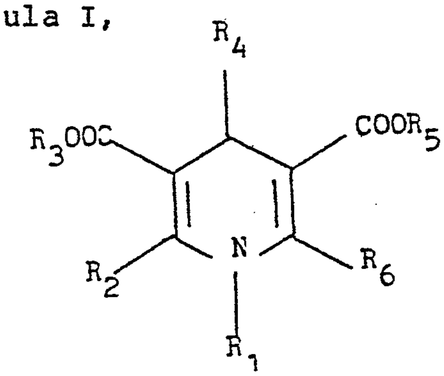

I

in which $R_1$ is hydrogen or alkyl C1 to 6,

$R_3$ and $R_5$, which may be the same or different, are each $-(CH_2)_x-(CHR_{34})_yR_{35}$,

x is a whole number from 0 to 7 inclusive,

y is 0 or 1,

$R_{34}$ is hydrogen, alkyl C1 to 6 or cycloalkyl containing up to and including 8 carbon atoms,

$R_{35}$ is hydrogen; alkyl C1 to 6 optionally substituted by hydrogen, by alkoxy C1 to 6, by alkanoyl C1 to 6, or by hydroxy; a 4, 5 or 6 membered oxygen, nitrogen or $S(O)_n$ containing heterocyclic ring which ring is optionally substituted by alkyl C1 to 6; cycloalkyl or cycloalkenyl containing up to and including 8 carbon atoms which cycloalkyl is in turn optionally bridged, substituted by alkyl C1 to 6 or is fused to a phenyl ring; a group $-CH=CHR_{13}$ in which $R_{13}$ is cycloalkyl containing up to and including 8 carbon atoms; or is an alkyl C1 to 6 amino group in which the alkyl group(s) are

optionally substituted by phenyl; or, when x is a whole number from 1 to 7 inclusive $R_{35}$ is CN, $-OSO_2R_{33}$ or a group of formula II or III,

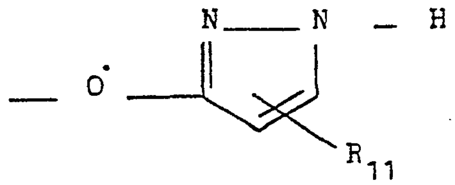

II

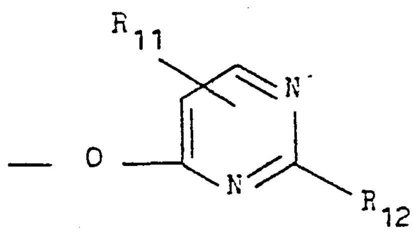

III

in which $R_{11}$ and $R_{12}$, which may be the same or different, are each alkyl C1 to 6 or phenyl, and

$R_{33}$ is alkyl C1 to 6 or alkyl C1 to 6-phenyl,

$R_2$ and $R_6$, which may be the same or different, are each CN, CHO or alkyl C1 to 6 optionally interrupted by an oxygen atom and optionally substituted by halogen, by hydroxy, by -COOH or an alkyl C1 to 6 ester thereof, by a C1 to 6 betaketoester group, by a 2-amino-6-hydroxy-pyridmidin-4-yl group or by a group $-NR_{14}R_{15}$,

$R_{14}$ and $R_{15}$, which may be the same or different, are each hydrogen, alkyl C1 to 6, -C(=NCN)Salkyl C1 to 6 or a group of formula IV,

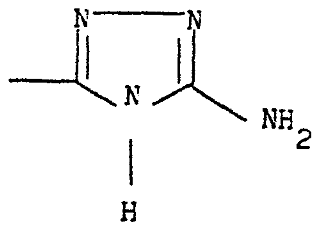

IV

or together form a chain $-CR_{16}=N-CR_{17}=CR_{18}-$, $-CH_2CH_2N(R_{19})CH_2CH_2-$ or -CO(o-phenylene)CO-,

$R_{16}$, $R_{17}$ and $R_{18}$, which may be the same or different, are each alkyl C1 to 6,

$R_{19}$ is hydrogen; alkyl C1 to 6 optionally substituted by phenyl which in turn is optionally substituted by halogen; $-CONR_{20}R_{21}$; or a 5 membered unsaturated ring containing nitrogen and optionally also sulphur,

$R_{20}$ and $R_{21}$, which may be the same or different, are each hydrogen or alkyl C1 to 6,

$R_4$ is a phenyl group carrying 3 or 4 substituents or is an unsaturated 5 membered heterocyclic group containing a single hetero atom selected from oxygen, sulphur or nitrogen, $R_4$ being substituted by $CF_3$, $XR_{22}$, nitro, halogen, CN, alkyl C1 to 6, formyl, dioxalanyl, $-N(R_{23})COR_{24}$, $-CONR_{25}R_{26}$, or amino which is optionally substituted by phenylsulphonyl or by trifluoroacetyl, and $R_4$ carries a substituent on the atom adjacent to the atom through which $R_4$ is connected

to the remainder of the molecule,

$R_{22}$ is phenyl or alkyl C1 to 6 optionally substituted by halogen,

X is O or $S(O)_n$,

n is 0, 1 or 2, and

$R_{23}$, $R_{24}$, $R_{25}$ and $R_{26}$, which may be the same or different, are each hydrogen or alkyl C1 to 6,

provided that i) when $R_1$ is hydrogen or methyl, $R_2$ is $CH_2F$, $R_3$ is methyl and $R_5$ is 1-methylethyl or cyclopentyl, then $R_4$ is not 2-(trifluoromethyl)-3-chloro-6-fluorophenyl,

ii) when $R_1$ is hydrogen, $R_2$ is $CH_2F$, $R_3$ is methyl and $R_5$ is 1-methylethyl then $R_4$ is not 2,3-dichloro-6-fluorophenyl, and

iii) when $R_4$ is a heterocyclic group then at least one of $R_2$ and $R_6$ is other than alkyl or alkoxy-alkyl,

and pharmaceutically acceptable acid addition salts of those compounds containing a basic nitrogen atom.

2. A compound according to Claim 1, wherein $R_4$ is of formula XIII,

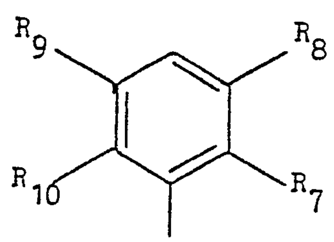

XIII

 

in which $R_7$ is trifluoromethyl, phenylthio, fluoro, phenylsulphonyl, nitro or chloro,

$R_8$ is nitrile, chloro, fluoro, phenylthio, methylthio, phenylsulphonylamino, trifluoroacetyl, trifluoromethyl, methoxy, propylthio, nitro or amino,

$R_9$ is hydrogen, fluoro or chloro, and

$R_{10}$ is hydrogen, fluoro, methoxy or chloro.

3. A compound according to Claim 2, wherein $R_1$ is hydrogen, $R_2$ is $-CH_2F$ or $CH_3$, $R_3$ is methyl, $R_5$ is 3-thietanyl, 1-methylethyl, cyclobutyl, 3-thietanyl-S,S-dioxide, 6-(5-phenyl-3-pyrazolyloxy)hexyl, cyclopropylmethyl, 6-((6-methyl-2-phenyl-4-pyrimidinyl)oxy) hexyl or ethyl, $R_6$ is methyl or (2-aminoethoxy)methyl and $R_4$ is 3-nitro-2-thienyl, or a group of formula XIII in which $R_7$ is $CF_3$, $R_8$ is Cl or CN, $R_9$ is hydrogen and $R_{10}$ is F.

4. 3-Methyl 5-(3-thietanyl) 4-(3-cyano-6-fluoro-2--(trifluoromethyl)phenyl)-2-(fluoromethyl)-1,4-dihydro-6-methyl-3,5-pyridinedicarboxylate,

3-Methyl 5-(6-((6-methyl-2-phenyl-4-pyrimidinyl)oxy) hexyl) 4-(3-chloro-6-fluoro-2-(trifluoromethyl)phenyl)-2-(fluoromethyl)-1,4-dihydro-6-methyl-3,5-pyridine-dicarboxylate or a pharmaceutically acceptable salt thereof, or

3-Ethyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate or a pharmaceutically acceptable salt thereof.

5. Methyl 1-methylethyl 4-(3-chloro-6-fluoro-2-(trifluoromethyl)phenyl)-1,4-dihydro-2,6-dimethyl-3,5-pyridinedicarboxylate,

3-Methyl 5-(1-methylethyl) 2-(fluoromethyl)-1,4-dihydro-6-methyl-4-(3-nitro-2-thienyl)-3,5-pyridinedicarboxylate,

3-Methyl 5-(1-methylethyl) 4-(3-cyano-6-fluoro-2-(trifluoromethyl)phenyl)-2-(fluoromethyl)-1,4-dihydro-6-methyl-3,5-pyridinedicarboxylate,

5-Cyclobutyl 3-methyl 4-(3-cyano-6-fluoro-2-(trifluoromethyl)-phenyl)-2-(fluoromethyl)-1,4-dihydro-6-methyl-3,5-pyridinedicarboxylate,

3-Methyl 5-(3-thietanyl-S,S-dioxide) 4-(3-chloro--6-fluoro-2-(trifluoromethyl)phenyl)-2-(fluoromethyl)-1,4-dihydro-6-methyl-3,5-pyridinedicarboxylate,

3-Methyl 5-(6-(5-phenyl-3-pyrazolyloxy)hexyl) 4-(3-cyano-6-fluoro-2-(trifluoromethyl)phenyl)-2-(fluoromethyl)-1,4-dihydro-6-methyl-3,5-pyridinedicarboxylate,

3-Methyl 5-(1-methylethyl) 4-(3,6-difluoro-2-(phenylthio)phenyl)-2-(fluoromethyl)-1,4-dihydro-6-methyl--3,5-pyridinedicarboxylate,

5-Cyclobutyl 3-methyl 4-(3,6-difluoro-2-(trifluoromethyl)phenyl)-2-(fluoromethyl)-1,4-dihydro-6-methyl-3,5-pyridinedicarboxylate,

3-Methyl 5-(1-methylethyl) 4-(2,5-difluoro-3-(phenylthio)phenyl)-2-(fluoromethyl)-1,4-dihydro-6-methyl-3,5-pyridinedicarboxylate,

5-Cyclopentyl 3-methyl 4-(3-cyano-6-fluoro-2-(trifluoromethyl)phenyl)-2-(fluoromethyl)-1,4-dihydro-6-methyl-3,5-pyridinedicarboxylate,

5-(2-Cyanoethyl) 3-methyl 4-(3-cyano-6-fluoro-2-(trifluoromethyl)phenyl)-2-(fluoromethyl)-1,4-dihydro-6-methyl-3,5-pyridinedicarboxylate,

3-Methyl 5-(2-methylpropyl) 4-(3-cyano-6-fluoro-2-(trifluoromethyl)phenyl)-2-(fluoromethyl)-1,4-dihydro-6-methyl-3,5-pyridinedicarboxylate,

3-Methyl 5-(1-methylethyl) 2-(fluoromethyl)-4-(6-fluoro-3-(methylthio)-2-(trifluoromethyl)phenyl)-1,4-dihydro-6-methyl-3,5-pyridinedicarboxylate,

3-Methyl 5-(1-methylethyl) 2-(fluoromethyl)-4-(6-fluoro-3-((phenylsulphonyl)amino)-2-(trifluoromethyl)phenyl)-1,4-dihydro-6-methyl-3,5-pyridinedicarboxylate,

3-Methyl 5-(1-methylethyl) 2-(fluoromethyl)-4-(6-fluoro-3-((2,2,2-trifluoroacetyl)amino)-2-(trifluoromethyl)phenyl)-1,4-dihydro-6-methyl-3,5-pyridinedicarboxylate,

3-Methyl 5-(l-methylethyl) 4-(6-fluoro-2,3-bis-(trifluoromethyl)phenyl)-2-(fluoromethyl)-1,4-dihydro-6-methyl-3,5-pyridinedicarboxylate,

3-Methyl 5-(l-methylethyl) 4-(6-fluoro-3-methoxy-2-(trifluoromethyl)phenyl)-2-(fluoromethyl)-1,4-dihydro-6-methyl-3,5-pyridinedicarboxylate,.

3-Methyl 5-(l-methylethyl) 2-(fluoromethyl)-4-(6-fluoro-3-(propylthio)-2-(trifluoromethyl)phenyl)-1,4-dihydro-6-methyl-3,5-pyridinedicarboxylate,

5-Cyclobutyl 3-methyl 4-(2,6-difluoro-3-nitrophenyl)-2-(fluoromethyl)-1,4-dihydro-6-methyl-3,5-pyridinedicarboxylate,

3-Methyl 5-(l-methylethyl) 4-(3-cyano-2,6-difluorophenyl)-2-(fluoromethyl)-1,4-dihydro-6-methyl-3,5-pyridinedicarboxylate,

3-Methyl 5-(l-methylethyl) 4-(3-chloro-6-methoxy-2-(trifluoromethyl)phenyl)-2-(fluoromethyl)-1,4-dihydro-6-methyl-3,5-pyridinedicarboxylate,

Dimethyl 1,4-dihydro-2,6-dimethyl-4-(2,3,5,6-tetrafluorophenyl)-3,5-pyridinedicarboxylate,

Dimethyl 4-(3-chloro-6-fluoro-2-(trifluoromethyl)-phenyl)-1,4-dihydro-2,6-dimethyl-3,5-pyridinedicarboxylate,

3-Methyl 5-(l-methylethyl) 2-(fluoromethyl)-1,4-dihydro-6-methyl-4-(2,4,5-trichlorothienyl)-3,5-pyridinedicarboxylate,

3-Methyl 5-(1-methylethyl) 4-(3,6-difluoro-2-(phenylsulphonyl)phenyl)-2-(fluoromethyl)-1,4-dihydro-6-methyl-3,5-pyridinedicarboxylate,

Di(1-methylethyl) 4-(3-chloro-6-fluoro-2-(trifluoromethyl)phenyl)-1,4-dihydro-2,6-dimethyl-3,5-pyridinedicarboxylate,

3-Methyl 5-(1-methylethyl) 2-(fluoromethyl)-1,4-dihydro-6-methyl-4-(1-methylpyrryl)-3,5-pyridinedicarboxylate,

3-Methyl 5-(3-thietanyl) 4-(3,6-difluoro-2-(trifluoromethyl)phenyl)-2-(fluoromethyl)-1,4-dihydro-6-methyl-3,5-pyridinedicarboxylate,

3-Methyl 5-((3-methyl-3-thietanyl)methyl) 4-(3-cyano-6-fluoro-2-(trifluoromethyl)phenyl)-2-(fluoromethyl)-1,4-dihydro-6-methyl-3,5-pyridinedicarboxylate,

3-Methyl 5-(1-methylethyl) 4-(3-(1,3-dioxolan-2-yl)-2-thienyl)-2-(fluoromethyl)-1,4-dihydro-6-methyl-3,5-pyridinedicarboxylate,

3-Methyl 5-(1-methylethyl) 4-(2,6-difluoro-3-nitrophenyl)-2-(fluoromethyl)-1,4-dihydro-6-methyl-3,5-pyridinedicarboxylate,

3-Methyl 5-(1-methylethyl) 4-(3-chloro-6-fluoro-2-nitrophenyl)-2-(fluoromethyl)-1,4-dihydro-6-methyl-3,5-pyridinedicarboxylate,

3-Methyl 5-(1-methylethyl) 4-(3,5-dichloro-2-fluoro--6-(trifluoromethyl)phenyl)-2-(fluoromethyl)-1,4-dihydro--6-methyl-3,5-pyridinedicarboxylate,

3-Methyl 5-(1-methylethyl) 4-(3,6-dichloro-2-(trifluormethyl)phenyl)-2-(fluoromethyl)-1,4-dihydro-6-methyl-3,5-pyridinedicarboxylate,

Diethyl 4-(2,3-dichloro-6-fluorophenyl)-1,4-dihydro-2,6-dimethyl-3,5-pyridinedicarboxylate,

Diethyl 4-(3-chloro-6-fluoro-2-(trifluoromethyl)-phenyl)-1,4-dihydro-2,6-dimethyl-3,5-pyridinedicarboxylate,

3-Methyl 5-(1-methylethyl) 4-(4,5-dichloro-3-thienyl)--2-(fluoromethyl)-1,4-dihydro-6-methyl-3,5-pyridine-dicarboxylate,

3-Methyl 5-(1-methylethyl) 2-(fluoromethyl)-1,4-dihydro-6-methyl-4-(3-methyl-2-thienyl)-3,5-pyridine-dicarboxylate,

3-Methyl 5-(1-methylethyl) 4-(3,6-difluoro-2--(trifluoromethyl)phenyl)-2-(fluoromethyl)-1,4-dihydro-6-methyl-3,5-pyridinedicarboxylate,

3-Methyl 5-(1-methylethyl) 2-(fluoromethyl)-4-(6-fluoro-3-nitro-2-(trifluoromethyl)phenyl)-1,4-dihydro-6--methyl-3,5-pyridinedicarboxylate,

3-Methyl 5-(1-methylethyl) 4-(2,5-difluoro-3-(trifluoromethyl)phenyl)-2-(fluoromethyl)-1,4-dihydro-6-methyl-3,5-pyridinedicarboxylate,

3-Methyl 5-(1-methylethyl) 4-(2-chloro-6-fluoro-3-nitrophenyl)-2-(fluoromethyl)-1,4-dihydro-6-methyl-3,5-pyridinedicarboxylate,

3-Methyl 5-(1-methylethyl) 2-(fluoromethyl)-1,4-dihydro-6-methyl-4-(2,3,6-trifluorophenyl)-3,5-pyridinedicarboxylate,

3-Methyl 5-(1-methylethyl) 4-(2,5-dichloro-3-(trifluoromethyl)phenyl)-2-(fluoromethyl)-1,4-dihydro-6-methyl-3,5-pyridinedicarboxylate,

3-Methyl 5-((3-methyl-3-oxetanyl)methyl) 4-(3-chloro-6-fluoro-2-(trifluoromethyl)phenyl)-2-(fluoromethyl)-1,4-dihydro-6-methyl-3,5-pyridinedicarboxylate,

5-(Dicyclopropylmethyl) 3-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-((3-methyl-3-oxetanyl)methyl) 4-(3-cyano-6-fluoro-2-(trifluoromethyl)phenyl)-2-(fluoromethyl)-1,4-dihydro-6-methyl-3,5-pyridinedicarboxylate,

3-Methyl 5-(3-oxetanyl) 4-(3-cyano-6-fluoro-2-(trifluoromethyl)phenyl)-2-(fluoromethyl)-1,4-dihydro-6-methyl-3,5-pyridinedicarboxylate,

(E) 5-(4-Cyclopropyl-3-butenyl) 3-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(3-oxetanyl) 4-(3,6-difluoro-2-(trifluoromethyl)phenyl)-2-(fluoromethyl)-1,4-dihydro-6-

methyl-3,5-pyridinedicarboxylate,

Ethyl methyl 4-(2,3-dichloro-6-fluorophenyl)-1,4-dihydro-2,6-dimethyl-3,5-pyridinedicarboxylate,

3-Methyl 5-(3-thietanyl) 4-(3-chloro-6-fluoro-2-(trifluoromethyl)phenyl)-2-(fluoromethyl)-1,4-dihydro-6-methyl-3,5-pyridinedicarboxylate,

5-(Cyclopropylmethyl) 3-methyl 4-(3-cyano-6-fluoro-2-(trifluoromethyl)phenyl)-2-(fluoromethyl)-1,4-dihydro-6-methyl-3,5-pyridinedicarboxylate,

5-(Cyclobutylmethyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-(Cyclopropylmethyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-(Cyclopropylmethyl) 3-methyl 4-(3,6-difluoro-2-(trifluoromethyl)phenyl)-2-(fluoromethyl)-1,4-dihydro-6-methyl-3,5-pyridinedicarboxylate,

3-Methyl 5-((1-methylcyclopropyl)methyl) 4-(3-cyano-6-fluoro-2-(trifluoromethyl)phenyl)-2-(fluoromethyl)-1,4-dihydro-6-methyl-3,5-pyridinedicarboxylate,

3-Methyl 5-(2-(methyl(phenylmethyl)amino)ethyl) 4-(3-chloro-6-fluoro-2-(trifluoromethyl)phenyl)-2-(fluoromethyl)-1,4-dihydro-6-methyl-3,5-pyridinedicarboxylate,

3-Methyl 5-(6-(5-phenyl-3-pyrazolyloxy)hexyl) 4-(3-chloro-6-fluoro-2-(trifluoromethyl)phenyl)-1,4-dihydro-2,6-dimethyl-3,5-pyridinedicarboxylate,

3-Methyl 5-(6-(5-phenyl-3-pyrazolyloxy)hexyl) 4-(3-cyano-6-fluoro-2-(trifluoromethyl)phenyl)-1,4-dihydro-2,6-dimethyl-3,5-pyridinedicarboxylate,

3-Methyl 5-(1-methylethyl) 4-(3-cyano-6-fluoro-2-(trifluoromethyl)phenyl)-1,4-dihydro-2,6-dimethyl-3,5-pyridinedicarboxylate,

5-Cyclobutyl 3-methyl 4-(3,6-difluoro-2-(trifluoromethyl)phenyl)-1,4-dihydro-2,6-dimethyl-3,5-pyridinedicarboxylate,

5-Cyclobutyl 3-methyl 4-(3-cyano-6-fluoro-2-(trifluoromethyl)phenyl)-1,4-dihydro-2,6-dimethyl-3,5-pyridinedicarboxylate,

3-Methyl 5-(6-(5-phenyl-3-pyrazolyloxy)hexyl) 4-(3,6-difluoro-2-(trifluoromethyl)phenyl)-1,4-dihydro-2,6-dimethyl-3,5-pyridinedicarboxylate,

3-Methyl 5-(2-(methyl(phenylmethyl)amino)ethyl) 4-(3-chloro-6-fluoro-2-(trifluoromethyl)phenyl)-1,4-dihydro-2,6-dimethyl-3,5-pyridinedicarboxylate,

3-Methyl 5-(6-(5-methyl-3-pyrazolyloxy)hexyl) 4-(3-chloro-6-fluoro-2-(trifluoromethyl)phenyl)-1,4-dihydro-2,6-dimethyl-3,5-pyridinedicarboxylate,

3-Methyl 5-(1-methylethyl) 4-(3-chloro-6-fluoro-2-

(trifluoromethyl)phenyl)-1,4-dihydro-2-(hydroxymethyl)-6-methyl-3,5-pyridinedicarboxylate,

3-Methyl 5-(1-methylethyl) 4-(3-chloro-6-fluoro-2-(trifluoromethyl)phenyl)-2-formyl-1,4-dihydro-6-methyl -3,5-pyridinedicarboxylate,

3-Methyl 5-(cis-3-thietanyl-S-oxide) 4-(3-chloro-6-fluoro-2-(trifluoromethyl)phenyl)-2-(fluoromethyl)-1,4-dihydro-6-methyl-3,5-pyridinedicarboxylate,

3-methyl 5-(trans-3-thietanyl-S-oxide) 4-(3-chloro-6-fluoro-2-(trifluoromethyl)phenyl)-2-(fluoromethyl)-1,4-dihydro-6-methyl-3,5-pyridinedicarboxylate,

3-Methyl 5-(1-methylethyl) 4-(3-chloro-6-fluoro-2-(trifluoromethyl)phenyl)-2-cyano-1,4-dihydro-6-methyl -3,5-pyridinedicarboxylate,

3-Methyl 5-(1-methylethyl) 4-(3-cyano-2-thienyl)-2-(fluoromethyl)-1,4-dihydro-6-methyl-3,5-pyridinedicarboxylate,

3-Methyl 5-(6-((6-methyl-2-phenyl-4-pyrimidinyl)oxy) hexyl) 4-(3-chloro-6-fluoro-2-(trifluoromethyl)phenyl)-2-(fluoromethyl)-1,4-dihydro-6-methyl-3,5-pyridine-dicarboxylate,

3-Methyl 5-(6-(5-phenyl-3-pyrazolyloxy)hexyl) 4-(3,6-difluoro-2-(trifluoromethyl)phenyl)-2-(fluoromethyl) -1,4-dihydro-6-methyl-3,5-pyridinedicarboxylate,

3-Methyl 5-(6-(5-methyl-3-pyrazolyloxy)hexyl)

4-(3-chloro-6-fluoro-2-(trifluoromethyl)phenyl)-2-(fluoromethyl)-1,4-dihydro-6-methyl-3,5-pyridinedicarboxylate,

3-Methyl 5-(1-methylethyl) 4-(3,6-difluoro-2-(trifluoromethyl)phenyl)-2-formyl-1,4-dihydro-6-methyl-3,5-pyridinedicarboxylate,

3-Methyl 5-(1-methylethyl) 2-(fluoromethyl)-4-(3-formyl-2-thienyl)-1,4-dihydro-6-methyl-3,5-pyridinedicarboxylate,

3-Ethyl 5-methyl 2-(4-aminobutyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 2-(4-aminobutyl)-4-(3-chloro-6-fluoro-2-(trifluoromethyl)phenyl)-6-(fluoromethyl)-1,4-dihydro-3,5-pyridinedicarboxylate ,

3-Methyl 5-(1-methylethyl) 4-(3-amino-6-fluoro-2-(trifluoromethyl)phenyl)-2-(fluoromethyl)-1,4-dihydro-6-methyl-3,5-pyridinedicarboxylate,

5-(2-Cyanoethyl) 3-methyl 4-(3-chloro-6-fluoro-2-(trifluoromethyl)phenyl)-2-(fluoromethyl)-1,4-dihydro-6-methyl-3,5-pyridinedicarboxylate,

4-(3-Chloro-6-fluoro-2-(trifluoromethyl)phenyl)-6-(fluoromethyl)-1,4-dihydro-5-(methoxycarbonyl)-2-methyl-3-pyridinecarboxylic acid,

4-(3-Cyano-6-fluoro-2-(trifluoromethyl)phenyl)-6-

(fluoromethyl)-1,4-dihydro-5-(methoxycarbonyl)-2-methyl-3-pyridinedicarboxylic acid,

5-(2-Cyanoethyl) 3-methyl 4-(3,6-difluoro-2-trifluoromethyl)phenyl)-2-(fluoromethyl)-1,4-dihydro-6-methyl-3,5-pyridinedicarboxylate,

4-(3,6-Difluoro-2-(trifluoromethyl)phenyl)-6-(fluoromethyl)-1,4-dihydro-5-(methoxycarbonyl)-2-methyl-3-pyridinecarboxylic acid,

3-Methyl 5-(6-(((4-methylphenyl)sulphonyl)oxy)hexyl) 4-(3-chloro-6-fluoro-2-(trifluoromethyl)phenyl)-2-(fluoromethyl)-1,4-dihydro-6-methyl-3,5-pyridinedicarboxylate,

3-Methyl 5-(6-(((4-methylphenyl)sulphonyl)oxy)hexyl) 4-(3,6-difluoro-2-(trifluoromethyl)phenyl)-2-(fluoromethyl)-1,4-dihydro-6-methyl-3,5-pyridinedicarboxylate,

3-Ethyl 5-methyl 4-(3-chloro-6-fluoro-2-(trifluoromethyl)phenyl-2-((2-(1,3-dihydro-1,3-dioxo-2H-isoindol-2-yl)ethoxy)methyl)-6-(fluoromethyl)-1,4-dihydro-3,5-pyridinedicarboxylate,

3-Ethyl 5-methyl 4-(3-chloro-6-fluoro-2-(trifloromethyl)phenyl)-2-(4-(1,3-dihydro-1,3-dioxo-2H-isoindol-2-yl)butyl)-6-(fluoromethyl)-1,4-dihydro-3,5-pyridinedicarboxylate,

5-Methyl 3-(1-methylethyl) 4-(3-chloro-6-fluoro-2-(trifluoromethyl)phenyl)-2-(4-(1,3-dihydro-1,3-dioxo-2H-isoindol-2-yl)butyl)-6-(fluoromethyl)-1,4-dihydro-3,5-pyridinedicarboxylate,

3-Ethyl 5-methyl 2-((2-aminoethoxy)methyl)-4-(3-chloro-6-fluoro-2-(trifluoromethyl)phenyl)-1,4-dihydro-6-methyl-3,5-pyridinedicarboxylate,

5-Methyl 3-(6-(5-phenyl-3-pyrazolyloxy)hexyl) 4-(3-chloro-6-fluoro-2-(trifluoromethyl)phenyl)-2-(fluoromethyl)-1,4-dihydro-6-methyl-3,5-pyridinedicarboxylate,

3-Ethyl 5-methyl 2-(4-((5-amino-1H-1,2,4-triazol-3-yl)amino)butyl)-4-(3-chloro-6-fluoro-2-(trifluoromethyl) phenyl)-6-(fluoromethyl)-1,4-dihydro-3,5-pyridinedicarboxylate,

5-Ethyl 3-methyl 4-(3-chloro-6-fluoro-2-(trifluoromethyl)phenyl)-2-(fluoromethyl)-1,4-dihydro-2-(4-(2,4,5-trimethyl-1H-imidazol-1-yl)butyl)-3,5-pyridinedicarboxylate,

3-Ethyl 5-methyl 2-((2-((5-amino-1H-1,2,4-triazol-3-yl)amino)ethoxy)methyl)-4-(3-chloro-6-fluoro-2-(trifluoromethyl)phenyl)-6-(fluoromethyl)-1,4-dihydro-3,5-pyridinedicarboxylate,

3-Ethyl 5-methyl 4-(3-chloro-6-fluoro-2-(trifluoromethyl)phenyl)-2-((2-(((cyanoimino)methylthio) methyl)amino)ethoxy)methyl)-6-(fluoromethyl)-1,4-dihydro-6-methyl-3,5-pyridinedicarboxylate,

5-Ethyl 3-methyl 4-(3-chloro-6-fluoro-2-(trifluoromethyl)phenyl)-2-(fluoromethyl)-1,4-dihydro-6-((2-(2,4,5-trimethyl-1H-imidazol-1-yl)ethoxy)methyl)-3,5-pyridinedicarboxylate,

3-Ethyl 5-methyl 4-(3-chloro-6-fluoro-2-(trifluoromethyl)phenyl)-2-((2-(dimethylamino)ethoxy) methyl)-6-(fluoromethyl)-1,4-dihydro-3,5-pyridinedicarboxylate,

5-Cyclobutyl 3-methyl 4-(3-cyano-6-fluoro-2-(trifluoromethyl)phenyl)-2-(fluoromethyl)-1,4-dihydro-1,6-dimethyl-3,5-pyridinedicarboxylate,

5-Methyl 3-(1-methylethyl) 2-((2-(1,3-dihydro-1,3-dioxo-2H-isoindol-2-yl)ethoxy)methyl)-6-(fluoromethyl)-1,4-dihydro-4-(2,3,6-trifluorophenyl)-3,5-pyridinedicarboxylate,

5-Methyl 3-(1-methylethyl) 2-((2-aminoethoxy)methyl) -6-(fluoromethyl)-1,4-dihydro-4-(2,3,6-trifluorophenyl)-3,5 -pyridinedicarboxylate,

3-Ethyl 5-methyl 4-(3-chloro-6-fluoro-2-(trifluoromethyl)phenyl)-2-((2-(4-((4-chlorophenyl)methyl) -1-piperazinyl)ethoxy)methyl)-6-(fluoromethyl)-1,4-dihydro -3,5-pyridinedicarboxylate,

5-Ethyl 3-methyl 4-(3-chloro-6-fluoro-2-(trifluoromethyl)phenyl)-1,4-dihydro-2-methyl-6-((2-(1-piperazinyl)ethoxy)methyl)-3,5-pyridinedicarboxylate,

5-Ethyl 3-methyl 4-(3-chloro-6-fluoro-2-

(trifluoromethyl)phenyl)-1,4-dihydro-2-methyl-6-((2-(4-((methylamino)carbonyl)-1-piperazinyl)ethoxy)methyl)-3,5-pyridinedicarboxylate,

5-Ethyl 3-methyl 4-(3-chloro-6-fluoro-2-(trifluoromethyl)phenyl)-2-(fluoromethyl)-1,4-dihydro-6-methyl-3,5-pyridinedicarboxylate,

5-Cyclobutyl 3-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(2-methylpropyl) 4-(3-chloro-6-fluoro-2-(trifluoromethyl)phenyl)-2-(fluoromethyl)-1,4-dihydro-6-methyl-3,5-pyridinedicarboxylate,

5-Methyl 3-(1-methylethyl) 4-(3-chloro-6-fluoro-2-(trifluoromethyl)phenyl)-2-(fluoromethyl)-1,4-dihydro-6-methyl-3,5-pyridinedicarboxylate,

3-Ethyl 5-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(3-oxetanyl) 4-(3-chloro-6-fluoro-2-(trifluoromethyl)phenyl)-2-(fluoromethyl)-1,4-dihydro-6-methyl-3,5-pyridinedicarboxylate,

3-Methyl 5-(tetrahydro-4H-pyran-4-yl) 4-(3-chloro-6-fluoro-2-(trifluoromethyl)phenyl)-2-(fluoromethyl)-1,4-dihydro-6-methyl-3,5-pyridinedicarboxylate,

3-Methyl 5-(2,2,2-trifluoroethyl) 4-(3-chloro-6-fluoro

-2-(trifluoromethyl)phenyl)-2-(fluoromethyl)-1,4-dihydro-6-methyl-3,5-pyridinedicarboxylate,

5-(2,3-Dihydro-2-indenyl) 3-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(2-methyl-1-(methylethyl)propyl) 4-(3-chloro-6-fluoro-2-(trifluoromethyl)phenyl)-2-(fluoromethyl)-1,4-dihydro-6-methyl-3,5-pyridinedicarboxylate,

5-(2,2-Dimethylpropyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-(1,1-Dimethylethyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-(2-Fluoro-1-(fluoromethyl)ethyl) 3-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(2,2,2-trifluoro-1-(trifluoromethyl)ethyl) 4-(3-chloro-6-fluoro-2-(trifluoromethyl)phenyl)-2-(fluoromethyl)-1,4-dihydro-6-methyl-3,5-pyridinedicarboxylate,

5-(Ethoxymethyl) 3-methyl 4-(3-chloro-6-fluoro-2-(trifluoromethyl)phenyl)-2-(fluoromethyl)-1,4-dihydro-6-

methyl-3,5-pyridinedicarboxylate,

3-Methyl 5-(2-oxopropyl) 4-(3-chloro-6-fluoro-2-(trifluoromethyl)phenyl)-2-(fluoromethyl)-1,4-dihydro-6-methyl-3,5-pyridinedicarboxylate,

5-(1-Ethylpropyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-(3-Cyclopentenyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-(3-Hydroxy-2-(hydroxymethyl)-2-methylpropyl) 3-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(cis-octahydro-endo-2-pentalenyl) 4-(3-chloro-6-fluoro-2-(trifluoromethyl)phenyl)-2-(fluoromethyl)-1,4-dihydro-6-methyl-3,5-pyridinedicarboxylate,

5-(2,2-Dimethyl-3-((methyl(phenylmethyl)amino)propyl) 3-methyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(1-methylethyl) 2-(difluoromethyl)-4-(3,6-difluoro-2-(trifluoromethyl)phenyl)-1,4-dihydro-6-methyl-3,5-pyridinedicarboxylate,

 

3-Methyl 5-(1-methylethyl) 4-(3-chloro-6-fluoro-2-(trifluoromethyl)phenyl)-2-(difluoromethyl)-1,4-dihydro-6-methyl-3,5-pyridinedicarboxylate,

5-(3-Azetidinyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-Methyl 3-(2,2,2-trichloroethyl) 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate,

4-(3-Chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-5-(methoxycarbonyl)-3-pyridinecarboxylic acid,

5-Methyl 3-(1-methylethyl) 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate,

5-Methyl 3-(1-methylethyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 4-(2,6-difluoro-3-nitrophenyl)-2-((2-(1,3-dihydro-1,3-dioxo-2H-isoindol-2-yl)ethoxy)methyl)-6-(fluoromethyl)-1,4-dihydro-3,5-pyridinedicarboxylate,

5-Methyl 3-(1-methylethyl) 2-((2-aminoethoxy)methyl) -4-(2,6-difluoro-3-nitrophenyl)-6-(fluoromethyl)-1,4-dihydro-3,5-pyridinedicarboxylate,

5-Ethyl 3-methyl 4-(3-chloro-6-fluoro-2-(trifluoromethyl)phenyl)-1,4-dihydro-2-methyl-6-((2-(4-(2-thiazolyl)-1-piperazinyl)ethoxy)methyl)-3,5-pyridinedicarboxylate,

5-Ethyl 3-methyl 4-(3-chloro-6-fluoro-2-(trifluoromethyl)phenyl)-2-(fluoromethyl)-1,4-dihydro-6-(4-methoxy-4-oxobutyl)-3,5-pyridinedicarboxylate,

4-(4-(3-Chloro-6-fluoro-2-(trifluoromethyl)phenyl)-3-(ethoxycarbonyl)-6-(fluoromethyl)-1,4-dihydro-5-(methoxycarbonyl)-2-pyridyl)butanoic acid,

3-Ethyl 5-methyl 4-(3-chloro-6-fluoro-2-(trifluoromethyl)phenyl)-2-(6-ethoxy-4,6-dioxohexyl)-6-(fluoromethyl)-1,4-dihydro-3,5-pyridinedicarboxylate,

3-Ethyl 5-methyl 2-(3-(2-amino-6-hydroxy-4-pyrimidyl) propyl)-4-(3-chloro-6-fluoro-2-(trifluoromethyl)phenyl)-6-(fluoromethyl)-1,4-dihydro-3,5-pyridinedicarboxylate,

5-Ethyl 3-methyl 4-(3-chloro-6-fluoro-2-(trifluoromethyl)phenyl)-2-(fluoromethyl)-1,4-dihydro-6-((2-methoxy-2-oxoethoxy)methyl)-3,5-pyridinedicarboxylate,

or a pharmaceutically acceptable acid addition salt of any one thereof which contains a basic nitrogen atom.

6. Use of a compound according to any one of the

preceding claims as a pharmaceutical.

7. A pharmaceutical formulation comprising a compound according to any one of Claims 1 to 5 in admixture with a pharmaceutically acceptable adjuvant, diluent or carrier.

8. A process for the production of a compound of formula I, as defined in Claim 1, or a pharmaceutically acceptable acid addition salt thereof,

which comprises

a) reaction of the compounds of formula V, VII, X and XIV,

$R_4CHO$ V

$R_5OOCCH_2COR_6$ VII

$R_3OOCCH_2COR_2$ X

$R_1NH_2$ XIV

or their partial condensates,

in which $R_1$, $R_2$, $R_3$, $R_4$, $R_5$ and $R_6$ are as defined in Claim 1,

b) production of a compound of formula I containing a group $-S(O)_m-$ in which m is 1 or 2 by selective oxidation of a corresponding compound of formula I in which m is 0 or 1,

c) production of a compound of formula I in which one or both of $R_2$ and $R_6$ is $-CHF_2$ or $-CH_2F$ by reaction of a corresponding compound of formula I in which one or both of $R_2$ and $R_6$ is -CHO or $-CH_2L$ respectively, where L

is -OH or a good leaving group, with a fluorinating agent,

d) production of a compound of formula I carrying a -CHO substituent by

i) selective hydrolysis of a corresponding compound of formula I carrying a $-CH(OR_{27})_2$ substituent,

in which $R_{27}$ is alkyl C1 to 6, or the two group $R_{27}$ together form a $-CH_2CH_2-$ chain, or

ii) selective oxidation of a corresponding compound of formula I carrying a $-CH_2OH$ substituent,

e) production of a compound of formula I in which one of $R_2$ and $R_6$ is $-CH_2OH$ by selective hydrolysis of a corresponding compound of formula I in which one of $R_2$ and $R_6$ is $-CH_2Z$ and Z is a good leaving group,

f) production of a compound of formula I carrying a group -CN by elimination of ROH from a corresponding compound of formula I carrying a group -CH=NOR, in which -OR is a good leaving group,

g) production of a compound of formula I in which at least one of $R_3$ and $R_5$ is hydrogen, or in which at least one of $R_2$ and $R_6$ carries a -COOH group, by reductive cleavage or hydrolysis of a corresponding compound of formula I in which at least one of $R_3$ and $R_5$ is other than hydrogen or in which at least one of $R_2$ and $R_6$ carries a carboxylic acid ester group,

h) production of a compound of formula I in which at

least one of $R_3$ and $R_5$ is other than hydrogen, or in which at least one of $R_2$ and $R_6$ carries a carboxylic acid ester group or a betaketoester group, by esterification or transesterification of a corresponding compound of formula I in which at least one of $R_3$ and $R_5$ is hydrogen or is a group $R_3$ or $R_5$ other than that desired in the end product, or in which at least one of $R_2$ and $R_6$ carries a -COOH group,

i) production of a compound of formula I in which at least one of $R_2$ and $R_6$ is methyl by selective reduction of a corresponding compound of formula I in which at least one of $R_2$ and $R_6$ is $-CH_2Y$ and Y is a group reducible to hydrogen,

j) production of a compound of formula I carrying an $-NH_2$ substituent by removal of a protecting group from a corresponding compound carrying a protected amino group,

k) production of a compound of formula I in which at least one of $R_3$ and $R_5$ is alkyl substituted by a group of formula II or III, by reaction of a corresponding compound of formula I in which at least one of $R_3$ and $R_5$ is alkyl substituted by a good leaving group, with a compound of formula XI or XII respectively,

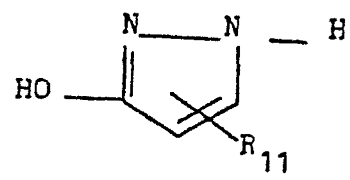

XI

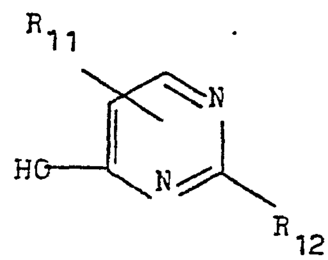

XII

or a tautomer or salt thereof,

in which $R_{11}$ and $R_{12}$ are as defined in Claim 1,

l) production of a compound of formula I in which at least one of $R_3$ and $R_5$ represents alkyl substituted by a group $-CH(R_{36})(CH_2OH)-CH_2OH$, in which $R_{36}$ represents hydrogen or alkyl, by selective hydrolysis of a corresponding compound of formula I in which at least one of $R_3$ and $R_5$ represents alkyl substituted by 3-oxetanyl which in turn is optionally substituted in the 3-position by alkyl,

m) production of a compound of formula I in which $R_1$ is alkyl C1 to 6, by C1 to 6 alkylation of a corresponding compound of formula I in which $R_1$ is hydrogen,

n) production of a compound of formula I in which one of

$R_{14}$ and $R_{15}$ is a group of formula IV, by reaction of a corresponding compound of formula I in which one of $R_{14}$ and $R_{15}$ is -C(=NCN)Salkyl C1 to 6 with hydrazine,

o) production of a compound of formula I in which $R_{14}$ and $R_{15}$ together form a chain $-CR_{16}=N-CR_{17}=CR_{18}-$, by reaction of a corresponding compound of formula I in which $R_{14}$ and $R_{15}$ are both hydrogen with ammonia and an alkanaldehyde and an alkanendione in which the two oxygen atoms are carried on adjacent carbon atoms,

p) production of a compound of formula I in which $R_{19}$ is alkylamino-carbonyl or a 5 membered ring, by reacting a corresponding compound of formula I in which $R_{19}$ is hydrogen with an alkylisocyanate or with a corresponding 5 membered ring compound carrying a good leaving group,

q) production of a compound of formula I in which $R_{14}$ or $R_{15}$ is a group -C(=NCN)Salkyl C1 to 6, by reaction of a corresponding compound of formula I in which at least one of $R_{14}$ and $R_{15}$ is hydrogen, with a di-alkyl(C1 to 6)N-cyanoiminodithiocarbonate,

r) production of a compound of formula I in which at least one of $R_2$ and $R_6$ is substituted by a 2-amino-6-hydroxy-pyrimidin-4-yl group, by reacting a corresponding compound of formula I in which at least one of $R_2$ and $R_6$ is substituted by a betaketoester group with guanidine, or

s) production of an optical isomer of a compound of formula I by resolution of a mixture of optical isomers of the compound,

and where desired or necessary converting the resulting compound of formula I to a pharmaceutically acceptable acid addition salt thereof or vice versa.

9. The use of a compound according to any one of Claims 1 to 5 make a pharmaceutical composition for use in the treatment of a cardiovascular condition.

10. A compound of formula $R_4$a CHO in which $R_4$ is a phenyl group carrying 3 or 4 substituents $R_4$a being substituted by $CF_3$, $XR_{22}$, nitro, halogen, CN, alkly Cl to 6, formyl, dioxalanyl, $-N(R_{23})COR_{24}$, $-CONR_{25}R_{26}$ or amino which is substituted by phenylsulphonyl or by trifluoroacetyl, $R_{22}$, $R_{23}$, $R_{24}$, $R_{25}$ and $R_{26}$ being as defined in Claim 1 and $R_4$a carries a fluoro or $CF_3$ substituent on the atom adjacent to the atom through which $R_4$a is connected to the -CHO group, provided that $R_4$a is not 2,3-dichloro-6-fluorophenyl, 3-chloro-6-fluoro-2-(trifluoromethyl)phenyl or 2,3,5,6-tetrafluorophenyl.

2194J(ir)/jaa

What we claim is:-

1. A process for the production of a compound of formula I,

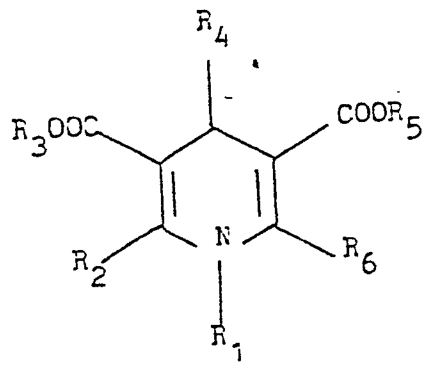

I

in which $R_1$ is hydrogen or alkyl C1 to 6,

$R_3$ and $R_5$, which may be the same or different, are each $-(CH_2)_x-(CHR_{34})_yR_{35}$,

x is a whole number from 0 to 7 inclusive,

y is 0 or 1,

$R_{34}$ is hydrogen, alkyl C1 to 6 or cycloalkyl containing up to and including 8 carbon atoms,

$R_{35}$ is hydrogen; alkyl C1 to 6 optionally substituted by hydrogen, by alkoxy C1 to 6, by alkanoyl C1 to 6, or by hydroxy; a 4, 5 or 6 membered oxygen, nitrogen or $S(O)_n$ containing heterocyclic ring which ring is optionally substituted by alkyl C1 to 6; cycloalkyl or cycloalkenyl containing up to and including 8 carbon atoms which cycloalkyl is in turn optionally bridged, substituted by alkyl C1 to 6 or is fused to a phenyl ring; a group $-CH=CHR_{13}$ in which $R_{13}$ is cycloalkyl containing up to and including 8 carbon atoms; or is an

alkyl C1 to 6 amino group in which the alkyl group(s) are optionally substituted by phenyl; or, when x is a whole number from 1 to 7 inclusive $R_{35}$ is CN, $-OSO_2R_{33}$ or a group of formula II or III,

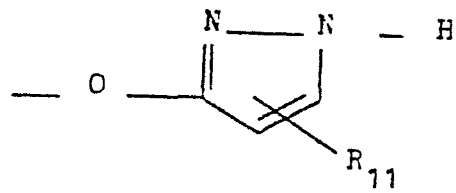

II

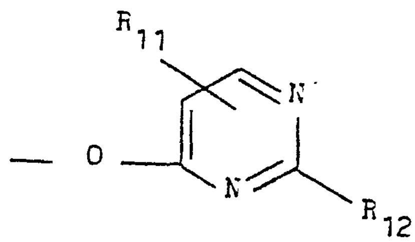

III

in which $R_{11}$ and $R_{12}$, which may be the same or different, are each alkyl C1 to 6 or phenyl, and

$R_{33}$ is alkyl C1 to 6 or alkyl C1 to 6-phenyl,

$R_2$ and $R_6$, which may be the same or different, are each CN, CHO or alkyl C1 to 6 optionally interrupted by an oxygen atom and optionally substituted by halogen, by hydroxy, by -COOH or an alkyl C1 to 6 ester thereof, by a C1 to 6 betaketoester group, by a 2-amino-6-hydroxy-pyridmidin-4-yl group or by a group $-NR_{14}R_{15}$,

$R_{14}$ and $R_{15}$, which may be the same or different, are each hydrogen, alkyl C1 to 6, -C(=NCN)Salkyl C1 to 6 or a group of formula IV,

 

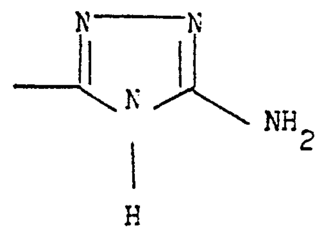

IV

or together form a chain $-CR_{16}=N-CR_{17}=CR_{18}-$, $-CH_2CH_2N(R_{19})CH_2CH_2-$ or -CO(o-phenylene)CO-,

$R_{16}$, $R_{17}$ and $R_{18}$, which may be the same or different, are each alkyl C1 to 6,

$R_{19}$ is hydrogen; alkyl C1 to 6 optionally substituted by phenyl which in turn is optionally substituted by halogen; $-CONR_{20}R_{21}$; or a 5 membered unsaturated ring containing nitrogen and optionally also sulphur,

$R_{20}$ and $R_{21}$, which may be the same or different, are each hydrogen or alkyl C1 to 6,

$R_4$ is a phenyl group carrying 3 or 4 substituents or is an unsaturated 5 membered heterocyclic group containing a single hetero atom selected from oxygen, sulphur or nitrogen, $R_4$ being substituted by $CF_3$, $XR_{22}$, nitro, halogen, CN, alkyl C1 to 6, formyl, dioxalanyl, $-N(R_{23})COR_{24}$, $-CONR_{25}R_{26}$, or amino which is optionally substituted by phenylsulphonyl or by trifluoroacetyl, and $R_4$ carries a substituent on the atom adjacent to the atom through which $R_4$ is connected

to the remainder of the molecule,

$R_{22}$ is phenyl or alkyl C1 to 6 optionally substituted by halogen,

X is O or $S(O)_n$,

n is O, 1 or 2, and

$R_{23}$, $R_{24}$, $R_{25}$ and $R_{26}$, which may be the same or different, are each hydrogen or alkyl C1 to 6,

provided that i) when $R_1$ is hydrogen or methyl, $R_2$ is $CH_2F$, $R_3$ is methyl and $R_5$ is 1-methylethyl or cyclopentyl, then $R_4$ is not 2-(trifluoromethyl)-3-chloro-6-fluorophenyl,

ii) when $R_1$ is hydrogen, $R_2$ is $CH_2F$, $R_3$ is methyl and $R_5$ is 1-methylethyl then $R_4$ is not 2,3-dichloro-6-fluorophenyl, and

iii) when $R_4$ is a heterocyclic group then at least one of $R_2$ and $R_6$ is other than alkyl or alkoxy-alkyl,

or a pharmaceutically acceptable acid addition salt of a compound of formula I containing a basic nitrogen atom, which comprises

a) reaction of the compounds of formula V, VII, X and XIV,

$R_4CHO$ V

$R_5OOCCH_2COR_6$ VII

$R_3OOCCH_2COR_2$ X

$R_1NH_2$ XIV

or their partial condensates,

in which $R_1$, $R_2$, $R_3$, $R_4$, $R_5$ and $R_6$ are as defined above,

b) production of a compound of formula I containing a group $-S(O)_m-$ in which m is 1 or 2 by selective oxidation of a corresponding compound of formula I in which m is 0 or 1,

c) production of a compound of formula I in which one or both of $R_2$ and $R_6$ is $-CHF_2$ or $-CH_2F$ by reaction of a corresponding compound of formula I in which one or both of $R_2$ and $R_6$ is -CHO or $-CH_2L$ respectively, where L is -OH or a good leaving group, with a fluorinating agent,

d) production of a compound of formula I carrying a -CHO substituent by

i) selective hydrolysis of a corresponding compound of formula I carrying a $-CH(OR_{27})_2$ substituent,

in which $R_{27}$ is alkyl C1 to 6, or the two group $R_{27}$ together form a $-CH_2CH_2-$ chain, or

ii) selective oxidation of a corresponding compound of formula I carrying a $-CH_2OH$ substituent,

e) production of a compound of formula I in which one of $R_2$ and $R_6$ is $-CH_2OH$ by selective hydrolysis of a corresponding compound of formula I in which one of $R_2$ and $R_6$ is $-CH_2Z$ and Z is a good leaving group,

f) production of a compound of formula I carrying a

group -CN by elimination of ROH from a corresponding compound of formula I carrying a group -CH=NOR, in which -OR is a good leaving group,

g) production of a compound of formula I in which at least one of $R_3$ and $R_5$ is hydrogen, or in which at least one of $R_2$ and $R_6$ carries a -COOH group, by reductive cleavage or hydrolysis of a corresponding compound of formula I in which at least one of $R_3$ and $R_5$ is other than hydrogen or in which at least one of $R_2$ and $R_6$ carries a carboxylic acid ester group,

h) production of a compound of formula I in which at least one of $R_3$ and $R_5$ is other than hydrogen, or in which at least one of $R_2$ and $R_6$ carries a carboxylic acid ester group or a betaketoester group, by esterification or transesterification of a corresponding compound of formula I in which at least one of $R_3$ and $R_5$ is hydrogen or is a group $R_3$ or $R_5$ other than that desired in the end product, or in which at least one of $R_2$ and $R_6$ carries a -COOH group,

i) production of a compound of formula I in which at least one of $R_2$ and $R_6$ is methyl by selective reduction of a corresponding compound of formula I in which at least one of $R_2$ and $R_6$ is $-CH_2Y$ and Y is a group reducible to hydrogen,

j) production of a compound of formula I carrying an

$-NH_2$ substituent by removal of a protecting group from a corresponding compound carrying a protected amino group,

k) production of a compound of formula I in which at least one of $R_3$ and $R_5$ is alkyl substituted by a group of formula II or III, by reaction of a corresponding compound of formula I in which at least one of $R_3$ and $R_5$ is alkyl substituted by a good leaving group, with a compound of formula XI or XII respectively,

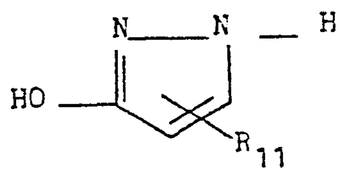

XI

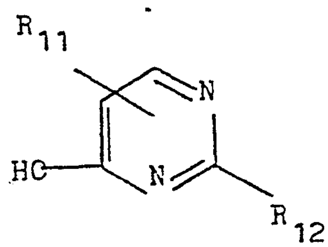

XII

or a tautomer or salt thereof,

in which $R_{11}$ and $R_{12}$ are as defined above,

l) production of a compound of formula I in which at least one of $R_3$ and $R_5$ represents alkyl substituted by a group $-CH(R_{36})(CH_2OH)-CH_2OH$, in which $R_{36}$ represents hydrogen or alkyl, by selective hydrolysis of a

corresponding compound of formula I in which at least one of $R_3$ and $R_5$ represents alkyl substituted by 3-oxetanyl which in turn is optionally substituted in the 3-position by alkyl,

m) production of a compound of formula I in which $R_1$ is alkyl C1 to 6, by C1 to 6 alkylation of a corresponding compound of formula I in which $R_1$ is hydrogen,

n) production of a compound of formula I in which one of $R_{14}$ and $R_{15}$ is a group of formula IV, by reaction of a corresponding compound of formula I in which one of $R_{14}$ and $R_{15}$ is -C(=NCN)Salkyl C1 to 6 with hydrazine,

o) production of a compound of formula I in which $R_{14}$ and $R_{15}$ together form a chain $-CR_{16}=N-CR_{17}=CR_{18}-$, by reaction of a corresponding compound of formula I in which $R_{14}$ and $R_{15}$ are both hydrogen with ammonia and an alkanaldehyde and an alkanendione in which the two oxygen atoms are carried on adjacent carbon atoms,

p) production of a compound of formula I in which $R_{19}$ is alkylamino-carbonyl or a 5 membered ring, by reacting a corresponding compound of formula I in which $R_{19}$ is hydrogen with an alkylisocyanate or with a corresponding 5 membered ring compound carrying a good leaving group,

q) production of a compound of formula I in which $R_{14}$ or $R_{15}$ is a group -C(=NCN)Salkyl C1 to 6, by reaction of a corresponding compound of formula I in which at least

one of $R_{14}$ and $R_{15}$ is hydrogen, with a di-alkyl(Cl to 6)N-cyanoiminodithiocarbonate,

r) production of a compound of formula I in which at least one of $R_2$ and $R_6$ is substituted by a 2-amino-6-hydroxy-pyrimidin-4-yl group, by reacting a corresponding compound of formula I in which at least one of $R_2$ and $R_6$ is substituted by a betaketoester group with guanidine, or

s) production of an optical isomer of a compound of formula I by resolution of a mixture of optical isomers of the compound,

and where desired or necessary converting the resulting compound of formula I to a pharmaceutically acceptable acid addition salt thereof or vice versa.

2. A process according to Claim 1, wherein $R_4$ is of formula XIII,

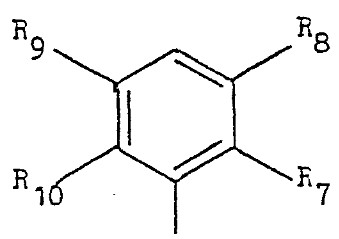

XIII

in which $R_7$ is trifluoromethyl, phenylthio, fluoro, phenylsulphonyl, nitro or chloro,

$R_8$ is nitrile, chloro, fluoro, phenylthio, methylthio, phenylsulphonylamino, trifluoroacetyl,

trifluoromethyl, methoxy, propylthio, nitro or amino,

$R_9$ is hydrogen, fluoro or chloro, and

$R_{10}$ is hydrogen, fluoro, methoxy or chloro.

3. A process according to Claim 2, wherein $R_1$ is hydrogen, $R_2$ is $-CH_2F$ or $CH_3$, $R_3$ is methyl, $R_5$ is 3-thietanyl, 1-methylethyl, cyclobutyl, 3-thietanyl-S,S-dioxide, 6-(5-phenyl-3-pyrazolyloxy)hexyl, cyclopropylmethyl, 6-((6-methyl-2-phenyl-4-pyrimidinyl)oxy) hexyl or ethyl, $R_6$ is methyl or (2-aminoethoxy)methyl and $R_4$ is 3-nitro-2-thienyl, or a group of formula XIII in which $R_7$ is $CF_3$, $R_8$ is Cl or CN, $R_9$ is hydrogen and $R_{10}$ is F.

4. A process according to Claim 1, wherein the compound of formula I is 3-Methyl 5-(3-thietanyl) 4-(3-cyano-6-fluoro-2--(trifluoromethyl)phenyl)-2-(fluoromethyl)-1,4-dihydro-6-methyl-3,5-pyridinedicarboxylate,

3-Methyl 5-(6-((6-methyl-2-phenyl-4-pyrimidinyl)oxy) hexyl) 4-(3-chloro-6-fluoro-2-(trifluoromethyl)phenyl)-2-(fluoromethyl)-1,4-dihydro-6-methyl-3,5-pyridinedicarboxylate or a pharmaceutically acceptable salt thereof, or

3-Ethyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate or a pharmaceutically acceptable salt thereof.

5. The use of a compound of formula I as defined in any one of Claims 1 to 4 to make a pharmaceutical composition for use in the treatment of a cardiovascular condition.

2208J(ir)/jaa